(19) Europäisches Patentamt
European Patent Office
Office européen des brevets

(11) EP 2 809 307 B1

(12) **EUROPEAN PATENT SPECIFICATION**

(45) Date of publication and mention of the grant of the patent:
**20.12.2017 Bulletin 2017/51**

(21) Application number: **13702971.6**

(22) Date of filing: **29.01.2013**

(51) Int Cl.:
***A61K 9/70*** (2006.01) ***A61K 31/407*** (2006.01)
***A61P 29/00*** (2006.01)

(86) International application number:
**PCT/EP2013/051660**

(87) International publication number:
**WO 2013/113690 (08.08.2013 Gazette 2013/32)**

(54) **PHARMACEUTICAL PATCH FOR TRANSDERMAL ADMINISTRATION OF (1R,4R)-6'-FLUORO-N,N-DIMETHYL-4-PHENYL-4',9'-DIHYDRO-3'H-SPIRO[CYCLOHEXANE-1,1'-PYRANO[3,4-B]INDOL]-4-AMINE**

PHARMAZEUTISCHES PFLASTER ZUR TRANSDERMALEN VERABREICHUNG VON (1R,4R)-6'-FLUOR-N,N-DIMETHYL-4-PHENYL-4',9'-DIHYDRO-3'H-SPIRO-[CYCLOHEXAN-1,1'-PYRANO[3,4,B]INDOL]-4-AMIN

TIMBRE PHARMACEUTIQUE POUR L'ADMINISTRATION TRANSDERMIQUE DE (1R,4R)-6'-FLUORO-N,N-DIMETHYL-4-PHENYL-4',9'-DIHYDRO-3'H-SPIRO[CYCLOHEXANE-1,1'-PYRANO[3,4-B]INDOL]-4-AMINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.01.2012 US 201261592850 P**
**30.03.2012 EP 12002356**

(43) Date of publication of application:
**10.12.2014 Bulletin 2014/50**

(73) Proprietor: **Grünenthal GmbH**
**52078 Aachen (DE)**

(72) Inventors:
- **FUHRHERR, Richard**
  **63500 Seligenstadt (DE)**
- **GALIA, Eric**
  **52078 Aachen (DE)**
- **KUGELMANN, Heinrich**
  **52068 Aachen (DE)**
- **WILL, Olaf**
  **52223 Stolberg (DE)**
- **GAUTROIS, Michael**
  **52152 Simmerath (DE)**
- **HARTWIG, Rod**
  **Cooper City, Florida 33330 (US)**
- **PERNAS, Enrique R.**
  **Miami, Florida 33140 (US)**
- **PRENNER, Lars**
  **53113 Bonn (DE)**
- **BREITENBACH, Armin**
  **51371 Leverkusen (DE)**
- **FRIEDRICH, Ingo**
  **52078 Aachen (DE)**
- **BRAUN, Sebastian**
  **42929 Wermelskirchen (DE)**

(74) Representative: **Bülle, Jan et al**
**Kutzenberger Wolff & Partner**
**Theodor-Heuss-Ring 23**
**50668 Köln (DE)**

(56) References cited:
**WO-A1-2004/043967 WO-A1-2008/040481**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The invention relates to a pharmaceutical patch for transdermal administration of the pharmacologically active ingredient (1r,4r)-6'-fluoro-N,N-dimethyl-4-phenyl-4',9'-dihydro-3'H-spiro[cyclohexane-1,1'-pyrano[3,4-b]indol]-4-amine or a physiologically acceptable salt thereof, the patch comprising a surface layer, an adhesive layer, and a removable protective layer, wherein the adhesive layer is located between the surface layer and the removable protective layer.

The pharmacologically active ingredient according to the invention is known from the prior art to exhibit analgesic properties and is particularly suitable for the treatment of acute, visceral, neuropathic or chronic pain (cf., e.g., WO 2004/043967 and WO 2008/040481).

While the pharmacologically active ingredient according to the invention has a sufficient bioavailability so that it can be administered orally, it is desirable to provide an alternative route of systemic administration. It is known that transdermal administration of a pharmacologically active ingredient can be advantageous compared to its oral administration, e.g. with respect to patient compliance.

The working principle of a pharmaceutical patch for transdermal administration relies on the release of the pharmacologically active ingredient from the patch, its penetration into and through the skin barrier, and its entry into the systemic circulation through the perfused subcutaneous tissue, where it then develops its pharmacological effect at the targeted receptors. The penetration of a pharmacologically active ingredient through the skin is largely determined by its physicochemical properties and so far, there are only relatively few preparations of pharmacologically active ingredients that are suitable for transdermal administration.

In general, besides the desired pharmacological effect of pain relief, a pharmaceutical patch for transdermal administration of an analgesic should satisfy the following requirements:

- good adhesion to the skin without skin irritations at the contact area, even after long term application;
- appropriate size that is as inconspicuous as possible;
- good shelf-life and storage stability, e.g. no recrystallization of the pharmacologically active ingredient, reduction or even suppression of chemical degradation of the pharmacologically active ingredient;
- low but sufficient content of the pharmacologically active ingredient to maintain therapeutic plasma concentrations over extended periods of time; and
- well adjusted flux rate to make available to the patient as much as possible of the pharmacologically active ingredient contained in the pharmaceutical patch over a predetermined period of time at a constant or nearly constant flux rate.

Only a very few pharmacologically active ingredients may be administered transdermally. Typically, the transdermal administrability of a given drug can be assessed in view of some of its characteristic features (cf. T.K. Ghosh et al., Transdermal and topical drug delivery systems; Interpharm Press (Buffalo Grove, Ill., 2002)), particularly

- its water solubility,
- its melting point, and
- its partition coefficient in the system octanol/water.

High water solubilities, low melting points as well as partition coefficients between 1 and 3.5 (log P, max. 4.0) have been reported in the literature as being generally desirable for transdermal administration.

The water solubility of (1r,4r)-6'-fluoro-N,N-dimethyl-4-phenyl-4',9'-dihydro-3'H-spiro[cyclohexane-1,1'-pyrano[3,4-b]indol]-4-amine (free base as well as hemicitrate) is very poor (less than 0.3 $\mu$g/ml), its melting point is very high (~300°C) and its partition coefficient substantially outside the desirable range (log P 5.3).

Therefore, one would typically expect that this pharmacologically active ingredient cannot be administered transdermally.

It is an object of the invention to provide an advantageous pharmaceutical preparation of (1r,4r)-6'-fluoro-N,N-dimethyl-4-phenyl-4',9'-dihydro-3'H-spiro[cyclohexane-1,1'-pyrano[3,4-b]indol]-4-amine or a physiologically acceptable salt thereof.

This object has been achieved by the subject-matter of the patent claims.

It has been surprisingly found that (1r,4r)-6'-fluoro-N,N-dimethyl-4-phenyl-4',9'-dihydro-3'H-spiro[cyclohexane-1,1'-pyrano[3,4-b]indol]-4-amine, in form of the free base or in form of its physiologically acceptable salts, can be administered transdermally, i.e. that pharmaceutical formulations can be found which release the pharmacologically active ingredient in a form that is capable of penetrating into the skin barrier and entering into the systemic circulation through

the perfused subcutaneous tissue in a sufficient quantity and rate to develop its desired analgesic effect.

The pharmaceutical patch according to the invention comprises a surface layer, an adhesive layer, and a removable protective layer, wherein the adhesive layer is located between the surface layer and the removable protective layer.

The adhesive layer is located between the surface layer and the removable protective layer. Preferably, the surface layer forms the outer surface of the pharmaceutical patch, i.e. when the pharmaceutical patch is applied to the skin the surface layer is the visible layer of the pharmaceutical patch.

Preferably, one of the two opposing surfaces of the adhesive layer is in intimate contact with, i.e. adjacent to the removable protective layer.

In a preferred embodiment, the other of the two opposing surfaces of the adhesive layer is in intimate contact with the surface layer, which in turn preferably forms on its outer surface the outer surface of the pharmaceutical patch. According to this embodiment of the invention, the pharmaceutical patch preferably consists of surface layer, adhesive layer and removable protective layer, so that the adhesive layer contains the pharmacologically active ingredient (drug-in-adhesive).

In another preferred embodiment, the other of the two opposing surfaces of the adhesive layer is not in intimate contact with the surface layer, which in turn preferably forms on its outer surface the outer surface of the pharmaceutical patch. Thus, according to this embodiment, at least one additional layer is present between the surface layer and the adhesive layer. According to this embodiment of the invention, the pharmaceutical patch preferably comprises the surface layer, the adhesive layer, the removable protective layer and one, two, three or more additional layers between the adhesive layer and the surface layer, so that the pharmacologically active ingredient can be present in the adhesive layer and/or in any one of said additional layers.

The total thickness of the pharmaceutical patch is not particularly limited. Preferably, the total thickness of the pharmaceutical patch is within the range of from more preferably 20 to 1000 μm, still more preferably 40 to 800 μm, yet more preferably 60 to 650 μm, even more preferably 80 to 550 μm, most preferably 100 to 450 μm, and in particular 150 to 400 μm. In a preferred embodiment, the total thickness of the pharmaceutical patch is within the range of 100±75 μm (i.e. from 25 μm to 175 μm), preferably 100±50 μm. In another preferred embodiment, the total thickness of the pharmaceutical patch is within the range of 150±100 μm, preferably 150±75 μm, more preferably 150±50 μm. In another preferred embodiment, the total thickness of the pharmaceutical patch is within the range of 200±150 μm, preferably 200±100 μm, more preferably 200±50 μm. In another preferred embodiment, the total thickness of the pharmaceutical patch is within the range of 300±250 μm, preferably 300±200 μm, more preferably 300±150 μm, still more preferably 300±100 μm, and yet more preferably 300±50 μm. In still another preferred embodiment, the total thickness of the pharmaceutical patch is within the range of 400±350 μm, preferably 400±300 μm, more preferably 400±250 μm, still more preferably 400±200 μm, yet more preferably 400±150 μm, even more preferably 400±100 μm, and most preferably 400±50 μm. In yet another preferred embodiment, the total thickness of the pharmaceutical patch is within the range of 500±400 μm, preferably 500±350 μm, more preferably 500±300 μm, still more preferably 500±250 μm, yet more preferably 500±200 μm, even more preferably 500±150 μm, most preferably 500±100 μm, and in particular 500±50 μm. In preferred embodiments, the aforementioned values include the removable protective layer. In another preferred embodiment, the aforementioned values exclude the removable protective layer.

In a preferred embodiment, the adhesive layer comprises at least a portion of the total amount of the pharmacologically active ingredient that is contained in the pharmaceutical patch.

In a preferred embodiment, the adhesive layer is adjacent to the removable protective layer and/or to the surface layer. Preferably, the adhesive layer is adjacent to the removable protective layer and to the surface layer. In a particularly preferred embodiment, the pharmaceutical patch is composed of the surface layer, the adhesive layer, and the removable protective layer and does not contain any additional layer.

In another preferred embodiment, the pharmaceutical patch further comprises at least one drug layer, which comprises at least a portion of the total amount of the pharmacologically active ingredient that is contained in the pharmaceutical patch.

Preferably, the drug layer comprises at least 10 wt.-%, more preferably at least 25 wt.-%, still more preferably at least 50 wt.-%, yet more preferably at least 75 wt.-%, even more preferably at least 85 wt.-%, most preferably at least 90 wt.-%, and in particular at least 95 wt.-% of the total amount of the pharmacologically active ingredient that is contained in the pharmaceutical patch. In an especially preferred embodiment, the drug layer comprises the total amount of the pharmacologically active ingredient that is contained in the pharmaceutical patch. A skilled person recognizes that when the drug layer is not identical with the adhesive layer, a certain amount of the pharmacologically active ingredient migrates from the drug layer into the adjacent drug permeable layer(s) for thermodynamic reasons until an equilibrium has been reached. Thus, even if the material forming the adhesive layer did not contain any pharmacologically active ingredient when the pharmaceutical patch was manufactured, the final product typically does contain pharmacologically active ingredient not only in the drug layer but also in the adhesive layer.

In a preferred embodiment, the drug layer is located between the adhesive layer and the surface layer. The

drug layer may be separated from the adhesive layer by a membrane or may be in intimate contact with, i.e. adjacent to the adhesive layer.

In a preferred embodiment, the drug layer comprises a portion of the total amount of the pharmacologically active ingredient and the adhesive layer comprises another portion of the total amount of the pharmacologically active ingredient that is contained in the pharmaceutical patch.

In another preferred embodiment, the adhesive layer does not comprise any pharmacologically active ingredient upon manufacture of the pharmaceutical patch, whereas typically there is an exchange of the pharmacologically active ingredient between adjacent layers until an equilibrium between the drug permeable layers has been reached. Preferably, when the pharmaceutical patch comprises a drug layer that is separate from the adhesive layer, the drug layer is located between the adhesive layer and the surface layer, in particular adjacent to the adhesive layer. In another preferred embodiment, the drug layer and at least a part of the adhesive layer are both in contact with the same side of the removable protective layer, wherein the area of the drug layer is preferably smaller than the area of the removable protective layer. The adhesive layer may either overlap with the drug layer or be only present in that part of the removable protective layer that is not in contact with the drug layer, for example by forming a ring or a frame around the drug layer.

In a preferred embodiment, the material of the adhesive layer only covers a portion of the adjacent layer(s), e.g. assumes the form of a grid or any other suitable pattern.

The drug layer may be present in form of a liquid, a semisolid, or a solid polymer matrix.

In a preferred embodiment, the drug layer comprises a liquid containing the pharmacologically active ingredient in form of a solution or suspension.

In another preferred embodiment, the drug layer is a semisolid, such as a gel, or a solid polymer matrix wherein the pharmacologically active ingredient is dispersed.

In a preferred embodiment, the total amount of the pharmacologically active ingredient is present in molecular dispersed form.

In another preferred embodiment, only a portion of the pharmacologically active ingredient is present in molecular dispersed form, while the remainder of the pharmacologically active ingredient is present is non-molecular dispersed form (e.g. in form of droplets, crystals and the like) serving the purpose of a depot, also called "microreservoir".

The pharmacologically active ingredient according to the invention, i.e. (1r,4r)-6'-fluoro-N,N-dimethyl-4-phenyl-4',9'-dihydro-3'H-spiro[cyclohexane-1,1'-pyrano[3,4-b]indol]-4-amine (free base), has the following structural formula (I):

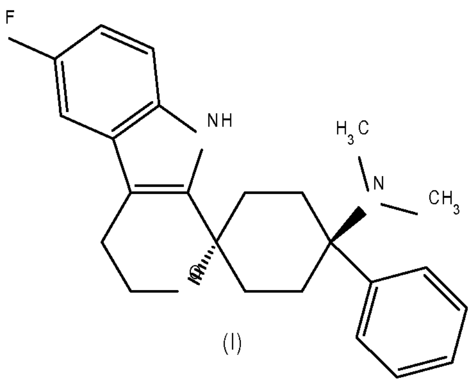

(I)

The pharmacologically active ingredient (free base) can alternatively be referred to as "1,1-(3-dimethylamino-3-phenylpentamethylene)-6-fluoro-1,3,4,9-tetrahydropyrano[3,4-b]indole (trans)". Unless expressly stated otherwise, all quantities refer to the weight of the free base.

The pharmacologically active ingredient can be present in the pharmaceutical patch according to the invention in form of the free base or as derivative thereof in any possible form, thereby particularly including solvates and polymorphs, salts, in particular acid addition salts and corresponding solvates and polymorphs. The hemicitrate is a preferred example of an acid addition salt.

The pharmacologically active ingredient can be present in the pharmaceutical patch according to the invention in form of the free base or in form of an acid addition salt, whereby any suitable acid capable of forming such an addition salt may be used. The conversion of the pharmacologically active ingredient into a corresponding addition salt, for example, via reaction with a suitable acid may be effected in a manner well known to those skilled in the art. Suitable acids include but are not limited to hydrochloric acid, hydrobromic acid, sulfuric acid, methanesulfonic acid, formic acid, acetic acid, oxalic acid, succinic acid, tartaric acid, mandelic acid, fumaric acid, lactic acid, citric acid, glutamic acid and/or aspartic acid. Salt formation is preferably effected in a solvent, for example, diethyl ether, diisopropyl ether, alkyl acetates, acetone and/or 2-butanone. Moreover, trimethylchlorosilane in aqueous solution is also suitable for the preparation of hydrochlorides.

Preferably, however, the pharmacologically active ingredient is present in form of the free base. It has been found that the transdermal bioavailability of the pharmacologically active ingredient in form of the free base is substantially higher (about 2-3 times higher) than the bioavailability of its hemicitrate.

Preferably, the pharmacologically active ingredient is contained in the adhesive layer, while a certain portion of the pharmacologically active ingredient may be contained in the adjacent layers e.g. due to migration and/or diffusion. The concentration of the pharmacologically active ingredient in the adhesive layer is at least 0.50 wt.-%, most preferably at least 0.60 wt.-% and in particular at least 0.70 wt.-%, relative to the total weight of the adhesive layer.

For the purpose of the specification, unless expressly stated otherwise, all weight percentages relate to the total weight of the pharmaceutical patch or to the total weight of a specific layer thereof in terms of total per dry unit. In this regard, "dry unit" shall encompass all constituents, irrespective of whether they are present in solid, semisolid or liquid form, but shall not encompass volatile solvents that are evaporated in course of the preparation of the pharmaceutical patch such as ethanol, heptane, ethyl acetate and the like. Thus, "dry unit" shall merely encompass the residual content of volatile solvent(s), if any.

In a preferred embodiment, the concentration of the pharmacologically active ingredient in the adhesive layer is at least 1.00 wt.-%, more preferably at least 1.25 wt.-%, still more preferably at least 1.50 wt.-%, yet more preferably at least 1.75 wt.-%, even more preferably at least 2.00 wt.-%, most preferably at least 2.25 wt.-% and in particular at least 2.50 wt.-%, relative to the total weight of the adhesive layer. In another preferred embodiment, the concentration of the pharmacologically active ingredient in the adhesive layer is at least 2.75 wt.-%, more preferably at least 3.00 wt.-%, still more preferably at least 3.25 wt.-%, yet more preferably at least 3.50 wt.-%, even more preferably at least 3.75 wt.-%, most preferably at least 4.00 wt.-% and in particular at least 4.25 wt.-%, relative to the total weight of the adhesive layer. In still another preferred embodiment, the concentration of the pharmacologically active ingredient in the adhesive layer is at least 4.50 wt.-%, more preferably at least 4.75 wt.-%, still more preferably at least 5.00 wt.-%, yet more preferably at least 5.25 wt.-%, even more preferably at least 5.50 wt.-%, most preferably at least 5.75 wt.-% and in particular at least 6.00 wt.-%, relative to the total weight of the adhesive layer.

It is principally desirable to provide the pharmacologically active ingredient in the adhesive layer at comparatively high concentrations, as this may positively influence the flux rate. As there is evidence, however, that the pharmacologically active ingredient tends to recrystallize at high concentrations, the concentration of the pharmacologically active ingredient in the adhesive layer is preferably at most 7.50 wt.-%, more preferably at most 5.00 wt.-%, still more preferably at most 2.50 wt.-%, yet more preferably at most 2.00 wt.-%, even more preferably at most 1.50 wt.-%, most preferably at most 1.00 wt.-%, and in particular at most 0.80 wt.-%, relative to the total weight of the adhesive layer (total per dry unit).

The total dose of the pharmacologically active ingredient that is contained in the pharmaceutical patch is not particularly limited and may depend upon various factors such as body weight of the subject to be treated and duration of application on the skin. The pharmacologically active ingredient is contained in the pharmaceutical patch in a therapeutically effective amount. The amount that constitutes a therapeutically effective amount varies according to the form of the pharmacologically active ingredient being present, the condition being treated, the severity of said condition, the patient being treated, and the prescribed duration of application of the pharmaceutical patch to the skin.

In a preferred embodiment, the pharmaceutical patch contains the pharmacologically active ingredient in a quantity so that during 12 hours or during 24 hours or during 48 hours or during 72 hours or during 96 hours or even during 168 hours of consecutive application of a series of pharmaceutical patches to the skin, i.e. under steady state conditions taking into account the depot effect of the skin, an amount of $20\pm15$ μg (i.e. from 5 μg to 35 μg), more preferably $20\pm12.5$ μg, still more preferably $20\pm10$ μg, most preferably $20\pm7.5$ μg, and in particular $20\pm5$ μg is systemically administered per day.

In another preferred embodiment, the pharmaceutical patch contains the pharmacologically active ingredient in a quantity so that during 12 hours or during 24 hours or during 48 hours or during 72 hours or during 96 hours or even during 168 hours of consecutive application of a series of pharmaceutical patches to the skin, i.e. under steady state conditions taking into account the depot effect of the skin, an amount of $40\pm35$ μg, more preferably $40\pm30$ μg, still more preferably $40\pm25$ μg, yet more preferably $40\pm20$ μg, even more preferably $40\pm15$ μg, most preferably $40\pm10$ μg, and in particular $40\pm5$ μg is systemically administered per day.

In another preferred embodiment, the pharmaceutical patch contains the pharmacologically active ingredient in a quantity so that during 12 hours or during 24 hours or during 48 hours or during 72 hours or during 96 hours or even during 168 hours of consecutive application of a series of pharmaceutical patches to the skin, i.e. under steady state conditions taking into account the depot effect of the skin, an amount of $60\pm45$ μg, more preferably $60\pm35$ μg, still more preferably $60\pm25$ μg, yet more preferably $60\pm20$ μg, even more preferably $60\pm15$ μg, most preferably $60\pm10$ μg, and in particular $60\pm5$ μg is systemically administered per day.

In another preferred embodiment, the pharmaceutical patch contains the pharmacologically active ingredient in a quantity so that during 12 hours or during 24 hours or during 48 hours or during 72 hours or during 96 hours or even during 168 hours of consecutive application of a series of pharmaceutical patches to the skin, i.e. under steady state

conditions taking into account the depot effect of the skin, an amount of 80±70 μg, more preferably 80±60 μg, still more preferably 80±50 μg, yet more preferably 80±40 μg, even more preferably 80±30 μg, most preferably 80±20 μg, and in particular 80±10 μg is systemically administered per day.

In still another preferred embodiment, the pharmaceutical patch contains the pharmacologically active ingredient in a quantity so that during 12 hours or during 24 hours or during 48 hours or during 72 hours or during 96 hours or even during 168 hours of consecutive application of a series of pharmaceutical patches to the skin, i.e. under steady state conditions taking into account the depot effect of the skin, an amount of 160±140 μg, more preferably 160±120 μg, still more preferably 160±100 μg, yet more preferably 160±80 μg, even more preferably 160±60 μg, most preferably 160±40 μg, and in particular 160±20 μg is systemically administered per day.

In another preferred embodiment, the pharmaceutical patch contains the pharmacologically active ingredient in a quantity so that during 12 hours or during 24 hours or during 48 hours or during 72 hours or during 96 hours or even during 168 hours of consecutive application of a series of pharmaceutical patches to the skin, i.e. under steady state conditions taking into account the depot effect of the skin, an amount of 280±250 μg, more preferably 280±200 μg, still more preferably 280±150 μg, yet more preferably 280±100 μg, even more preferably 280±75 μg, most preferably 280±50 μg, and in particular 280±25 μg is systemically administered per day.

In yet another preferred embodiment, the pharmaceutical patch contains the pharmacologically active ingredient in a quantity so that during 12 hours or during 24 hours or during 48 hours or during 72 hours or during 96 hours or even during 168 hours of consecutive application of a series of pharmaceutical patches to the skin, i.e. under steady state conditions taking into account the depot effect of the skin, an amount of 400±350 μg, more preferably 400±300 μg, still more preferably 400±250 μg, yet more preferably 400±200 μg, even more preferably 400±150 μg, most preferably 400±100 μg, and in particular 400±50 μg is systemically administered per day.

In another preferred embodiment, the pharmaceutical patch contains the pharmacologically active ingredient in a quantity so that during 12 hours or during 24 hours or during 48 hours or during 72 hours or during 96 hours or even during 168 hours of consecutive application of a series of pharmaceutical patches to the skin, i.e. under steady state conditions taking into account the depot effect of the skin, an amount of 600±400 μg, more preferably 600±300 μg, still more preferably 600±200 μg, yet more preferably 600±150 μg, even more preferably 600±100 μg, most preferably 600±75 μg, and in particular 600±50 μg is systemically administered per day.

Preferably, the total dose of the pharmacologically active ingredient that is contained in the pharmaceutical patch satisfies the following requirement:

$$\text{dose contained in patch}\,[\mu g] = \frac{\text{intended duration of application}\,[\text{days}] \cdot \text{desired systemic daily dose}\,[\mu g]}{\text{bioavailability}\,[\%]} \cdot 100$$

In a preferred embodiment, the desired daily dose amounts to 20±15 μg (i.e. from 5 μg to 35 μg), more preferably 20±10 μg; or 40±20 μg, more preferably 40±10 μg; or 80±40 μg, more preferably 80±20 μg; or 160±80 μg, more preferably 160±40 μg; or 400±200 μg, more preferably 400±100 μg. The intended duration of application is preferably 1, 2, 3, 4, 5, 6, or 7 days. The bioavailability is preferably as high as possible and can be determined for a given pharmaceutical patch by routine experimentation.

Preferably, the transdermal bioavailability is within the range of 2.0±1.8% (i.e. from 0.2% to 3.8%), more preferably 2.0±1.6%, still more preferably 2.0±1.4%, yet more preferably 2.0±1.2%, even more preferably 2.0±1.0%, most preferably 2.0±0.8%, and in particular 2.0±0.6%. In a preferred embodiment, the transdermal bioavailability is within the range of 5.0±4.5%, more preferably 5.0±4.0%, still more preferably 5.0±3.5%, yet more preferably 5.0±3.0%, even more preferably 5.0±2.5%, most preferably 5.0±2.0%, and in particular 5.0±1.5%. In another preferred embodiment, the transdermal bioavailability is within the range of 10±8.0%, more preferably 10±7.0%, still more preferably 10±6.0%, yet more preferably 10±5.0%, even more preferably 10±4.0%, most preferably 10±3.0%, and in particular 10±2.0%. In still another preferred embodiment, the transdermal bioavailability is within the range of 15±13%, more preferably 15±11%, still more preferably 15±9.0%, yet more preferably 15±7.0%, even more preferably 15±6.0%, most preferably 15±5.0%, and in particular 15±4.0%. In yet another preferred embodiment, the transdermal bioavailability is within the range of 20±18%, more preferably 20±16%, still more preferably 20±14%, yet more preferably 20±12%, even more preferably 20±10%, most preferably 20±8.0%, and in particular 20±6.0%.

Preferably, the area concentration of the pharmacologically active ingredient in the adhesive layer and the drug layer, respectively, is within the range of from 0.01 to 10 $g/m^2$.

In a preferred embodiment, the area concentration of the pharmacologically active ingredient is within the range of 0.20±0.18 $g/m^2$ (i.e. from 0.02 $g/m^2$ to 0.38 $g/m^2$), more preferably 0.20±0.15 $g/m^2$, still more preferably 0.20±0.13 $g/m^2$, yet more preferably 0.20±0.10 $g/m^2$, even more preferably 0.20±0.08 $g/m^2$, most preferably 0.20±0.05 $g/m^2$, and in particular 0.20±0.03 $g/m^2$.

In another preferred embodiment, the area concentration of the pharmacologically active ingredient is within

the range of 0.40±0.35 $g/m^2$, more preferably 0.40±0.30 $g/m^2$, still more preferably 0.40±0.25 $g/m^2$, yet more preferably 0.40±0.20 $g/m^2$, even more preferably 0.40±0.15 $g/m^2$, most preferably 0.40±0.10 $g/m^2$, and in particular 0.40±0.05 $g/m^2$. In still another preferred embodiment, the area concentration of the pharmacologically active ingredient is within the range of 1.00±0.85 $g/m^2$, more preferably 1.00±0.80 $g/m^2$, still more preferably 1.00±0.75 $g/m^2$, yet more preferably 1.00±0.70 $g/m^2$, even more preferably 1.00±0.65 $g/m^2$, most preferably 1.00±0.60 $g/m^2$, and in particular 1.00±0.55 $g/m^2$. In yet another preferred embodiment, the area concentration of the pharmacologically active ingredient is within the range of 3.00±2.50 $g/m^2$, more preferably 3.00±2.25 $g/m^2$, still more preferably 3.00±2.00 $g/m^2$, yet more preferably 3.00±1.75 $g/m^2$, even more preferably 3.00±1.50 $g/m^2$, most preferably 3.00±1.25 $g/m^2$, and in particular 3.00±1.00 $g/m^2$. In another preferred embodiment, the area concentration of the pharmacologically active ingredient is within the range of 6.00±5.00 $g/m^2$, more preferably 6.00±4.50 $g/m^2$, still more preferably 6.00±4.00 $g/m^2$, yet more preferably 6.00±3.50 $g/m^2$, even more preferably 6.00±3.00 $g/m^2$, most preferably 6.00±2.50 $g/m^2$, and in particular 6.00±2.00 $g/m^2$.

In a preferred embodiment, the pharmaceutical patch upon application to the human skin provides over a period of at least 6 hours, more preferably at least 12 hours, release of the pharmacologically active ingredient at a rate of at least 1.0 $ng{\cdot}cm^{-2}{\cdot}h^{-1}$ or at least 2.5 $ng{\cdot}cm^{-2}{\cdot}h^{-1}$ or at least 5.0 $ng{\cdot}cm^{-2}{\cdot}h^{-1}$; more preferably at least 7.5 $ng{\cdot}cm^{-2}{\cdot}h^{-1}$ or at least 10 $ng{\cdot}cm^{-2}{\cdot}h^{-1}$ or at least 15 $ng{\cdot}cm^{-2}{\cdot}h^{-1}$; still more preferably at least 25 $ng{\cdot}cm^{-2}{\cdot}h^{-1}$ or at least 50 $ng{\cdot}cm^{-2}{\cdot}h^{-1}$ or at least 75 $ng{\cdot}cm^{-2}{\cdot}h^{-1}$; yet more preferably at least 100 $ng{\cdot}cm^{-2}{\cdot}h^{-1}$ or at least 150 $ng{\cdot}cm^{-2}{\cdot}h^{-1}$ or at least 200 $ng{\cdot}cm^{-2}{\cdot}h^{-1}$; even more preferably at least 250 $ng{\cdot}cm^{-2}{\cdot}h^{-1}$ or at least 300 $ng{\cdot}cm^{-2}{\cdot}h^{-1}$ or at least 350 $ng{\cdot}cm^{-2}{\cdot}h^{-1}$; most preferably at least 400 $ng{\cdot}cm^{-2}{\cdot}h^{-1}$ or at least 450 $ng{\cdot}cm^{-2}{\cdot}h^{-1}$ or at least 500 $ng{\cdot}cm^{-2}{\cdot}h^{-1}$; and in particular at least 550 $ng{\cdot}cm^{-2}{\cdot}h^{-1}$ or at least 600 $ng{\cdot}cm^{-2}{\cdot}h^{-1}$ or at least 650 $ng{\cdot}cm^{-2}{\cdot}h^{-1}$.

In a preferred embodiment, the pharmaceutical patch upon application to the human skin provides over a period of at least 6 hours, more preferably at least 12 hours, release of the pharmacologically active ingredient at a rate of 5.0±4.6 $ng{\cdot}cm^{-2}{\cdot}h^{-1}$ (i.e. from 0.4 $ng{\cdot}cm^{-2}{\cdot}h^{-1}$ to 9.6 $ng{\cdot}cm^{-2}{\cdot}h^{-1}$), more preferably 5.0±4.2 $ng{\cdot}cm^{-2}{\cdot}h^{-1}$, still more preferably 5.0±3.8 $ng{\cdot}cm^{-2}{\cdot}h^{-1}$, yet more preferably 5.0±3.4 $ng{\cdot}cm^{-2}{\cdot}h^{-1}$, even more preferably 5.0±3.0 $ng{\cdot}cm^{-2}{\cdot}h^{-1}$, most preferably 5.0±2.6 $ng{\cdot}cm^{-2}{\cdot}h^{-1}$ and oin particular 5.0±2.2 $ng{\cdot}cm^{-2}{\cdot}h^{-1}$.

In another preferred embodiment, the pharmaceutical patch upon application to the human skin provides over a period of at least 6 hours, more preferably at least 12 hours, release of the pharmacologically active ingredient at a rate of 50±46 $ng{\cdot}cm^{-2}{\cdot}h^{-1}$, more preferably 50±42 $ng{\cdot}cm^{-2}{\cdot}h^{-1}$, still more preferably 50±38 $ng{\cdot}cm^{-2}{\cdot}h^{-1}$, yet more preferably 50±34 $ng{\cdot}cm^{-2}{\cdot}h^{-1}$, even more preferably 50±30 $ng{\cdot}cm^{-2}{\cdot}h^{-1}$, most preferably 50±26 $ng{\cdot}cm^{-2}{\cdot}h^{-1}$ and in particular 50±22 $ng{\cdot}cm^{-2}{\cdot}h^{-1}$.

In still another preferred embodiment, the pharmaceutical patch upon application to the human skin provides over a period of at least 6 hours, more preferably at least 12 hours, release of the pharmacologically active ingredient at a rate of 500±460 $ng{\cdot}cm^{-2}{\cdot}h^{-1}$, more preferably 500±420 $ng{\cdot}cm^{-2}{\cdot}h^{-1}$, still more preferably 500±380 $ng{\cdot}cm^{-2}{\cdot}h^{-1}$, yet more preferably 500±340 $ng{\cdot}cm^{-2}{\cdot}h^{-1}$, even more preferably 500±300 $ng{\cdot}cm^{-2}{\cdot}h^{-1}$, most preferably 500±260 $ng{\cdot}cm^{-2}{\cdot}h^{-1}$ and in particular 500±220 $ng{\cdot}cm^{-2}{\cdot}h^{-1}$.

In yet another preferred embodiment, the pharmaceutical patch upon application to the human skin provides over a period of at least 6 hours, more preferably at least 12 hours, release of the pharmacologically active ingredient at a rate of 5000±4600 $ng{\cdot}cm^{-2}{\cdot}h^{-1}$, more preferably 5000±4200 $ng{\cdot}cm^{-2}{\cdot}h^{-1}$, still more preferably 5000±3800 $ng{\cdot}cm^{-2}{\cdot}h^{-1}$, yet more preferably 5000±3400 $ng{\cdot}cm^{-2}{\cdot}h^{-1}$, even more preferably 5000±3000 $ng{\cdot}cm^{-2}{\cdot}h^{-1}$, most preferably 5000±2600 $ng{\cdot}cm^{-2}{\cdot}h^{-1}$, and in particular 5000±2200 $ng{\cdot}cm^{-2}{\cdot}h^{-1}$.

In a preferred embodiment, the pharmaceutical patch according to the invention provides plasma concentrations of the pharmacologically active ingredient over a period of at least 6 hours, more preferably at least 12 hours upon repeated application to the human skin, i.e. under steady state conditions taking into account the depot effect of the skin, of at least 10 $pg{\cdot}ml^{-1}$, at least 25 $pg{\cdot}ml^{-1}$ or at least 50 $pg{\cdot}ml^{-1}$; more preferably at least 75 $pg{\cdot}ml^{-1}$, at least 100 $pg{\cdot}ml^{-1}$ or at least 150 $pg{\cdot}ml^{-1}$; still more preferably at least 200 $pg{\cdot}ml^{-1}$, at least 250 $pg{\cdot}ml^{-1}$ or at least 300 $pg{\cdot}ml^{-1}$; yet more preferably at least 350 $pg{\cdot}ml^{-1}$, at least 400 $pg{\cdot}ml^{-1}$ or at least 450 $pg{\cdot}ml^{-1}$; even more preferably at least 500 $pg{\cdot}ml^{-1}$, at least 550 $pg{\cdot}ml^{-1}$ or at least 600 $pg{\cdot}ml^{-1}$; most preferably at least 650 $pg{\cdot}ml^{-1}$, at least 700 $pg{\cdot}ml^{-1}$ or at least 750 $pg{\cdot}ml^{-1}$; and in particular at least 800 $pg{\cdot}ml^{-1}$, at least 850 $pg{\cdot}ml^{-1}$ or at least 900 $pg{\cdot}ml^{-1}$.

In a preferred embodiment, the pharmaceutical patch according to the invention provides plasma concentrations of the pharmacologically active ingredient over a period of at least 6 hours, more preferably at least 12 hours upon repeated application to the human skin, i.e. under steady state conditions taking into account the depot effect of the skin, of 10±8.0 $pg{\cdot}ml^{-1}$, more preferably 10±7.0 $pg{\cdot}ml^{-1}$, still more preferably 10±6.0 $pg{\cdot}ml^{-1}$, yet more preferably 10±5.0 $pg{\cdot}ml^{-1}$, even more preferably 10±4.0 $pg{\cdot}ml^{-1}$, most preferably 10±3.0 $pg{\cdot}ml^{-1}$, and in particular 10±2.0 $pg{\cdot}ml^{-1}$.

In another preferred embodiment, the pharmaceutical patch according to the invention provides plasma concentrations of the pharmacologically active ingredient over a period of at least 6 hours, more preferably at least 12 hours upon repeated application to the human skin, i.e. under steady state conditions taking into account the depot effect of the skin, of 20±16 $pg{\cdot}ml^{-1}$, more preferably 20±14 $pg{\cdot}ml^{-1}$, still more preferably 20±12 $pg{\cdot}ml^{-1}$, yet more preferably 20±10 $pg{\cdot}ml^{-1}$, even more preferably 20±8.0 $pg{\cdot}ml^{-1}$, most preferably 20±6.0 $pg{\cdot}ml^{-1}$, and in particular 20±4.0 $pg{\cdot}ml^{-1}$.

In still another preferred embodiment, the pharmaceutical patch according to the invention provides plasma concentrations of the pharmacologically active ingredient over a period of at least 6 hours, more preferably at least 12 hours upon repeated application to the human skin, i.e. under steady state conditions taking into account the depot effect of the skin, of 40±32 pg·ml$^{-1}$, more preferably 40±28 pg·ml$^{-1}$, still more preferably 40±24 pg·ml$^{-1}$, yet more preferably 40±20 pg·ml$^{-1}$, even more preferably 40±16 pg·ml$^{-1}$, most preferably 40±12 pg·ml$^{-1}$, and in particular 40±8.0 pg·ml$^{-1}$.

In yet another preferred embodiment, the pharmaceutical patch according to the invention provides plasma concentrations of the pharmacologically active ingredient over a period of at least 6 hours, more preferably at least 12 hours upon repeated application to the human skin, i.e. under steady state conditions taking into account the depot effect of the skin, of 75±64 pg·ml$^{-1}$, more preferably 75±56 pg·ml$^{-1}$, still more preferably 75±48 pg·ml$^{-1}$, yet more preferably 75±40 pg·ml$^{-1}$, even more preferably 75±32 pg·ml$^{-1}$, most preferably 75±24 pg·ml$^{-1}$, and in particular 75±16 pg·ml$^{-1}$.

In another preferred embodiment, the pharmaceutical patch according to the invention provides plasma concentrations of the pharmacologically active ingredient over a period of at least 6 hours, more preferably at least 12 hours upon repeated application to the human skin, i.e. under steady state conditions taking into account the depot effect of the skin, of 150±128 pg·ml$^{-1}$, more preferably 150±112 pg·ml$^{-1}$, still more preferably 150±96 pg·ml$^{-1}$, yet more preferably 150±80 pg·ml$^{-1}$, even more preferably 150±64 pg·ml$^{-1}$, most preferably 150±48 pg·ml$^{-1}$, and in particular 150±32 pg·ml$^{-1}$.

In still another preferred embodiment, the pharmaceutical patch according to the invention provides plasma concentrations of the pharmacologically active ingredient over a period of at least 6 hours, more preferably at least 12 hours upon repeated application to the human skin, i.e. under steady state conditions taking into account the depot effect of the skin, of 300±256 pg·ml$^{-1}$, more preferably 300±224 pg·ml$^{-1}$, still more preferably 300±192 pg·ml$^{-1}$, yet more preferably 300±160 pg·ml$^{-1}$, even more preferably 300±128 pg·ml$^{-1}$, most preferably 300±96 pg·ml$^{-1}$, and in particular 300±64 pg·ml$^{-1}$.

In yet another preferred embodiment, the pharmaceutical patch according to the invention provides plasma concentrations of the pharmacologically active ingredient over a period of at least 6 hours, more preferably at least 12 hours upon repeated application to the human skin, i.e. under steady state conditions taking into account the depot effect of the skin, of 600±512 pg·ml$^{-1}$, more preferably 600±448 pg·ml$^{-1}$, still more preferably 600±384 pg·ml$^{-1}$, yet more preferably 600±320 pg·ml$^{-1}$, even more preferably 600±256 pg·ml$^{-1}$, most preferably 600±192 pg·ml$^{-1}$, and in particular 600±128 pg·ml$^{-1}$.

In another preferred embodiment, the pharmaceutical patch according to the invention provides plasma concentrations of the pharmacologically active ingredient over a period of at least 6 hours, more preferably at least 12 hours upon repeated application to the human skin, i.e. under steady state conditions taking into account the depot effect of the skin, of at least 1.0 ng·ml$^{-1}$, at least 2.5 ng·ml$^{-1}$ or at least 5.0 ng·ml$^{-1}$; more preferably at least 7.5 ng·ml$^{-1}$, at least 10.0 ng·ml$^{-1}$ or at least 15.0 ng·ml$^{-1}$; still more preferably at least 20.0 ng·ml$^{-1}$, at least 25.0 ng·ml$^{-1}$ or at least 30.0 ng·ml$^{-1}$; yet more preferably at least 35.0 ng·ml$^{-1}$, at least 40.0 ng·ml$^{-1}$ or at least 45.0 ng·ml$^{-1}$; even more preferably at least 50.0 ng·ml$^{-1}$, at least 55.0 ng·ml$^{-1}$ or at least 60.0 ng·ml$^{-1}$; most preferably at least 65.0 ng·ml$^{-1}$, at least 70.0 ng·ml$^{-1}$ or at least 75.0 ng·ml$^{-1}$; and in particular at least 80.0 ng·ml$^{-1}$, at least 85.0 ng·ml$^{-1}$ or at least 90.0 ng·ml$^{-1}$.

The pharmaceutical patch according to the invention preferably contains a crystallization inhibitor which inhibits the crystallization of the pharmacologically active ingredient within the adhesive layer and drug layer, respectively. Thus, the crystallization inhibitor is preferably contained in the same layer as the pharmacologically active ingredient. Preferably, the content of the crystallization inhibitor within said layer is within the range of from 1.0 to 20 wt.-%, more preferably 2.5 to 17.5 wt.-%, still more preferably 5.0 to 15 wt.-%, yet more preferably 6.0 to 14 wt.-%, even more preferably 7.0 to 13 wt.-%, most preferably 8.0 to 12 wt.-%, and in particular 9.0 to 11 wt.-%, relative to the total weight of said layer. Preferred crystallization inhibitors include but are not limited to polyvinylpyrrolidones (povidone, polyvidone) (e.g. Kollidon 25), N-vinyl-1-aza-cycloheptan-2-one homopolymers, N-vinylpiperidin-2-one homopolymers, polyethylene glycol, poloxamer (e.g. Lutrol F127), and copovidone (e.g. Kollidon VA64).

Preferably, the molar ratio of the pharmacologically active ingredient to the crystallization inhibitor is within the range of from 1000 : 1 to 1 : 1000, more preferably 250 : 1 to 1 : 250, still more preferably 100 : 1 to 1 : 100, yet more preferably 50 : 1 to 1 : 50, even more preferably 25 : 1 to 1 : 25, most preferably 10 : 1 to 1 : 10, and in particular 5 : 1 to 1 : 5.

In a preferred embodiment, the molar ratio of the pharmacologically active ingredient to the crystallization inhibitor is within the range of 1 : 10±7 (i.e. from 1 : 3 to 1 : 17), more preferably 1 : 10±6, still more preferably 1 : 10±5, yet more preferably 1 : 10±4, even more preferably 1 : 10±3, most preferably 1 : 10±2, and in particular 1 : 10±1. In a preferred embodiment, the molar ratio of the pharmacologically active ingredient to the crystallization inhibitor is within the range of 1 : 20±14, more preferably 1 : 20±12, still more preferably 1 : 20±10, yet more preferably 1 : 20±8, even more preferably 1 : 20±6, most preferably 1 : 20±4, and in particular 1 : 20±2. In another preferred embodiment, the molar ratio of the pharmacologically active ingredient to the crystallization inhibitor is within the range of 1 : 45±37, more preferably 1 : 45±35, still more preferably 1 : 45±33, yet more preferably 1 : 45±31, even more preferably 1 : 45±29,

most preferably 1 : 45±27, and in particular 1 : 45±25. In still another preferred embodiment, the molar ratio of the pharmacologically active ingredient to the crystallization inhibitor is within the range of 1 : 70±42, more preferably 1 : 70±36, still more preferably 1 : 70±30, yet more preferably 1 : 70±24, even more preferably 1 : 70±18, most preferably 1 : 70±12, and in particular 1 : 70±6. In yet another preferred embodiment, the molar ratio of the pharmacologically active ingredient to the crystallization inhibitor is within the range of 1 : 90±70, more preferably 1 : 90±60, still more preferably 1 : 90±50, yet more preferably 1 : 90±40, even more preferably 1 : 90±30, most preferably 1 : 90±20, and in particular 1 : 90±10. In yet another preferred embodiment, the molar ratio of the pharmacologically active ingredient to the crystallization inhibitor is within the range of 1 : 110±70, more preferably 1 : 110±60, still more preferably 1 : 110±50, yet more preferably 1 : 110±40, even more preferably 1 : 110±30, most preferably 1 : 110±20, and in particular 1 : 110±10.

The pharmaceutical patch according to the invention preferably contains a permeation component which enhances percutaneous penetration and permeation of the pharmacologically active ingredient through human skin, i.e. one or more percutaneous penetration enhancers. Percutaneous penetration enhancers are known to the skilled person (cf., e.g., Smith et al., Percutaneous Penetration Enhancers, CRC Press, 1995).

Preferably, the layer of the pharmaceutical patch which contains the pharmacologically active ingredient, i.e. the adhesive layer and/or the drug layer, contains at least one percutaneous penetration enhancer.

Preferably, the relative weight ratio of the pharmacologically active ingredient to the permeation component is within the range of from 25 : 1 to 1 : 1000, more preferably 10 : 1 to 1 : 250, still more preferably 5 : 1 to 1 : 100, yet more preferably 1 : 1 to 1 : 50, even more preferably 1 : 2 to 1 : 25, most preferably 1 : 6 to 1 : 20, and in particular 1 : 9 to 1 : 17. Preferably, the molar ratio of the pharmacologically active ingredient to the permeation component is within the range of from 1000 : 1 to 1 : 1000, more preferably 250 : 1 to 1 : 250, still more preferably 100 : 1 to 1 : 100, yet more preferably 50 : 1 to 1 : 50, even more preferably 25 : 1 to 1 : 25, most preferably 10 : 1 to 1 : 10, and in particular 5 : 1 to 1 : 5.

In a preferred embodiment, the molar ratio of the pharmacologically active ingredient to the permeation component is within the range of 1 : 20±14 (i.e. from 1 : 6 to 1 : 34), more preferably 1 : 20±12, still more preferably 1 : 20±10, yet more preferably 1 : 20±8, even more preferably 1 : 20±6, most preferably 1 : 20±4, and in particular 1 : 20±2. In another preferred embodiment, the molar ratio of the pharmacologically active ingredient to the permeation component is within the range of 1 : 45±37, more preferably 1 : 45±35, still more preferably 1 : 45±33, yet more preferably 1 : 45±31, even more preferably 1 : 45±29, most preferably 1 : 45±27, and in particular 1 : 45±25. In still another preferred embodiment, the molar ratio of the pharmacologically active ingredient to the permeation component is within the range of 1 : 70±42, more preferably 1 : 70±36, still more preferably 1 : 70±30, yet more preferably 1 : 70±24, even more preferably 1 : 70±18, most preferably 1 : 70±12, and in particular 1 : 70±6. In yet another preferred embodiment, the molar ratio of the pharmacologically active ingredient to the permeation component is within the range of 1 : 90±70, more preferably 1 : 90±60, still more preferably 1 : 90±50, yet more preferably 1 : 90±40, even more preferably 1 : 90±30, most preferably 1 : 90±20, and in particular 1 : 90±10. In yet another preferred embodiment, the molar ratio of the pharmacologically active ingredient to the permeation component is within the range of 1 : 110±70, more preferably 1 : 110±60, still more preferably 1 : 110±50, yet more preferably 1 : 110±40, even more preferably 1 : 110±30, most preferably 1 : 110±20, and in particular 1 : 110±10.

Preferably, the permeation component is contained in the same layer of the pharmaceutical patch that also contains the pharmacologically active ingredient or at least a portion thereof. Preferably, the content of the permeation component within said layer is within the range of from 1.0 to 20 wt.-%, more preferably 2.5 to 17.5 wt.-%, still more preferably 5.0 to 15 wt.-%, yet more preferably 6.0 to 14 wt.-%, even more preferably 7.0 to 13 wt.-%, most preferably 8.0 to 12 wt.-%, and in particular 9.0 to 11 wt.-%, relative to the total weight of said layer.

In a preferred embodiment, the permeation component comprises at least one percutaneous penetration enhancer having a HLB value (hydrophilic-lipophilic balance) within the range of 4.0±3.5 (i.e. from 0.5 to 7.5), more preferably 4.0±3.0, still more preferably 4.0±2.5, yet more preferably 4.0±2.0, even more preferably 4.0±1.5, most preferably 4.0±1.0, and in particular 4.0±0.5. In another preferred embodiment, the permeation component comprises at least one percutaneous penetration enhancer having a HLB value (hydrophilic-lipophilic balance) within the range of 8±7, more preferably 8±6, still more preferably 8±5, yet more preferably 8±4, even more preferably 8±3, most preferably 8±2, and in particular 8±1. In still another preferred embodiment, the permeation component comprises at least one percutaneous penetration enhancer having a HLB value (hydrophilic-lipophilic balance) within the range of 12±7, more preferably 12±6, still more preferably 12±5, yet more preferably 12±4, even more preferably 12±3, most preferably 12±2, and in particular 12±1. In yet another preferred embodiment, the permeation component comprises at least one percutaneous penetration enhancer having a HLB value (hydrophilic-lipophilic balance) within the range of 16±7, more preferably 16±6, still more preferably 16±5, yet more preferably 16±4, even more preferably 16±3, most preferably 16±2, and in particular 16±1. In another preferred embodiment, the permeation component comprises at least one percutaneous penetration enhancer having a HLB value (hydrophilic-lipophilic balance) within the range of 20±7, more preferably 20±6, still more preferably 20±5, yet more preferably 20±4, even more preferably 20±3, most preferably

20±2, and in particular 20±1. In another preferred embodiment, the permeation component comprises at least one percutaneous penetration enhancer having a HLB value (hydrophilic-lipophilic balance) within the range of 24±7, more preferably 24±6, still more preferably 24±5, yet more preferably 24±4, even more preferably 24±3, most preferably 24±2, and in particular 24±1.

Preferred percutaneous penetration enhancers include but are not limited to:

a) sulfoxides such as dimethylsulfoxide (DMSO) and decylmethylsulfoxide;

b) ethers such as diethylene glycol monoethyl ether (transcutol) and diethylene glycol monomethyl ether;

c) surfactants such as sodium laurate, sodium lauryl sulfate, cetyltrimethylammonium bromide, benzalkonium chloride, poloxamers, polysorbates (e.g. polysorbate 80) and lecithin;

d) 1 -substituted azacycloheptan-2-ones such as 1-n-dodecylcyclazacycloheptan-2-one;

e) alcohols and fatty alcohols such as ethanol, propanol, octanol, dodecanol, oleyl alcohol, benzyl alcohol, and the like;

f) polyols, esters of polyols and ethers of polyols such as propylene glycol, ethylene glycol, diethylene glycol, dipropylene glycol, glycerol, butanediol, polyethylene glycol, polyvinyl alcohol (e.g. Mowiol 4-88), triacetine and polyethylene glycol monolaurate;

g) organic acids such as salicylic acid and salicylates, citric acid, levulinic acid, caprylic acid and succinic acid; as well as dicarboxylic acids and their esters such as dibutylene sebacate;

h) fatty acids such as lauric acid, oleic acid and valeric acid; fatty acid esters such as isopropyl myristate, isopropyl palmitate, methylpropionate, propylene glycol monolaureate, lauryl lactate, oleyl oleate and ethyl oleate;

i) amides and other nitrogenous compounds such as urea, dimethylacetamide, dimethylformamide, 2-pyrrolidone, 1-methyl-2-pyrrolidone, ethanolamine, diethanolamine, triethanolamine and laurocapram (Azone®);

j) terpenes;

k) alkanones;

l) other oligomers or polymers;

and mixtures of any of the foregoing.

Preferably, the permeation component comprises as percutaneous penetration enhancer a non-cyclic compound of formula $C_{2n}H_{4n+2}O_n$, where index n is 2, 3 or 4; preferably diethylene glycol monomethylether, dipropylene glycol or a mixture thereof.

Preferably, the permeation component comprises one or more percutaneous penetration enhancers selected from transcutol (diethylene glycol monoethylether), oleyl alcohol, dipropylene glycol, levulinic acid and mixtures thereof.

Preferably, the pharmaceutical patch has an area of at least 5 $cm^2$, more preferably at least 7,5 $cm^2$, still more preferably at least 10 $cm^2$, and most preferably at least 15 $cm^2$.

In a preferred embodiment, the pharmaceutical patch has an area, i.e. total surface area when being applied to the skin, within the range of 200±150 $cm^2$ (i.e. from 50 $cm^2$ to 350 $cm^2$), more preferably 200±125 $cm^2$, still more preferably 200±100 $cm^2$, yet more preferably 200±75 $cm^2$, even more preferably 150±50 $cm^2$, most preferably 150±25 $cm^2$, and in particular 150±10 $cm^2$. In another preferred embodiment, the pharmaceutical patch has an area within the range of 300±150 $cm^2$, more preferably 300±125 $cm^2$, still more preferably 300±100 $cm^2$, yet more preferably 300±75 $cm^2$, even more preferably 300±50 $cm^2$, most preferably 300±25 $cm^2$, and in particular 300±10 $cm^2$. In still another preferred embodiment, the pharmaceutical patch has an area within the range of 400±150 $cm^2$, more preferably 400±125 $cm^2$, still more preferably 400±100 $cm^2$, yet more preferably 400±75 $cm^2$, even more preferably 400±50 $cm^2$, most preferably 400±25 $cm^2$, and in particular 400±10 $cm^2$.

In a preferred embodiment, the pharmaceutical patch has an area, i.e. total surface area when being applied to the skin, within the range of 25±20 $cm^2$, more preferably 25±15 $cm^2$, still more preferably 25±10 $cm^2$. In another preferred embodiment, the pharmaceutical patch has an area, i.e. total surface area when being applied to the skin, within the range of 50±40 $cm^2$, more preferably 50±35 $cm^2$, still more preferably 50±30 $cm^2$, yet more preferably 50±25 $cm^2$, even more preferably 50±20 $cm^2$, most preferably 50±15 $cm^2$, and in particular 50±10 $cm^2$. In still another preferred

embodiment, the pharmaceutical patch has an area within the range of 75±40 $cm^2$, more preferably 75±35 $cm^2$, still more preferably 75±30 $cm^2$, yet more preferably 75±25 $cm^2$, even more preferably 75±20 $cm^2$, most preferably 75±15 $cm^2$, and in particular 75±10 $cm^2$. In yet another preferred embodiment, the pharmaceutical patch has an area within the range of 100±80 $cm^2$, more preferably 100±60 $cm^2$, still more preferably 100±50 $cm^2$, yet more preferably 100±40 $cm^2$, even more preferably 100±30 $cm^2$, most preferably 100±20 $cm^2$, and in particular 100±10 $cm^2$. In another preferred embodiment, the pharmaceutical patch has an area within the range of 150±80 $cm^2$, more preferably 150±60 $cm^2$, still more preferably 150±50 $cm^2$, yet more preferably 150±40 $cm^2$, even more preferably 150±30 $cm^2$, most preferably 150±20 $cm^2$, and in particular 150±10 $cm^2$. In another preferred embodiment, the pharmaceutical patch has an area within the range of 200±80 $cm^2$, more preferably 200±60 $cm^2$, still more preferably 200±50 $cm^2$, yet more preferably 200±40 $cm^2$, even more preferably 200±30 $cm^2$, most preferably 200±20 $cm^2$, and in particular 200±10 $cm^2$. In another preferred embodiment, the pharmaceutical patch has an area within the range of 250±80 $cm^2$, more preferably 250±60 $cm^2$, still more preferably 250±50 $cm^2$, yet more preferably 250±40 $cm^2$, even more preferably 250±30 $cm^2$, most preferably 250±20 $cm^2$, and in particular 250±10 $cm^2$.

The pharmaceutical patch according to the invention comprises a surface layer.

The term "surface layer" as used herein refers to any layer that represents the surface layer after the application of the pharmaceutical patch. This definition includes permanent backing layer commonly used for pharmaceutical patches as well as thin non-removable films that are typically used in thin flexible patches.

In a preferred embodiment, the surface layer comprises one or more polymers selected from the group consisting of polyurethanes, polyester elastomers, polyether block amides, polyacrylates, ethylene vinyl acetates, ethylene acrylate copolymers, ionomer resins, polyvinyl chloride, polyvinylidene chloride, polyesters and polyolefins, such as polyethylene; polyolefins, in particular polyethylene, polyesters, ethylene vinylacetate copolymers and polyurethanes are particularly preferred.

The surface layer may be a laminate, preferably comprising a polymer film, such as a polyester film, and aluminum foil and/or heat seal layer.

In a preferred embodiment, the surface layer consists of a polyester film and an ethylene vinylacetate copolymer heat seal layer.

The thickness of the surface layer is not particularly limited. Preferably, the surface layer has a thickness within the range of from 0.1 to 5000 μm. In a preferred embodiment, the surface layer has a thickness within the range of from 0.5 to 1000 μm, more preferably from 1 to 750 μm, still more preferably from 5 to 500 μm, most preferably from 10 to 250 μm, and in particular from 20 to 150 μm or from 40 to 100 μm.

In a preferred embodiment, the surface layer has a thickness within the range of 25±20 μm (i.e. from 5 μm to 45 μm), more preferably 25±15 μm, still more preferably 25±10 μm, and yet more preferably 25±5 μm.

The pharmaceutical patch according to the invention comprises a removable protective layer (release liner).

Preferably, the removable protective layer comprises a polymer film and a silicone coating or fluoropolymer coating. Preferably, the polymer film is a polyolefin, in particular polyethylene or polypropylene film or polyester, in particular polyethylene terephthalate film.

In a preferred embodiment, the removable protective layer is a silicone coated polyolefin or silicone coated polyester film, such as a silicone coated polyethylene terephthalate, polypropylene or polyethylene film.

In another preferred embodiment, the removable protective layer is a fluoropolymer coated polyolefin or polyester film, such as a fluoropolymer coated polyethylene terephthalate, polypropylene or polyethylene film.

The thickness of the removable protective layer is not particularly limited. Preferably, the removable protective layer has a thickness within the range of from 0.1 to 500 μm. In a preferred embodiment, the removable protective layer has a thickness within the range of from 0.5 to 400 μm, more preferably from 1 to 300 μm, still more preferably from 5 to 250 μm, most preferably from 10 to 200 μm, and in particular from 20 to 150 μm or from 40 to 100 μm.

In a preferred embodiment, the removable protective layer has a thickness within the range of 75±70 μm (i.e. from 5 μm to 145 μm), more preferably 75±60 μm, still more preferably 75±50 μm, yet more preferably 75±40 μm, even more preferably 75±30 μm, most preferably 75±20 μm, and in particular 75±10 μm. In another preferred embodiment, the removable protective layer has a thickness within the range of 100±70 μm, more preferably 100±60 μm, still more preferably 100±50 μm, yet more preferably 100±40 μm, even more preferably 100±30 μm, most preferably 100±20 μm, and in particular 100±10 μm.

The pharmaceutical patch according to the invention comprises an adhesive layer.

In a preferred embodiment, the adhesive layer comprises at least a portion of the total amount of the pharmacologically active ingredient that is contained in the pharmaceutical patch. Preferably, the adhesive layer comprises at least 10 wt.-%, more preferably at least 25 wt.-%, still more preferably at least 50 wt.-%, yet more preferably at least 75 wt.-%, most preferably at least 90 wt.-%, and in particular at least 95 wt.-% of the total amount of the pharmacologically active ingredient that is contained in the pharmaceutical patch.

In another preferred embodiment, the adhesive layer does not contain the pharmacologically active ingredient. According to this embodiment, the pharmaceutical patch comprises an additional drug layer that in turn contains the

total amount or at least a portion of the pharmacologically active ingredient, taking into account that after manufacture there typically is an exchange of the pharmacologically active ingredient between adjacent layers until an equilibrium has been reached.

Preferably, the adhesive layer comprises a polymer that forms a matrix in which the pharmacologically active ingredient is dispersed (drug-in-adhesive). The adhesive layer comprises a polyacrylate based pressure sensitive adhesive. The thickness of the adhesive layer is not particularly limited and may depend upon a number of factors such as function within the patch (e.g. drug-in-adhesive), content of pharmacologically active ingredient and excipients, prescribed duration of application of pharmaceutical patch on the skin, and the like.

Preferably, the adhesive layer has a thickness within the range of from 1.0 to 1000 μm.

In a preferred embodiment, the adhesive layer has a thickness within the range of from 50±35 μm (i.e. from 15 μm to 85 μm), more preferably 50±30 μm, still more preferably 50±25 μm, yet more preferably 50±20 μm, even more preferably 50±15 μm, most preferably 50±10 μm, and in particular 50±5 μm. In another preferred embodiment, the adhesive layer has a thickness within the range of from 75±70 μm, more preferably 75±60 μm, still more preferably 75±50 μm, yet more preferably 75±40 μm, even more preferably 75±30 μm, most preferably 75±20 μm, and in particular 75±10 μm. In still another preferred embodiment, the adhesive layer has a thickness within the range of from 100±70 μm, more preferably 100±60 μm, still more preferably 100±50 μm, yet more preferably 100±40 μm, even more preferably 100±30 μm, most preferably 100±20 μm, and in particular 100±10 μm. In yet another preferred embodiment, the adhesive layer has a thickness within the range of from 200±175 μm, more preferably 200±150 μm, still more preferably 200±125 μm, yet more preferably 200±100 μm, even more preferably 200±75 μm, most preferably 200±50 μm, and in particular 200±25 μm. In another preferred embodiment, the adhesive layer has a thickness within the range of from 300±175 μm, more preferably 300±150 μm, still more preferably 300±125 μm, yet more preferably 300±100 μm, even more preferably 300±75 μm, most preferably 300±50 μm, and in particular 300±25 μm. In still another preferred embodiment, the adhesive layer has a thickness within the range of from 400±175 μm, more preferably 400±150 μm, still more preferably 400±125 μm, yet more preferably 400±100 μm, even more preferably 400±75 μm, most preferably 400±50 μm, and in particular 400±25 μm. In yet another preferred embodiment, the adhesive layer has a thickness within the range of from 500±175 μm, more preferably 500±150 μm, still more preferably 500±125 μm, yet more preferably 500±100 μm, even more preferably 500±75 μm, most preferably 500±50 μm, and in particular 500±25 μm.

Preferably, particularly when the adhesive layer contains the pharmacologically active ingredient or a portion thereof, the area weight of the adhesive layer is within the range of from 1.0 to 160 g/$m^2$, more preferably 5.0 to 125 g/$m^2$ or 45 to 155 g/$m^2$, still more preferably 10 to 100 g/$m^2$ or 55 to 145 g/$m^2$, yet more preferably 20 to 90 g/$m^2$ or 65 to 135 g/$m^2$, even more preferably 30 to 80 g/$m^2$ or 75 to 125 g/$m^2$, most preferably 40 to 70 g/$m^2$ or 85 to 115 g/$m^2$, and in particular 50 to 60 g/$m^2$ or 95 to 105 g/$m^2$.

In preferred embodiments a comparatively low area weight may positively influence the shelf-life of the pharmaceutical patch.

The ratio of the thickness of the surface layer to the thickness of the adhesive layer is not particularly limited. In a preferred embodiment, the thickness of the surface layer is greater than the thickness of the adhesive layer. In another preferred embodiment, the thickness of the adhesive layer is greater than the thickness of the surface layer.

In a preferred embodiment, the adhesive layer provides a peel strength of 5.5±5.0 N/25 mm, more preferably 5.5±4.5 N/25 mm, still more preferably 5.5±4.0 N/25 mm, yet more preferably 5.5±3.5 N/25 mm, even more preferably 5.5±3.0 N/25 mm, most preferably 5.5±2.5 N/25 mm, and in particular 5.5±2.0 N/25 mm.

In another preferred embodiment, the adhesive layer provides a peel strength of 2.0±1.8 N/25 mm, more preferably 2.0±1.6 N/25 mm, still more preferably 2.0±1.4 N/25 mm, yet more preferably 2.0±1.2 N/25 mm, even more preferably 2.0±1.0 N/25 mm, most preferably 2.0±0.8 N/25 mm, and in particular 2.0±0.6 N/25 mm.

Preferably, the peel test is performed as further specified in the experimental section.

In a preferred embodiment, the pressure sensitive adhesive is a polysilicone based pressure sensitive adhesive. Preferably, said polysilicone based pressure sensitive adhesive forms a matrix in which the pharmacologically active ingredient is embedded. Polysilicone based pressure sensitive adhesives are commercially available, e.g. under the trademarks BIO-PSA 7-4301, BIO-PSA 7-4302, BIO-PSA 7-4302/3, BIO-PSA 7-4201, BIO-PSA 7-4202, BIO-PSA 7-4101, BIO-PSA 7-4102, BIO-PSA 7-4601, BIO-PSA 7-4602, BIO-PSA 7-4602/3, BIO-PSA 7-4501, BIO-PSA 7-4502, BIO-PSA 7-4503, BIO-PSA 7-4401 and BIO-PSA 7-4402 by Dow Corning Corporation. The polysilicone based pressure sensitive adhesive preferably contains a solvent such as ethyl acetate or heptane and has a solids content of approx. 55-65 wt.-% solids before being dried during the preparation of the plaster. Especially preferred polysilicone based adhesives are commercially available by Dow Corning Corporation under the trademarks BIO PSA 7-4501 (solvent: heptane); BIO PSA 7-4502 (solvent: ethyl acetate); and BIO PSA 7-4503 (solvent: toluene).

In a preferred embodiment, the pressure sensitive adhesive is a polysilicone based pressure sensitive adhesive that is supplied in heptane or ethyl acetate. These solvents are typically removed during the manufacture of the pharmaceutical patch, though residual traces of solvent may be analytically detectable.

Preferably, the silicone polymers contained in the polysilicone based pressure sensitive adhesives are produced through a condensation reaction of a silanol endblocked polydimethylsiloxane (PDMS) with a silicate resin.

Preferably, the polysilicone based pressure sensitive adhesive provides a peel adhesion, preferably measured in accordance with Dow Corning Corp. corporate test method 0964A, of 300±200 g/cm (i.e. from 100 g/cm to 500 g/cm), more preferably 300±100 g/cm, still more preferably 300±50 g/cm; or 400±200 g/cm, more preferably 400±100 g/cm, still more preferably 400±50 g/cm; or 500±200 g/cm, more preferably 500±100 g/cm, still more preferably 500±50 g/cm; or 600±200 g/cm, more preferably 600±100 g/cm, still more preferably 600±50 g/cm; or 700±200 g/cm, more preferably 700±100 g/cm, still more preferably 700±50 g/cm; or 800±200 g/cm, more preferably 800±100 g/cm, still more preferably 800±50 g/cm; or 900±200 g/cm, more preferably 900±100 g/cm, still more preferably 900±50 g/cm.

In preferred embodiments polysilicone based pressure sensitive adhesives stabilize the pharmacologically active ingredient with respect to the formation of undesired degradation products. Formation of said degradation products can be suppressed especially when the concentration of the pharmacologically active ingredient in the adhesive layer that comprises the polysilicone based pressure sensitive adhesive is comparatively low.

According to this embodiment, the concentration of the pharmacologically active ingredient in the adhesive layer that comprises the polysilicone based pressure sensitive adhesive is preferably at most 1.00 wt.-%, more preferably at most 0.80 wt.-%, still more preferably at most 0.70 wt.-%, yet more preferably at most 0.60 wt.-%, even more preferably at most 0.50 wt.-%, most preferably at most 0.40 wt.-%, and in particular at most 0.30 wt.-%, relative to the total weight of the adhesive layer (total per dry unit).

According to this embodiment, the adhesive layer preferably contains the pharmacologically active ingredient, the polysilicone based pressure sensitive adhesive and optional auxiliary substances (excipients) such as one or more percutaneous penetration enhancers, antioxidants, and the like.

According to this embodiment, the adhesive layer comprises the polysilicone based pressure sensitive adhesive preferably in combination with a permeation component, preferably comprising transcutol (diethylene glycol monoethylether), optionally in combination with dipropylene glycol. Preferably, the content of the transcutol and the optionally present dipropylene glycol is in each case independently of one another within the range of from 0.1 to 20 wt.-%, more preferably 0.2 to 15 wt.-%, still more preferably 0.5 to 10 wt.-%, yet more preferably 1.0 to 9.0 wt.-%, even more preferably 2.0 to 8.0 wt.-%, most preferably 3.0 to 7.0 wt.-%, and in particular 4.0 to 6.0 wt.-%, relative to the total weight of the adhesive layer. When dipropylene glycol is present as additional percutaneous penetration enhancer besides transcutol, the relative weight ratio of dipropylene glycol to transcutol is preferably within the range of from 10:1 to 1:10, more preferably 7.5:1 to 1:7.5, still more preferably 5:1 to 1:5, yet more preferably 4:1 to 1:4, even more preferably 3:1 to 1:3, most preferably 2:1 to 1:2, and in particular 1.5:1 to 1:1.5.

Preferred compositions of adhesive layers that comprise polysilicone based pressure sensitive adhesives are summarized as embodiments $A^1$ to $A^{12}$ in the table here below (all values as percentages relative to the total weight of the adhesive layer, total per dry unit):

| [wt.%] | $A^1$ | $A^2$ | $A^3$ | $A^4$ | $A^5$ | $A^6$ |
|---|---|---|---|---|---|---|
| pharmacologically active ingredient (free base) | 0.01-1.00 | 0.03-0.95 | 0.04-0.95 | 0.05-0.90 | 0.06-0.90 | 0.08-0.85 |
| silicone based pressure sensitive adhesive | 59.00-99.99 | 64.05-99.97 | 69.05-99.96 | 71.10-99.95 | 73.10-99.94 | 75.15-99.92 |
| percutaneous penetration enhancer(s) | 0-30.00 | 0-25.00 | 0-20.00 | 0-18.00 | 0-16.00 | 0-14.00 |
| other excipients | 0-10.00 | 0-10.00 | 0-10.00 | 0-10.00 | 0-10.00 | 0-10.00 |
| | | | | | | |
| | $A^7$ | $A^8$ | $A^9$ | $A^{10}$ | $A^{11}$ | $A^{12}$ |
| pharmacologically active ingredient (free base) | 0.10-0.80 | 0.12-0.70 | 0.14-0.60 | 0.16-0.50 | 0.18-0.40 | 0.20-0.30 |
| silicone based pressure sensitive adhesive | 78.17-98.80 | 81.30-97.38 | 83.40-95.86 | 85.50-93.84 | 86.60-92.82 | 87.70-91.80 |

(continued)

| | $A^7$ | $A^8$ | $A^9$ | $A^{10}$ | $A^{11}$ | $A^{12}$ |
|---|---|---|---|---|---|---|
| percutaneous penetration enhancer(s) | 0.10-12.00 | 0.50-10.00 | 1.00-9.00 | 2.00-8.00 | 3.00-7.00 | 4.00-6.00 |
| other excipients | 1.00-9.00 | 2.00-8.00 | 3.00-7.00 | 4.00-6.00 | 4.00-6.00 | 4.00-6.00 |

It has been unexpectedly found that at concentrations within the range of from 0.20 to 0.30 wt.-%, relative to the total weight of the adhesive layer containing a silicone polymer (total per dry unit), the pharmacologically active ingredient can be formulated in dispersed form that exhibits a satisfactory shelf-life, i.e. that does not tend to recrystallize, and provides acceptable flux rates.

In another preferred embodiment, the pressure sensitive adhesive is an polyacrylate based pressure sensitive adhesive. Preferably, said polyacrylate based pressure sensitive adhesive forms a matrix in which the pharmacologically active ingredient is embedded. Polyacrylate based pressure sensitive adhesives are commercially available, e.g. under the trademark DuroTAK®, especially the 87 series, e.g. Duro-Tak® 87-202A; Duro-Tak® 87-208A; Duro-Tak® 87-502A; Duro-Tak® 87-503A; Duro-Tak® 87-2051; Duro-Tak® 87-2054; Duro-Tak® 87-2287; Duro-Tak® 87-2353; Duro-Tak® 87-2510; Duro-Tak® 87-2516; Duro-Tak® 87-4098; Duro-Tak® 87-4287; Duro-Tak® 87-9088; and Duro-Tak® 87-9301; or Duro-Tak® 387-2516.

When the pressure sensitive adhesive is an polyacrylate based pressure sensitive adhesive, e.g. Duro-Tak® 87-4287, it can contain a cationic copolymer based on dimethylaminoethyl methacrylate, butyl methacrylate, and methyl methacrylate, e.g. EUDRAGIT® E PO. According to this embodiment, the weight amount of EUDRAGIT® E PO is preferably 0.01 to 70 wt.-%, more preferably 10±9 wt.-%, or 20±10 wt.-%, or 30±10 wt.-%, or 40±10 wt.-%, or 50±10 wt.-% relative to the total combined weight of the acrylate pressure sensitive adhesive and the EUDRAGIT® E PO.

The polyacrylate based pressure sensitive adhesive may contain one or more acrylate homopolymers or one or more acrylate copolymers or mixtures thereof.

For the purpose of the specification, "(meth)acryl" shall refer to both, methacryl as well as acryl.

In a preferred embodiment, the adhesive layer comprises an acrylate copolymer comprising monomer units originating from monomers A which are selected from $C_{1-18}$-alkyl (meth)acrylates and monomers B which are copolymerizable with monomers A. Thus, the acrylate copolymer is derived from at least one monomer of the type of monomers A and at least one monomer of the type of monomers B.

In a preferred embodiment, the acrylate copolymer is derived from two different monomers (bipolymer), three different monomers (terpolymer) or four different monomers (quaterpolymer). Terpolymers are particularly preferred.

Preferred monomers A are selected from the group consisting of methyl (meth)acrylate, ethyl (meth)acrylate, propyl (meth)acrylate, butyl (meth)acrylate, pentyl (meth)acrylate, hexyl (meth)acrylate, cyclohexyl (meth)acrylate, octyl (meth)acrylate, isobornyl (meth)acrylate, and mixtures thereof. 2-Ethylhexyl (meth)acrylate is a preferred representative of an octyl (meth)acrylate.

Preferred monomers B are selected from the group consisting of 2-hydroxyethyl (meth)-acrylate, glyceryl mono(meth)acrylate, glycidyl (meth)acrylate, acrylamide, N,N-diethyl-(meth)acrylamide, 2-ethoxyethyl (meth)acrylate, 2-ethoxyethoxyethyl (meth)acrylate, tetrahydrofuryl (meth)acrylate, vinyl acetate, N-vinyl pyrrolidone and mixtures thereof.

In a preferred embodiment, the acrylate copolymer is derived from a monomer composition comprising monomer units having at least one hydroxyl functional group, preferably selected from 2-hydroxyethyl (meth)acrylate and glyceryl mono(meth)acrylate.

In a particularly preferred embodiment, the acrylate copolymer is derived from a monomer composition comprising vinyl acetate, 2-ethylhexyl acrylate and 2-hydroxyethyl acrylate (terpolymer), optionally also comprising glycidyl methacrylate (quaterpolymer).

In another preferred embodiment, the acrylate copolymer that is contained in the adhesive layer does not comprise any monomer units having free hydroxyl functional groups.

Preferred embodiments $B^1$ to $B^8$ of acrylate copolymers that are preferably contained in the adhesive layer are summarized in the table here below:

| [wt.-%] | $B^1$ | $B^2$ | $B^3$ | $B^4$ | $B^5$ | $B^6$ | $B^7$ | $B^8$ |
|---|---|---|---|---|---|---|---|---|
| vinyl acetate | 5-55 | 10-50 | 15-45 | 17-40 | 17-40 | 17-40 | 17-40 | 17-40 |
| 2-ethylhexyl acrvlate | 45-95 | 50-90 | 55-85 | 60-80 | 55-75 | 55-75 | 55-75 | 55-75 |
| 2-hydroxyethyl acrylate | 0-10 | 0-9.0 | 0-8.0 | 0-7.0 | 1.0-9.0 | 2.0-8.0 | 3.0-7.0 | 4.0-6.0 |

(continued)

| [wt.-%] | B1 | B2 | B3 | B4 | B5 | B6 | B7 | B8 |
|---|---|---|---|---|---|---|---|---|
| glycidyl methacrylate | 0-5.0 | 0-4.0 | 0-3.0 | 0-2.0 | - | - | - | - |

It has been found that different acrylate copolymers, i.e. acrylate copolymers originating from different comonomers and/or different relative amounts of comonomers, have a different influence on the chemical stability of the pharmacologically active ingredient, its maximal concentration in dispersed form at a sufficient shelf-life as well as its flux rate.

According to this embodiment, the concentration of the pharmacologically active ingredient in the adhesive layer that comprises the polyacrylate based pressure sensitive adhesive is preferably at most 1.10 wt.-%, more preferably at most 1.05 wt.-%, still more preferably at most 1.00 wt.-%, yet more preferably at most 0.95 wt.-%, even more preferably at most 0.90 wt.-%, most preferably at most 0.85 wt.-%, and in particular at most 0.80 wt.-%, relative to the total weight of the adhesive layer (total per dry unit). According to this embodiment, the concentration of the pharmacologically active ingredient in the adhesive layer that comprises the polyacrylate based pressure sensitive adhesive is preferably at least 0.35 wt.-%, more preferably at least 0.40 wt.-%, still more preferably at least 0.45 wt.-%, yet more preferably at least 0.55 wt.-%, even more preferably at least 0.60 wt.-%, most preferably at least 0.65 wt.-%, and in particular at least 0.70 wt.-%, relative to the total weight of the adhesive layer (total per dry unit).

According to this embodiment, the adhesive layer preferably contains the pharmacologically active ingredient, the polyacrylate based pressure sensitive adhesive and optional auxiliary substances (excipients) such as one or more percutaneous penetration enhancers, antioxidants, and the like.

According to this embodiment, the adhesive layer comprises the polyacrylate based pressure sensitive adhesive preferably in combination with a crystallization inhibitor, preferably comprising polyvinylpyrrolidone (e.g. Kollidon 25), and/or in combination with a permeation component, preferably comprising dipropylene glycol, optionally in combination with or oleyl alcohol. The combination of dipropylene glycol (permeation enhancer) with polyvinylpyrrolidone (crystallization inhibitor) is most preferred. Preferably, the content of the dipropylene glycol and the optionally present polyvinylpyrrolidone and oleyl alcohol, respectively, is in each case independently of one another within the range of from 0.1 to 20 wt.-%, more preferably 0.2 to 15 wt.-%, still more preferably 0.5 to 10 wt.-%, yet more preferably 1.0 to 9.0 wt.-%, even more preferably 2.0 to 8.0 wt.-%, most preferably 3.0 to 7.0 wt.-%, and in particular 4.0 to 6.0 wt.-%, relative to the total weight of the adhesive layer. When polyvinylpyrrolidone is present as crystallization inhibitor and/or oleyl alcohol is present as additional percutaneous penetration enhancer besides dipropylene glycol, the relative weight ratio of dipropylene glycol to polyvinylpyrrolidone and oleyl alcohol, respectively, is preferably within the range of from 10:1 to 1:10, more preferably 7.5:1 to 1:7.5, still more preferably 5:1 to 1:5, yet more preferably 4:1 to 1:4, even more preferably 3:1 to 1:3, most preferably 2:1 to 1:2, and in particular 1.5:1 to 1:1.5.

It has been surprisingly found that the combination of dipropylene glycol as permeation enhancer and polyvinyl pyrrolidone (Kollidon 25) as crystallization inhibitor provides particularly beneficial permeation performance of the pharmacologically active ingredient through the skin, particularly in adhesive layers containing polyacrylate based pressure sensitive adhesives.

Preferred compositions of adhesive layers that comprise polyacrylate based pressure sensitive adhesives are summarized as embodiments $C^1$ to $C^{12}$ in the table here below (all values as percentages relative to the total weight of the adhesive layer, total per dry unit):

| [wt.%] | C1 | C2 | C3 | C4 | C5 | C6 |
|---|---|---|---|---|---|---|
| pharmacologically active ingredient (free base) | 0.15-1.35 | 0.17-1.30 | 0.25-1.25 | 0.30-1.20 | 0.35-1.15 | 0.40-1.10 |
| acrylate based pressure sensitive adhesive | 35.65-99.85 | 42.17-99.80 | 48.75-99.75 | 55.30-98.70 | 61.85-98.15 | 63.40-97.50 |
| percutaneous penetration enhancer(s) | 0-45.00 | 0-40.00 | 0-35.00 | 1.00-30.00 | 1.50-25.00 | 2.00-20.00 |
| other excipients | 0-18.00 | 0-16.50 | 0-15.00 | 0-13.50 | 0-12.00 | 0-10.50 |
| | | | | | | |

(continued)

| [wt.%] | $C^1$ | $C^2$ | $C^3$ | $C^4$ | $C^5$ | $C^6$ |
|---|---|---|---|---|---|---|
| | $C^7$ | $C^8$ | $C^9$ | $C^{10}$ | $C^{11}$ | $C^{12}$ |
| pharmacologically active | 0.45-1.05 | 0.50-1.00 | 0.55-0.95 | 0.60-0.90 | 0.65-0.85 | 0.70-0.80 |
| ingredient (free base) | | | | | | |
| acrylate based pressure sensitive adhesive | 72.45-96.55 | 76.50-94.00 | 79.05-92.95 | 81.60-91.90 | 84.15-90.85 | 86.70-89.80 |
| percutaneous penetration enhancer(s) | 2.50-17.50 | 5.00-15.00 | 6.00-14.00 | 7.00-13.00 | 8.00-12.00 | 9.00-11.00 |
| other excipients | 0.50-9.00 | 0.50-7.50 | 0.50-6.00 | 0.50-4.50 | 0.50-3.00 | 0.50-1.50 |

It has been unexpectedly found that at concentrations within the range of from 0.50 to 1.00 wt.-%, relative to the total weight of the adhesive layer containing an acrylate polymer (total per dry unit), the pharmacologically active ingredient can be formulated in molecular dispersed form that exhibits a satisfactory shelf-life, i.e. that does not tend to recrystallize, and excellent flux rates. In this regard, the acrylate polymer is superior over the silicone polymer, particularly when it contains one or more percutaneous permeation enhancers selected from dipropylene glycol and oleyl alcohol and/or a crystallization inhibitor such as polyvinylpyrrolidone.

Preferably, the preferred acrylate copolymers according to any of embodiments $B^1$ to $B^8$ can be contained in any of the preferred compositions of adhesive layers according to any of embodiments $C^1$ to $C^{12}$, i.e.: $B^1C^1$, $B^1C^2$, $B^1C^3$, $B^1C^4$, $B^1C^5$, $B^1C^6$, $B^1C^7$, $B^1C^8$, $B^1C^9$, $B^1C^{10}$, $B^1C^{11}$, and $B^1C^{12}$; $B^2C^1$, $B^2C^2$, $B^2C^3$, $B^2C^4$, $B^2C^5$, $B^2C^6$, $B^2C^7$, $B^2C^8$, $B^2C^9$, $B^2C^{10}$, $B^2C^{11}$, and $B^2C^{12}$; $B^3C^1$, $B^3C^2$, $B^3C^3$, $B^3C^4$, $B^3C^5$, $B^3C^6$, $B^3C^7$, $B^3C^8$, $B^3C^9$, $B^3C^{10}$, $B^3C^{11}$, and $B^3C^{12}$; $B^4C^1$, $B^4C^2$ $B^4C^3$, $B^4C^4$, $B^4C^5$, $B^4C^6$, $B^4C^7$, $B^4C^8$, $B^4C^{10}$, $B^4C^{11}$, and $B^4C^{12}$; $B^5C^1$, $B^5C^2$, $B^5C^3$, $B^5C^4$, $B^5C^5$, $B^5C^6$, $B^5C^7$, $B^5C^8$, $B^5C^9$, $B^5C^{10}$, $B^5C^{11}$, and $B^5C^{12}$; $B^6C^1$, $B^6C^2$, $B^6C^3$, $B^6C^4$, $B^6C^5$, $B^6C^6$, $B^6C^7$, $B^6C^8$, $B^6C^9$, $B^6C^{10}$, $B^6C^{11}$, and $B^6C^{12}$; $B^7C^1$, $B^7C^2$, $B^7C^3$, $B^7C^4$, $B^7C^5$, $B^7C^6$, $B^7C^7$, $B^7C^8$, $B^7C^9$, $B^7C^{10}$, $B^7C^{11}$, and $B^7C^{12}$; $B^8C^1$, $B^8C^2$, $B^8C^3$, $B^8C^4$, $B^8C^5$, $B^8C^6$, $B^8C^7$, $B^8C^8$, $B^8C^9$, $B^8C^{10}$, $B^8C^{11}$, and $B^8C^{12}$.

In another preferred embodiment, the pressure sensitive adhesive contained in the adhesive layer comprises a polyisobutylene based pressure sensitive adhesive (e.g. Duro-Tak® 87-6908).

In still another preferred embodiment, the pressure sensitive adhesive contained in the adhesive layer comprises a styrenic rubber based pressure sensitive adhesive (polystyrene based pressure sensitive adhesive) (e.g.Duro-Tak® 87-6911).

In yet another preferred embodiment, the pressure sensitive adhesive contained in the adhesive layer comprises a mixture of two or more different pressure sensitive adhesives, e.g. a combination of two different polyacrylate based pressure sensitive adhesives, or a combination of an polyacrylate based pressure sensitive adhesive with a polysilicone based pressure sensitive adhesive; or a combination of an polyacrylate based pressure sensitive adhesive with a polyisobutylene based pressure sensitive adhesive or styrenic rubber based pressure sensitive adhesive; or a combination of a polysilicone based pressure sensitive adhesive with a polyisobutylene based pressure sensitive adhesive or styrenic rubber based pressure sensitive adhesive.

The layer of the pharmaceutical patch that contains the pharmacologically active ingredient or a portion thereof, i.e. the adhesive layer and the drug layer, respectively, may contain other pharmaceutical excipients that are conventionally contained in pharmaceutical patches.

Preferably, the adhesive layer comprises an antioxidant. Suitable antioxidants include but are not limited to alpha-tocopherol, butyl hydroxytoluene or n-propylgalat.

Preferably, the content of the antioxidant is within the range of from 0.01 to 10 wt.-%, more preferably 0.05 to 7.5 wt.-%, still more preferably 0.1 to 2.5 wt.-%, yet more preferably 0.5 to 1.5 wt.-%, even more preferably 0.7 to 1.3 wt.-%, most preferably 0.8 to 1.2 wt.-%, and in particular 0.9 to 1.1 wt.-%, relative to the total weight of the adhesive layer.

In a preferred embodiment, particularly when the adhesive layer does not contain the pharmacologically active ingredient (taking into account that after manufacture there typically is an exchange of the pharmacologically active ingredient between adjacent layers until an equilibrium has been reached), the area of the adhesive layer corresponds to the area of the pharmaceutical patch.

In another preferred embodiment, particularly when the adhesive layer contains the pharmacologically active

ingredient, the total area of the adhesive layer can be divided into at least two portions of different composition: an inner area containing the pharmacologically active ingredient and an outer rim surrounding said inner area like a frame, said outer rim preferably not containing pharmacologically active ingredient. The area of said outer rim is not particularly limited but preferably amounts to e.g. about 5% of the total area of the adhesive layer.

In preferred embodiments, the pharmaceutical patch according to the invention exhibits satisfactory storage stability and shelf-life.

Preferably, after storage of the pharmaceutical patch for 3 month at 40°C and 75% relative humidity in a sealed glass container, the degradation of the pharmacologically active ingredient does not exceed 5%, more preferably 4%, still more preferably 3%, yet more preferably 2%, and most preferably 1.5%.

Preferably, after storage of the pharmaceutical patch for 3 month at 5±3°C in a sealed glass container, the degradation of the pharmacologically active ingredient does not exceed 4%, more preferably 3%, still more preferably 2%, yet more preferably 1.5%, most preferably 1%, and in particular 0.75%.

In a preferred embodiment of the pharmaceutical patch according to the invention

- the pharmacologically active ingredient is present in form of the free base, and/or the concentration of the pharmacologically active ingredient in the adhesive layer is at least 0.50 wt.-%, relative to the total weight of the adhesive layer, and/or

- the pharmaceutical patch upon application to the human skin provides over a period of at least 6 hours release of the pharmacologically active ingredient at a rate of at least 10 $ng \cdot cm^{-2} \cdot h^{-1}$; and/or

- the pharmaceutical patch provides plasma concentrations of the pharmacologically active ingredient over a period of at least 6 hours upon consecutive application of a series of pharmaceutical patches to the human skin, i.e. under steady state conditions taking into account the depot effect of the skin, of at least 100 $pg \cdot ml^{-1}$; and/or

- the adhesive layer comprises a polymer that forms a matrix in which the pharmacologically active ingredient is dispersed (drug-in-adhesive) ; and/or

- the adhesive layer comprises an polyacrylate based pressure sensitive adhesive, preferably comprising an acrylate copolymer derived from a monomer composition comprising vinyl acetate, 2-ethylhexyl acrylate and 2-hydroxyethyl acrylate (terpolymer), optionally also comprising glycidyl methacrylate; and/or

- the adhesive layer comprises a cationic copolymer based on dimethylaminoethyl methacrylate, butyl methacrylate, and methyl methacrylate; and/or

- the adhesive layer contains one or more percutaneous penetration enhancer(s), preferably selected from the group consisting of transcutol (diethylene glycol monoethylether), oleyl alcohol, dipropylene glycol and mixtures thereof; and/or

- the adhesive layer contains one or more crystallization inhibitors, preferably selected from polyvinylpyrrolidone (e.g. Kollidon 25); and/or

- the adhesive layer comprises an antioxidant in an amount within the range of from 0.01 to 10 wt.-%.

In another preferred embodiment of the pharmaceutical patch according to the invention

- the pharmacologically active ingredient is present in form of the free base, and/or

- the concentration of the pharmacologically active ingredient in the adhesive layer is at least 0.20 wt.-%, relative to the total weight of the adhesive layer, and/or

- the adhesive layer comprises a polymer that forms a matrix in which the pharmacologically active ingredient is dispersed (drug-in-adhesive) ; and/or

- the adhesive layer comprises a polysilicone based pressure sensitive adhesive, and/or

- the adhesive layer contains one or more percutaneous penetration enhancer(s), preferably selected from the group consisting of transcutol (diethylene glycol monoethylether), oleyl alcohol, dipropylene glycol and mixtures thereof;

and/or

- the adhesive layer contains one or more crystallization inhibitors, preferably selected from polyvinylpyrrolidone (e.g. Kollidon 25).

The pharmaceutical patch according to the invention may be prepared by standard techniques for the manufacture of pharmaceutical patches. Such standard techniques are known to the skilled person (cf., e.g., H.A.E. Benson et al., Topical and Transdermal Drug Delivery: Principles and Practice, John Wiley & Sons; 2011; A.K. Banga, Transdermal and Intradermal Delivery of Therapeutic Agents: Application of Physical Technologies, CRC Press Inc; 2011).

Another aspect of the invention relates to a pharmaceutical patch as described above for use in the treatment of pain, preferably moderate to severe pain. The pain may be acute or chronic, central or peripheral, visceral or neuropathic.

The pharmaceutical patch according to the invention is suitable for use in the treatment of neuropathic pain, preferably chronic neuropathic pain such as painful diabetic neuropathy. Preferably, the pain is moderate, severe, or moderate to severe.

For the purpose of the specification, neuropathic pain is pain that originates from nerve damage or nerve malfunction. Preferably, the neuropathic pain is selected from acute neuropathic pain and chronic neuropathic pain. Neuropathic pain may be caused by damage or disease affecting the central or peripheral portions of the nervous system involved in bodily feelings (the somatosensory system). Preferably, the pharmaceutical patch according to the invention is for use in the treatment of chronic neuropathic pain or acute neuropathic pain, peripheral neuropathic pain or central neuropathic pain, mononeuropathic pain or polyneuropathic pain. When the neuropathic pain is chronic, it may be chronic peripheral neuropathic pain or chronic central neuropathic pain, in a preferred embodiment chronic peripheral mononeuropathic pain or chronic central mononeuropathic pain, in another preferred embodiment chronic peripheral polyneuropathic pain or chronic central polyneuropathic pain. When the neuropathic pain is acute, it may be acute peripheral neuropathic pain or acute central neuropathic pain, in a preferred embodiment acute peripheral mononeuropathic pain or acute central mononeuropathic pain, in another preferred embodiment acute peripheral polyneuropathic pain or acute central polyneuropathic pain. The invention also relates to the pharmacologically active ingredient according to the invention a physiologically acceptable salt thereof for use in the treatment of neuropathic pain as described above.

Central neuropathic pain is found in spinal cord injury, multiple sclerosis, and some strokes. Fibromyalgia is potentially a central pain disorder and is responsive to medications that are effective for neuropathic pain. Accordingly, the pharmaceutical patch according to the invention is also suitable for the treatment of fibromyalgia. Aside from diabetic neuropathy and other metabolic conditions, the common causes of painful peripheral neuropathies are herpes zoster infection, HIV-related neuropathies, nutritional deficiencies, toxins, remote manifestations of malignancies, genetic, and immune mediated disorders or physical trauma to a nerve trunk. Neuropathic pain is common in cancer as a direct result of cancer on peripheral nerves (e.g., compression by a tumor), or as a side effect of chemotherapy, radiation injury or surgery.

The pharmaceutical patch according to the invention is also suitable for use in the treatment of nociceptive pain, preferably acute or chronic nociceptive pain. Preferably, the pain is moderate, severe, or moderate to severe.

Nociceptive pain refers to the discomfort that results when a stimulus causes tissue damage to the muscles, bones, skin or internal organs. For the purpose of the specification, nociceptive pain is caused by stimulation of peripheral nerve fibers that respond only to stimuli approaching or exceeding harmful intensity (nociceptors), and may be classified according to the mode of noxious stimulation; the most common categories being "thermal" (heat or cold), "mechanical" (crushing, tearing, etc.) and "chemical" (iodine in a cut, chili powder in the eyes). Nociceptive pain may also be divided into "visceral," "deep somatic" and "superficial somatic" pain.

Visceral pain describes a type of nociceptive pain originating in the body's internal organs or their surrounding tissues. This form of pain usually results from the infiltration of harmful cells, as well as the compression or extension of healthy cells. Patients suffering from visceral pain tend to feel generally achy, as this pain tends to not be localized to a specific area. Cancer is a common source of visceral pain.

Somatic pain is nociceptive pain that results from some injury to the body. It's generally localized to the affected area and abates when the body repairs the damage to that area. Deep somatic pain is initiated by stimulation of nociceptors in ligaments, tendons, bones, blood vessels, fasciae and muscles, and is dull, aching, poorly-localized pain. Examples include sprains and broken bones. Superficial pain is initiated by activation of nociceptors in the skin or superficial tissues, and is sharp, well-defined and clearly located.

According to the invention, nociceptive pain is preferably classified chronic if it has occurred for at least 3 months. Preferably, the chronic nociceptive pain is selected from chronic visceral pain, chronic deep somatic pain and chronic superficial somatic pain.

Preferred causes of nociceptive pain according to the invention include broken or fractured bones, bruises, burns, cuts, inflammation (from infection or arthritis), and sprains. Thus, nociceptive pain includes post-operative pain,

cancer pain, low back pain, and inflammatory pain.

In preferred embodiments, the pain to be treated is selected from the group consisting of pain being or being associated with panic disorder [episodic paroxysmal anxiety] [F41.0]; dissociative [conversion] disorders [F44]; persistent somatoform pain disorder [F45.4]; pain disorders exclusively related to psychological factors [F45.41]; nonorganic dyspareunia [F52.6]; other enduring personality changes [F62.8]; sadomasochism [F65.5]; elaboration of physical symptoms for psychological reasons [F68.0]; migraine [G43]; other headache syndromes [G44]; trigeminal neuralgia [G50.0]; atypical facial pain [G50.1]; phantom limb syndrome with pain [G54.6]; phantom limb syndrome without pain [G54.7]; acute and chronic pain, not elsewhere classified [G89]; ocular pain [H57.1]; otalgia [H92.0]; angina pectoris, unspecified [I20.9]; other specified disorders of nose and nasal sinuses [J34.8]; other diseases of pharynx [J39.2]; temporomandibular joint disorders [K07.6]; other specified disorders of teeth and supporting structures [K08.8]; other specified diseases of jaws [K10.8]; other and unspecified lesions of oral mucosa [K13.7]; glossodynia [K14.6]; other specified diseases of anus and rectum [K62.8]; pain in joint [M25.5]; shoulder pain [M25.51]; sacrococcygeal disorders, not elsewhere classified [M53.3]; spine pain [M54.]; radiculopathy [M54.1]; cervicalgia [M54.2]; sciatica [M54.3]; low back pain [M54.5]; pain in thoracic spine [M54.6]; other dorsalgia [M54.8]; dorsalgia, unspecified [M54.9]; other shoulder lesions [M75.8]; other soft tissue disorders, not elsewhere classified [M79]; myalgia [M79.1]; neuralgia and neuritis, unspecified [M79.2]; pain in limb [M79.6]; other specified disorders of bone [M89.8]; unspecified renal colic [N23]; other specified disorders of penis [N48.8]; other specified disorders of male genital organs [N50.8]; mastodynia [N64.4]; pain and other conditions associated with female genital organs and menstrual cycle [N94]; mittelschmerz [N94.0]; other specified conditions associated with female genital organs and menstrual cycle [N94.8]; pain in throat and chest [R07]; pain in throat [R07.0]; chest pain on breathing [R07.1]; precordial pain [R07.2]; other chest pain [R07.3]; chest pain, unspecified [R07.4]; abdominal and pelvic pain [R10]; acute abdomen pain [R10.0]; pain localized to upper abdomen [R10.1]; pelvic and perineal pain [R10.2]; pain localized to other parts of lower abdomen [R10.3]; other and unspecified abdominal pain [R10.4]; flatulence and related conditions [R14]; abdominal rigidity [R19.3]; other and unspecified disturbances of skin sensation [R20.8]; pain associated with micturition [R30]; other and unspecified symptoms and signs involving the urinary system [R39.8]; headache [R51]; pain, not elsewhere classified [R52]; acute pain [R52.0]; chronic intractable pain [R52.1]; other chronic pain [R52.2]; pain, unspecified [R52.9]; other complications of cardiac and vascular prosthetic devices, implants and grafts [T82.8]; other complications of genitourinary prosthetic devices, implants and grafts [T83.8]; other complications of internal orthopaedic prosthetic devices, implants and grafts [T84.8]; other complications of internal prosthetic devices, implants and grafts, not elsewhere classified [T85.8]; wherein the information in brackets refers to the classification according to ICD-10.

Preferably, the pharmaceutical patch is designed for application to the skin for a period of least 1 day, more preferably at least 2 days, most preferably at least 3 days or at least 3.5 days, and in particular 3 days, 3.5 days, 4 days or 7 days. Thus, according to this embodiment, continuous administration of the pharmacologically active ingredient can be achieved by removing a used pharmaceutical patch after the predetermined period has expired and replacing it by a fresh pharmaceutical patch.

In a preferred embodiment, the pharmaceutical patch is designed for application to the skin for an application period A, followed by a treatment period T during which no pharmaceutical patch is applied to the skin. Thus, according to this embodiment, continuous administration of the pharmacologically active ingredient can be achieved by removing a used pharmaceutical patch after the application period A has expired and replacing it by a fresh pharmaceutical patch after the treatment period has expired as well. It has been surprisingly found that the pharmacological half life $t_{1/2}$ of the pharmacologically active ingredient is comparatively high so that its pharmacological effect lasts for a long time after administration has been interrupted or terminated. Preferred durations of application period A and treatment period T are summarized as embodiments $D^1$ to $D^{17}$ in the table here below:

| [days] | $D^1$ | $D^2$ | $D^3$ | $D^4$ | $D^5$ | $D^6$ | $D^7$ | $D^8$ | $D^9$ | $D^{10}$ | $D^{11}$ | $D^{12}$ | $D^{13}$ | $D^{12}$ | $D^{15}$ | $D^{16}$ | $D^{17}$ |
|---|---|---|---|---|---|---|---|---|---|---|---|---|---|---|---|---|---|
| A | 1 | 1 | 2 | 2 | 1 | 3 | 2 | 3 | 3 | 1 | 4 | 2 | 4 | 3 | 4 | 4 | 3.5 |
| T | 1 | 2 | 1 | 2 | 3 | 1 | 3 | 2 | 3 | 4 | 1 | 4 | 2 | 4 | 3 | 4 | 3.5 |

The locations of the skin to which the pharmaceutical patch according to the invention is to be applied are not particularly limited. Preferably, the pharmaceutical patch according to the invention is applied to the skin of the breast or the skin of the back.

In a preferred embodiment, the pharmaceutical patches according to the invention are repeatedly applied to the same location on the skin, i.e. after a first pharmaceutical patch has been used and needs to be replaced by a second pharmaceutical patch in order to maintain the desired pharmacological effect, said second pharmaceutical patch is preferably applied to the same location on the skin to which said first pharmaceutical patch was applied before.

In another preferred embodiment, particularly when the patient has a sensitive skin, the pharmaceutical patches

according to the invention are applied to the different locations on the skin, i.e. after a first pharmaceutical patch has been used and needs to be replaced by a second pharmaceutical patch in order to maintain the desired pharmacological effect, said second pharmaceutical patch is preferably applied to a location on the skin differing from the location on the skin to which said first pharmaceutical patch was applied before.

The pharmaceutical patch according to the invention is for administration to the skin of an mammal, preferably of a human (pediatrics or adults).

The following examples further illustrate the invention but are not to be construed as limiting its scope.

For the purpose of the specification, "API" refers to the pharmacologically active ingredient, unless expressly stated otherwise in form of its free base.

For the purpose of the specification, "DEGR1", "DEGR2", "DEGR3" and "DEGR4" are identified degradation products of the API. DEGR2 has been confirmed as being non-toxic.

## EXAMPLES

Peel strength working instruction

The peel strength is the force, measured in Newton, that must be exerted to remove the patch with a defined velocity from a stainless steel test plate.

*Equipment and materials*

Tensile testing device (e.g. Zwick BT1-FR2.5TN.D14) including PC/application
Adequate load cell (e.g. 100 N)
Grips enabling measurement in 90° angle
Peeling apparatus (Sliding table), for measuring at 90° angle
Stainless steel test plate eg. V4A-AISI 316L
Separation aid: adhesive tape (e.g. tesafix 4963)
Cleaning agent: acetone
Die cut and die cut form (25 mm width)

*Sample preparation*

25 mm wide stripes are die cut out of the patches, which are equilibrated to the climatic measuring conditions (23°C ± 3°C / 50% ±10% r.h.) for at least 2 h prior to measurement.

*Test procedure*

The 100 N load cell and the sliding table including the stainless steel testing plate are installed. The test plate is cleaned with acetone.

After the separation aid is affixed to the patch, the release liner is removed completely from the patch and the patch is adhered to the stainless steel plate without pressure, air bubbles or wrinkles. The separation aid is then fixed into the clamp of the tensile testing device in such a way that the patch is peeled off of the test plate in a 90° angle running a test velocity of 300 mm/min. The measurement has to be carried out in a timeframe of 30 to 60 seconds after adhering the sample to the test plate.

*Evaluation of the data*

The standard procedure comprises 6 samples. The average value among the mean forces occurred within the measurement range is reported as N /25 mm.

Example 1:

Patches with a layer sequence protective layer/adhesive layer/protective layer and differing in the formulation of the adhesive were prepared as follows:

Adhesives used:

Duro-Tak 87-9301: 36.90 wt.-% solids; non-functional acrylic (no hydroxyl, no carboxyl groups)

Durotak 87-2287: 51.00 wt.-% solids; copolymer of vinyl acetate (28%), 2-ethylhexylacrylate (67%), hydroxyethyl acrylate (4.9%) and glycidyl methacrylate (0.1%))

Durotak 87-4098: 39.66 wt.-% solids; copolymer of 2-ethylhexylacrylate and vinyl acetate BIO PSA 7-4503 59.40 wt.-% solids; silicone adhesive, non-functional, non-reactive, especially with amino groups.

Adhesive formulations of API at 1 wt.-% and 3 wt.-% in different adhesives were prepared in 20 mL vials and mixed on a jar roller. API at 1 wt.-% and 3 wt.-% in Duro-Tak 87-2287 (Ex. 1-3 and 1-4) were found to be insoluble. The other acrylate-based adhesive formulations were used to cast one sheet on a silicone coated polyester or polyethylene film (Loparex Primeliner® FLS release liner). Silicone-based adhesive formulations (Ex. 1-7 and 1-8) were cast onto a fluoropolymer coated polyester film (3M Scotchpak® 1022 release liner). A Gardco Automatic Drawdown machine was used to spread the adhesive at a thickness to target 100 g/m$^2$. The sheet was dried for five minutes at room temperature, then for seven minutes at 92 ºC. It was laminated with another sheet of the respective protective layer. The sheets were visually examined to verify API solubility, and checked for crystallization. The sheets were cut into round patches (3.9 cm$^2$), stored at room temperature in polypropylene ziplock bags and were checked for crystallization formation with time.

| Ingredients of adhesive layer [g][1)] (dry wt.-%)[2)] | Ex. 1-1 form.1 | Ex. 1-2 form.2 | Ex. 1-3 form.3 | Ex. 1-4 form.4 | Ex. 1-5 form.5 | Ex.1-6 form.6 | Ex.1-7 form. 7 | Ex. 1-8 form. 8 |
|---|---|---|---|---|---|---|---|---|
| API free base | 0.05 (1) | 0.15 (3) | 0.05 (1) | 0.15 (3) | 0.05 (1) | 0.15 (3) | 0.05 (1) | 0.15 (3) |
| acrylic polymer adhesive (DT87-9301) | 13.41 (99) | 13.14 (97) | - | - | - | - | - | - |
| acrylic polymer adhesive (DT87-2287) | - | - | 9.71 (99) | 9.51 (97) | - | -- | - | - |
| acrylic polymer adhesive (DT87-4098) | - | - | - | - | 12.48 (99) | 12.23 (97) | - | - |
| silicone adhesive BIO PSA 7-4503 | - | - | - | - | - | - | 12.48 (99) | 12.23 (97) |
| additional toluene | 0.25 (-) | 0.25 (-) | 0.50 (-) | 0.50 (-) | 0.25 (-) | 0.25 (-) | - | - |
| additional isopropanol | 0.25 (-) | 0.25 (-) | 0.50 (-) | 0.50 (-) | 0.25 (-) | 0.25 (-) | - | - |
| solubility of API in adhesive | soluble | soluble | insoluble | insoluble | soluble | soluble | soluble | soluble |
| appearance of casted sheet | clear | cloudy | - | - | clear | cloudy | some undissolved API | some undissolved API |
| [1)]: adhesive composition (5 g batch) after mixing; [2)]: composition of adhesive layer after drying | | | | | | | | |

Example 2:

According to the procedure of Example 1, further patches containing transcutol, oleyl alcohol or dipropylene glycol as additives were prepared. The final adhesive formulations and results are summarized here below:

| Ingredients of adhesive layer [g][1)] (dry wt.-%)[2)] | Ex. 2-1 | Ex. 2-2 | Ex. 2-3 | Ex. 2-4 | Ex. 2-5 | Ex.2-6 |
|---|---|---|---|---|---|---|
| API free base | 0.05 (1) | 0.05 (1) | 0.05 (1) | 0.05 (1) | 0.05 (1) | 0.05 (1) |
| acrylic polymer adhesive (DT87-9301) | 12.74 (94) | 12.74 (94) | 12.74 (94) | - | - | - |
| silicone adhesive BIO PSA 7-4503 | - | - | - | 7.91 (94) | 7.91 (94) | 7.91 (94) |
| transcutol | 0.25 (5) | - | - | 0.25 (5) | - | - |
| oleyl alcohol | - | 0.25 (5) | - | - | 0.25 (5) | - |
| dipropylene glycol | - | - | 0.25 (5) | - | - | 0.25 (5) |
| solubility of API in adhesive mixture | soluble | soluble | soluble | soluble | soluble | insoluble |
| appearance of casted sheet | clear | clear | clear | clear | clear | - |
| [1)]: adhesive composition (5 g batch) after mixing; [2)]: composition of adhesive layer after drying | | | | | | |

An in-vitro permeation study was performed with the adhesive formulations of the patches to show permeation of drug through synthetic membranes. The in-vitro experiments were conducted using Hanson Microette Franz Cell apparatus. The Franz-diffusion Cell consists of a donor compartment and a receiver compartment. A donut-shaped dosage wafer (inside diameter 15 mm) was fixated on top of each membrane and approximately 300 mg of the adhesive formulation were applied on top of the membrane to top the dosage wafer compartment. The membrane was sandwiched between the two compartments. A 40% aqueous PEG-400 solution (vol.-% PEG-400) was used as the receiving medium. The diffusion cells temperature was maintained at 32.5 °C ± 0.5 °C. At predetermined times (2, 4, 6, 8, 12 and 24 hours) samples (aliquots of 500 μl) were withdrawn from the receiving compartment and immediately replaced by the same volume of fresh receiver medium. The permeation area (effective surface of sample well) was 1.76 $cm^2$ and the volume of the individual Franz Cell was 7 mL.

The synthetic membranes selected were: Nylon μm, Polysulfone, 0.45 μm, Cellulose acetate, 0.45 μm and SilTech (non-porous, silicone).

Flux studies with all five adhesive formulations were conducted using a Nylon membrane. The permeability results expressed in terms of cumulative amount (Q; μg/$cm^2$) and flux (μg/$cm^2$ x h) are summarized here below:

| Example | Total Flux Time | Q Permeated (μg) | Q per Surface Area[1)] (μg/$cm^2$) | Flux = dQ/dT (μg/$cm^2$ h) |
|---|---|---|---|---|
| 2-1 | 24 h | 9.60 | 5.45 | 0.20 |
| 2-2 | 24 h | 0.0 | 0.0 | 0.00 |
| 2-3 | 24 h | 24.60 | 13.98 | 0.44 |
| 2-4 | 24 h | 11.80 | 6.70 | 0.44 |
| 2-5 | 24 h | 0.70 | 0.40 | 0.03 |
| [1)]: effective area of sample well: 1.76 $cm^2$ | | | | |

Ex. 2-3 exhibits the best permeation through Nylon membranes.

Based on these results, a comparative flux study on the adhesive formulation of Ex. 2-3 using Nylon, Polysulfone, Cellulose acetate and SilTech (silicone) membranes was performed. The nylon membrane was run in duplicate to verify the reproducibility of permeation through this membrane. A summary of the results is presented in the table below:

| Membrane | Total Flux Time | Q Permeated (μg) | Q per Surface Area[1)] (μg/$cm^2$) | Flux = dQ/dT (μg/$cm^2$ h) |
|---|---|---|---|---|
| Polysulfone | 24 h | 23.08 | 13.12 | 0.40 |
| Nylon | 24 h | 20.74 | 11.78 | 0.35 |
| Nylon | 24 h | 20.53 | 11.67 | 0.36 |
| Cellulose acetate | 24 h | 17.09 | 9.71 | 0.39 |

(continued)

| Membrane | Total Flux Time | Q Permeated (μg) | Q per Surface Area1) (μg/cm$^2$) | Flux = dQ/dT (μg/cm$^2$ h) |
|---|---|---|---|---|
| Sil-Tech (SiliconE) | 24 h | 15.61 | 8.87 | 0.38 |
| 1): effective area of sample well: 1.76 cm$^2$ | | | | |

As a result, nylon and cellulose acetate produced the most controlled results, that is, membranes showing the lowest permeation which would potentially be indicative of an ex vivo model. These membranes represent opposite polarities cellulose acetate being highly polar and porous (0.45 μm) and silicone being non-polar and non-porous. The other 3 membranes exhibited a high initial permeation and significant tailing off which indicates the membrane is not rate-controlling, but merely a membrane that allows dissolution of the drug from the adhesive matrix.

Example 3:

Patches were prepared from the adhesive composition of the patch according to Ex. 2-3 (Ex. 3-1) and from six further adhesive compositions containing transcutol, oleyl alcohol and/or dipropylene glycol as additives.

The adhesive formulations were prepared in 20 mL vials and mixed on a jar roller. The formulations were used to cast one sheet on a silicone coated polyester or polyethylene film (Loparex Primeliner® FLS release liner). A Gardco Automatic Drawdown machine was used to spread the adhesive at a thickness to target 100 g/m$^2$. The sheet was dried for five minutes at room temperature, then for seven minutes at 92 ºC. It was laminated with a polyester laminate (3M Scotchpak® 9754 Surface layer, polyester film with an ethylene vinylacetate copolymer heat seal layer). Some 3.9 cm$^2$ round patches were cut from each sheet and packaged in heat sealed foil pouches.

The patches were visually examined to verify API solubility, and checked for crystallization. The sheets were stored at room temperature in polypropylene ziplock bags and were checked for crystallization formation with time.

The final adhesive formulations and results are summarized here below:

| Ingredients of adhesive layer [g] (dry wt.-%) | Ex. 3-1 | Ex. 3-2 | Ex. 3-3 | Ex. 3-4 | Ex. 3-5 | Ex. 3-6 | Ex. 3-7 |
|---|---|---|---|---|---|---|---|
| API free base | 0.05 (1) | 0.05 (1) | 0.05 (1) | 0.05 (1) | 0.05 (1) | 0.05 (1) | 0.05 (1) |
| acrylic polymer adhesive (DT87-9301) | 12.74 (94) | 11.07 (89) | 10.45 (84) | 11.07 (89) | 11.07 (89) | 11.07 (89) | 10.45 (84) |
| transcutol | - | - | - | 0.25 (5) | - | - | - |
| oleyl alcohol | - | - | - | - | 0.25 (5) | - | - |
| dipropylene glycol | 0.25(5) | 0.50 (10) | 0.75 (15) | 0.25 (5) | 0.25 (5) | 0.25 (5) | 0.25 (5) |
| polyvinyl pyrrolidone (Kollidon 25) | - | - | - | - | - | 0.25 (5) | 0.50 (10) |
| solubility of API in adhesive mixture | soluble | soluble | soluble | soluble | soluble | soluble | soluble |
| appearance of casted sheet directly after production | clear | clear | clear | clear | clear | clear | clear |
| appearance of casted sheet after 7 days | clear | cloudy, uneven | clear | clear | clear | clear | cloudy, uneven |

According to Example 2, a comparative flux study was conducted on the patches using cellulose acetate and silicone membranes.

The results are depicted in Figures 1 and 2 as well as in the tables here below.

Cellulose membrane:

| Example | Total Flux Time | Q Permeated (μg) | Q per Surface Area[1)] (μg/cm$^2$) | Flux = dQ/dT (μg/cm$^2$ h) |
|---|---|---|---|---|
| 3-1 | 24 h | 4.10 | 2.33 | 0.19 |
| 3-1 | 24 h | 7.15 | 4.06 | 0.18 |
| 3-3 | 24 h | 0.53 | 0.30 | 0.02 |
| 3-4 | 24 h | 7.35 | 4.17 | 0.17 |
| 3-5 | 24 h | 26.90 | 15.28 | 0.64 |
| 3-6 | 24 h | 23.17 | 13.16 | 0.77 |
| [1)]: effective area of sample well: 1.76 cm$^2$ | | | | |

Sil-Tech (Silicone) membrane:

| Example | Total Flux Time | Q Permeated (μg) | Q per Surface Area[1)] (μg/cm$^2$) | Flux = dQ/dT (μg/cm$^2$ h) |
|---|---|---|---|---|
| 3-1 | 24 h | 7.38 | 4.19 | 0.15 |
| 3-1 | 24 h | 7.26 | 4.13 | 0.14 |
| 3-3 | 24 h | 8.63 | 4.90 | 0.21 |
| 3-4 | 24 h | 6.18 | 3.51 | 0.13 |
| 3-5 | 24 h | 16.31 | 9.27 | 0.37 |
| 3-6 | 24 h | 20.49 | 11.64 | 0.39 |
| [1)]: effective area of sample well: 1.76 cm$^2$ | | | | |

The results above indicate that Examples 3-5 and 3-6 show the best flux profiles when using both polar (cellulose acetate) and non-polar (silicone) membranes although the flux is higher with the cellulose acetate membranes.

Example 4:

In accordance with Example 3, a patch was prepared from the adhesive composition according to the patch of Example 2-4, a fluoropolymer coated polyester film (3M Scotchpak® 1022) as protective layer and a polyester laminate (3M Scotchpak® 9754, polyester film with an ethylene vinylacetate copolymer heat seal layer) as surface layer.

A comparative study was conducted to evaluate the permeation behavior of API from the transdermal patches according to Examples 3-5, 3-6 and 4-1 through artificial skin constructs in comparison with two solutions (solution A: 300 μg/mL API in ethanol/DMSO/PEG 200 = 8:1:1; solution B: saturated solution of API in PEG 400).

Although the performance of the two solutions was better than the performance of the transdermal patches, these tests revealed that the pharmacologically active ingredient, when being contained in a pharmaceutical patch, can also penetrate into and through artificial skin. Furthermore, these tests indicate that pharmaceutical patches containing the pharmacologically active ingredient in an polyacrylate based pressure sensitive adhesive (drug-in-adhesive) are superior with respect to skin permeation performance over pharmaceutical patches containing the pharmacologically active ingredient in a polysilicone based pressure sensitive adhesive (drug-in-adhesive).

Example 5:

Chemical stability testing was conducted with the patches of Examples and 3-5, 3-6 and 4. The conditions and detected amounts of impurities DEGR1 and DEGR2 are summarized here below:

| Ex. | Timepoint | Storing conditions [°C/% r.h.] | Assay [%] | Impurity DEGR1 [%] | Impurity DEGR2 [%] |
|---|---|---|---|---|---|
| 3-5 | start | -/- | 105.48 | 1.12 | 0.33 |
| | 1 month | 40/75 | 103.97 | 1.25 | 0.59 |
| | 6 days | 60/- | 105.61 | 1.44 | 0.88 |
| 4 | start | -/- | 108.33 | n.d. | 0.36 |
| | 1 month | 40/75 | 104.79 | n.d. | 0.40 |
| | 6 days | 60/- | 108.77 | n.d. | 0.41 |
| 3-6 | start | -/- | 106.58 | 1.15 | 0.32 |
| | 1 month | 40/75 | 105.35 | 1.28 | 0.42 |
| | 6 days | 60/- | 105.82 | 1.34 | 0.56 |
| n.d.: not detected; r.h.: relative humidity | | | | | |

As DEGR2 itself exhibits pharmacological activity and is non-toxic, it can be concluded from the above data that formulation 4 is storage stable, while the storage stability of formulations 3-5 and 3-6 is not satisfactory.

Example 6:

The patches according to the previous Examples showed some re-crystallization of the matrices containing 1 wt.-% of API. In order to develop non-recrystallizing maximum API loads, variations of the patches according to Examples 3-5, 3-6 and 4-1 containing varying amounts of API were prepared according to the following procedure.

API in form of the free base is dissolved in isopropanol/toluene (1:1). The solution is sonicated, the other ingredients are added and the mixture is stirred overnight. A Gardco Automatic Drawdown machine is used to spread the adhesive composition at a thickness of 100 g/m$^2$ (unless stated otherwise) on a protective layer. The laminate is dried for ten minutes at room temperature and for 10 minutes at 70°C before it is laminated with a sheet of Surface layer and cut to the desired size (9 cm$^2$ unit). The resulting patches were tested microscopically for recrystallization at 5 storing conditions after 1, 2 and 4 weeks.

Storing conditions:
a) 2-8 °C
b) 40°C/75% relative humidity, open to air
c) 40°C/75% relative humidity, sealed
d) 25°C/60% relative humidity, sealed

Protective layer: silicone coated polyethylene or polyester film (Loparex Primeliner® FLS), used for the acrylate-based adhesives

fluoropolymer coated polyester film (3M Scotchpak® 1022), used for the silicone-based adhesives

Surface layer: polyester laminate (3M Scotchpak® 9754, polyester film with an ethylene vinylacetate copolymer heat seal layer)

| Example | Excipients (dry wt.-%) | API (dry wt.-%) | physicochemically stable after 4 weeks at all conditions tested |
|---|---|---|---|
| 6-1 | Bio-PSA 4503/Transcutol (94/5)) | 1 | no |
| 6-2 | Bio-PSA 4503/Transcutol (94.5/5)) | 0.5 | no |
| 6-3 | Bio-PSA 4503/Transcutol (94.6/5)) | 0.4 | no |
| 6-4 | Bio-PSA 4503/Transcutol (94.7/5)) | 0.3 | no |
| 6-5 | Bio-PSA 4503/Transcutol (94.8/5)) | 0.2 | yes |
| 6-6 | DT87-9301/Oleyl alcohol/DPG (89/5/5) | 1 | no |

(continued)

| Example | Excipients (dry wt.-%) | API (dry wt.-%) | physicochemically stable after 4 weeks at all conditions tested |
|---|---|---|---|
| 6-7 | DT87-9301/Oleyl alcohol/DPG (89.1/5/5) | 0.9 | no |
| 6-8 | DT87-9301/Oleyl alcohol/DPG (89.2/5/5) | 0.8 | no |
| 6-9 | DT87-9301/Oleyl alcohol/DPG (89.3/5/5) | 0.7 | no |
| 6-10 | DT87-9301/Oleyl alcohol/DPG (89.5/5/5) | 0.5 | yes |
| 6-11 | DT87-9301/PVP/DPG (88.5/5/5) | 1.5 | no |
| 6-12 | DT87-9301/PVP/DPG (88.75/5/5) | 1.25 | no |
| 6-13 | DT87-9301/PVP/DPG (89/5/5) | 1 | no |
| 6-14 | DT87-9301/PVP/DPG (89.25/5/5) | 0.75 | yes |
| 6-15 | DT87-9301/PVP/DPG (89.5/5/5) | 0.5 | yes |
| PVP: polyvinyl pyrrolidone (Kollidon 25); DPG: dipropylene glycol | | | |

It can be concluded from the above data that in polyacrylate based adhesive layers a substantially higher API load can be achieved than in polysilicone based adhesive layers. Further, it can be concluded from the above data that in the presence of polyvinylpyrrolidone (PVP) a substantially higher API load can be achieved than in the presence of oleyl alcohol.

Example 7:

Variations of the patch according to Ex. 6-14 (acrylic adhesive) were prepared according to the procedure of Example 6 using different antioxidants. The amount of impurity DEGR1 was determined:

| Ex. | Antioxidant [1 wt.-%] | Other excipients [wt.-%] | DEGR1 [%] |
|---|---|---|---|
| 7-1 | alpha-tocopherol | DT87-9301/PVP/DPG/API (88.25/5/5/0.75) | 0.90 |
| 7-2 | butyl hydroxytoluene | DT87-9301/PVP/DPG/API (88.25/5/5/0.75) | 0.86 |
| 7-3 | n-propylgalat | DT87-9301/PVP/DPG/API (88.25/5/5/0.75) | 0.93 |

It can be concluded from the above data that compared to formulation 3-6 of Example 5 (1.15%), the addition of antioxidants reduces the initial content of undesired degradation product DEGR1. In this regard, the stabilizing effect of different antioxidants is equivalent to one another.

Example 8:

Patches were prepared according to the procedure of Example 6, one based on acrylate adhesive Durotak 87-9301 and the others based on acrylate adhesive Durotak 87-4287. Further, the area weight of the adhesive was varied. The resulting patches were exposed to short term and stress stability testing and the amount of impurities DEGR1 and API was determined.

Formulation A: DT87-9301/PVP/DPG/API (89.25/5/5/0.75)
Formulation B-1: DT87-4287/PVP/DPG/API (89.25/5/5/0.75)
Formulation B-2: DT87-4287/PVP/DPG/BHT/API (88.25/5/5/1.00/0.75)

Durotak 87-4287 (40 wt.-% solids, copolymer of vinyl acetate (17-40%), 2-ethylhexylacrylate (55-75%) and hydroxyethyl acrylate (5.2%)).

| Ex. | Form. | area weight adhesive [g/m²] | Batch size [g] | Start DEGR1 [wt.-%] | 1 month 40°C/75% r.h. DEGR1 [wt.-%] | 6 days/60°C DEGR1 [wt.-%] |
|---|---|---|---|---|---|---|
| 8-1 | A | 100 | 180 | 0.95 | not tested | not tested |
| 8-2 | B-1 | 100 | 40 | 0.21 | 1.06 | 1.30 |
| 8-3 | B-1 | 55 | 40 | 0.15 | 0.70 | 0.85 |
| 8-4 | B-2 | 100 | 40 | 0.19 | 0.77 | 0.99 |
| 8-5 | B-2 | 55 | 40 | 0.14 | 0.58 | 0.71 |
| 8-6 | B-2 | 100 | 140 | 0.19 | not tested | 0.63 |
| 8-7 | B-2 | 55 | 140 | 0.17 | 1.06 | 0.57 |

It becomes evident from the above table that the change from acrylic adhesive Durotak 87-9301 to Durotak 87-4287 led to a significantly decreased level of DEGR1 after manufacturing. The amount of DEGR1 is further dependent on the area weight and thickness of the adhesive.

The patches according to Example 8-7 were chosen for further short term and accelerated stability studies:

| Time point/ conditions | Assay [%] | DEGR1 [wt.-%] | DEGR2 [wt.-%] | RRT 0.55 [wt.-%] | RRT 0.59 [wt.-%] | RRT 0.31 [wt.-%] | RRT 0.40 [wt.-%] | Sum all [wt.-%] | Sum unknown [wt.-%] |
|---|---|---|---|---|---|---|---|---|---|
| Start | 123.16 | 0.17 | 0.00 | 0.00 | 0.00 | 0.00 | 0.00 | 0.17 | 0.00 |
| 1 month 40°C/ 75% r.h. | 101.67 | 1.06 | 0.18 | 0.09 | 0.00 | 0.00 | 0.00 | 1.32 | 0.09 |
| 3 months 40°C/ 75% r.h. | 103.75 | 1.10 | 0.14 | 0.00 | 0.00 | 0.00 | 0.00 | 1.23 | 0.00 |
| 4.5 months 40°C/75% r.h. | 96.5 | 1.13 | 0.23 | 0.06 | 0.05 | 0.00 | 0.08 | 1.55 | 0.18 |

| Time point/ conditions | Assay [%] | DEGR1 [wt.-%] | DEGR2 [wt.-%] | RRT 0.55 [wt.-%] | RRT 0.59 [wt.-%] | RRT 0.31 [wt.-%] | RRT 0.40 [wt.-%] | Sum all [wt.-%] | Sum unknown [wt.-%] |
|---|---|---|---|---|---|---|---|---|---|
| Start | 123.16 | 0.17 | 0.00 | 0.00 | 0.00 | 0.00 | 0.00 | 0.17 | 0.00 |
| 1 month 2-8°C | 95.45 | 0.50 | 0.06 | 0.05 | 0.00 | 0.00 | 0.00 | 0.61 | 0.05 |
| 2 months 2-8°C | 98.48 | 0.61 | 0.00 | 0.00 | 0.00 | 0.00 | 0.00 | 0.61 | 0.00 |
| 3 months 2-8°C | 106.73 | 0.61 | 0.06 | 0.00 | 0.00 | 0.00 | 0.00 | 0.67 | 0.00 |
| 4.5 months 2-8°C | 97.30 | 0.56 | 0.08 | 0.05 | 0.05 | 0.00 | 0.00 | 0.74 | 0.10 |
| RRT0.55, RRT0.59, RRT0.31 and RRT0.40 are unknown impurities characterized by a specific HPLC retention time | | | | | | | | | |

It can be concluded from the above data that the formation of degradation product DEGR1 during manufacture and storage can be suppressed by

- replacing Durotak 87-9301 by Durotak 87-4287;
- reducing the thickness of the adhesive layer (100 g/m² -> 55 g/m²); and
- adding antioxidants (e.g. BHT 1%).

Example 9:

According to the procedure of Example 6, patches were prepared from the acrylic adhesive compositions given

in the table here below. The resulting patches were tested microscopically for recrystallization after 4 weeks storing at 40°C/75% relative humidity, open to air.

| Ex. | Excipients (dry wt.-%) | | | | | physicochemically |
|---|---|---|---|---|---|---|
| | DT87-4287 | PVP | DPG | BHT | API | stable (crystal free) |
| 9-1 | 89.25 | 5.00 | 5.00 | - | 0.75 | yes |
| 9-2 | 88.25 | 5.00 | 5.00 | 1.00 | 0.75 | yes |
| 9-3 | 88.00 | 5.00 | 5.00 | 1.00 | 1.00 | no |
| 9-4 | 87.75 | 5.00 | 5.00 | 1.00 | 1.25 | no |
| 9-5 | 87.50 | 5.00 | 5.00 | 1.00 | 1.50 | no |
| PVP: polyvinyl pyrrolidone (Kollidon 25); DPG: dipropylene glycol; BHT: butylhydroxy toluene | | | | | | |

It can be concluded from the above data that when replacing Durotak 87-9301 by Durotak 87-4287 the API load cannot be further increased.

Example 10:

According to the procedure of Example 6, patches were prepared from the silicone adhesive compositions given in the table here below. The resulting patches were tested microscopically for recrystallization after 4 weeks storing at 40°C/75% relative humidity, open to air.

| Ex. | Excipients (dry wt.-%) | | | | physicochemically stable (crystal free) |
|---|---|---|---|---|---|
| | BIOPSA 7-4503 | transcutol | PVP | API | |
| 10-1 | 94.50 | 5.00 | - | 0.50 | no |
| 10-2 | 94.60 | 5.00 | - | 0.40 | no |
| 10-3 | 94.70 | 5.00 | - | 0.30 | no |
| 10-4 | 94.80 | 5.00 | - | 0.20 | yes |
| 10-5 | 89.00 | 5.00 | 5.00 | 1.00 | no |
| 10-6 | 89.50 | 5.00 | 5.00 | 0.50 | no |
| 10-7 | 89.60 | 5.00 | 5.00 | 0.40 | no |
| 10-8 | 89.70 | 5.00 | 5.00 | 0.30 | yes |

The patches according to Examples 10-4 and 10-8 were exposed to short term and stress stability testing. The amount of impurities DEGR1 and API was determined at different times.

| Ex. | area weight adhesive [g/m$^2$] | Batch size | Start | | 1 month 40°C/ 75% r.h. | 6 days/60°C | 7 months/RT |
|---|---|---|---|---|---|---|---|
| | | | DEGR1 | DEGR2 | DEGR1 | DEGR1 | DEGR1 |
| | | [g] | [wt.-%] | [wt.-%] | [wt.-%] | [wt.-%] | [wt.-%] |
| 10-4 | 100 | 40 | n.d. | 0.18 | n.d. | n.d. | - |
| 10-8 | 100 | 40 | 0.24 | 0.23 | 0.38 | n.d. | n.d. |
| n.d.: not detected | | | | | | | |

It can be concluded from the above data that this formulations exhibits excellent storage stability and shelf-life.

Example 11:

Patches containing acrylic adhesive and silicone adhesive were prepared according to the procedure of Example 6. The amount of impurity DEGR1 was detected and the resulting patches were tested microscopically for recrystallization at 5 different storing conditions.

Storing conditions: a) 2-8 °C
b) 40°C/75% relative humidity, open to air
c) 40°C/75% relative humidity, sealed
d) 25°C/60% relative humidity, sealed

| Ex. | Excipients (dry wt.-%) DT87-4287 | BIOPSA 7-4503 | API | area weight adhesive [g/m$^2$] | physicochemically stable after 4 weeks at all conditions tested | DEGR1 Start [wt.-%] |
|---|---|---|---|---|---|---|
| 11-1 | 49.85 | 49.85 | 0.30 | 55 | no | 0.85 |
| 11-2 | 49.80 | 49.80 | 0.40 | 55 | no | 0.60 |
| 11-3 | 49.75 | 49.75 | 0.50 | 55 | no | 0.43 |
| 11-4 | 49.70 | 49.70 | 0.60 | 55 | no | 0.50 |

It can be concluded from the above data that two-component-formulation systems did neither provide advantages with regards to increased API load as compared to one-component silicone systems nor lower initial DEGR1 levels as compared to one-component-acrylate systems.

Example 12: Studies on hairless rats

In accordance with Example 3, laminates were prepared from the following acrylate adhesive compositions:

Solution 12A: Ethanol:DMSO:Lipoxol (PEG) 8:1:1 with API hemicitrate; dose as indicated in the table below, concentration variable (volume constant); and
Solution 12B: Solution of API hemicitrate or free base in PEG400; dose as indicated in the table below, concentration variable (volume constant);
Patch 12C: 89 wt.-% DT87-9301, 5 wt.-% dipropylene glycol, 5 wt.-% polyvinyl pyrrolidone, 1 wt.-% API free base (dry wt.-%); and
Patch 12D: 89.25 wt.-% DT87-4287, 5 wt.-% dipropylene glycol, 5 wt.-% polyvinyl pyrrolidone, 1 wt.-% butylhydroxy tolulol, 0.75 wt.-% API free base (dry wt.-%); and
Patch 12E: 94.8 wt.-% Bio-PSA 7-4503, 5 wt.-% Transcutol, 0.2 wt.-% API free base (dry wt.-%)

Patches of different sizes (3, 5, 12, 12.5 and 25 cm$^2$) were cut from the laminate.
The analgesic efficacy of the patches was investigated in comparison to the efficacy of a topically applied PEG 400 solution of API (600 μg/mL) in the burning ray (tail flick) test on the rat by the method of D'Amour and Smith (J. Pharm. Exp. Ther. 72, 74 79 (1941). Female hairless rats (OFA hr/hr, Charles River USA) weighing approx. 200 g were used. The animals were placed individually in special test cages and the base of the tail was exposed to a focused ray of heat from an electric lamp. The lamp intensity was adjusted such that the time from switching on the lamp to sudden jerking away of the tail (pain latency) was 3-5 seconds in untreated animals. Before topical administration of the drug-containing plaster or solution, the animals were pretested twice in the course of five minutes and the mean of these measurements was calculated as the pretest mean.
The pain measurement and the plasma concentrations were determined at different points in time after application of the plaster or solution (see table below).
The analgesic action was determined as the increase in the pain latency (% MPE) according to the following formula: [(T1-T0)/(T2-T0)]*100. In this, T0 is the latency time before and T1 the latency time after administration of the substance, T2 is the maximum exposure time (12 sec).

| | Study no. 1 | Study no. 2 | Study no. 3 | Study no. 4 |
|---|---|---|---|---|
| Species | Female hairless rats OFA hr/hr (Charles River USA) | | | |
| Animals | 36 (12 per group) | 20 (8 per group and 4 control animals) | (8 per group) | (8 per group) |
| Vehicle | Ethanol:DMSO:Lip oxol (8:1:1) (Solution 12A) | PEG 400 (Solution 12B) | PEG 400 and Patch 12C | PEG 400 + SEDDS Patch 12D + 12E |

(continued)

| | Study no. 1 | Study no. 2 | Study no. 3 | Study no. 4 |
|---|---|---|---|---|
| Dose | 50 μg/kg<br>API hemicitrate 100 μg/kg<br>API hemicitrate 200 μg/kg<br>API hemicitrate | 375 μg/kg<br>API hemicitrate<br>1500 μg/kg<br>API<br>Vehicle only | 300 μg/kg<br>API<br>25 $cm^2$ TDS (2060 μg)<br>12.5 $cm^2$ TDS (1030 μg)<br>5 $cm^2$ TDS (412 μg) | 1029 μg/kg<br>API PEG400<br>1029 μg/kg<br>API SEDDS<br>12.5 $cm^2$ TDS (2763 μg)<br>25 $cm^2$ TDS (2591 μg) |
| Admin. | 0.5 mL/kg | 0.5 mL/kg | 0.5 mL/kg for group 1 | 180μL/Animal<br>via Finn Chamber (3 $cm^2$) |
| Treatment duration | 24 hours | 24 hours | 24 hours | 30 hours |
| Tail-flick | 2 and 24h | 2, 6, 24, 29 and 48h | 2, 6, 24, 29 and 51h | 2, 6, 12 (solutions only), 24, 29 and 51 h |
| PK | 2.25, 4, 6, 10, 24.25, 26, 30 and 34h | 2.25, 4, 6.25, 10, 24.25, 26, 30, 34 and 48.25h | 2.25, 4, 6.25, 10, 24.25, 26, 30, 34 and 51.25h | 2.25, 4, 6.25, 10, 24.25, 26, 30, 34 and 51.25 h |
| SEDDS - Self-Emulsifying Drug Delivery System | | | | |

*a) Study no. 1*

The compositions of the solutions that were applied to the different groups are summarized in the table here below. The results of the tail-flick test and the plasma concentration measurements are depicted in Figure 3.

| group | vehicle and amount | dose [μg/ animal] | $C_{max}$ [ng/mL] | $AUC_{0-t}$ [h·ng/mL] | $t_{last}$ [h] | F [T/R] |
|---|---|---|---|---|---|---|
| ref. | API free base (i.v.) (Sprague-Dawley) | 32 $^A$ | 11.7±0.940 (8%) | 16.1±1.91 (12%) | 12-24 | - |
| 1 | 102 μg/kg; hemicitrate API | 16 $^B$ | 0.425±0.230 (54%) | 6.33±2.30 (36%) | 30-34 | 76% |
| 2 | 52 μg/kg; API hemicitrate | 8.3 $^B$ | 0.240±0.080 (33%) | 4.28±1.09 (25%) | 34 | 99% |
| 3 | 188 μg/kg; API hemicitrate | 30 $^B$ | 1.48±1.26 (85%) | 15.5±7.12 (46%) | 30-34 | 99% |
| A) Body weight = 160 g/ animal<br>B) Body weight = 200 g/ animal | | | | | | |

Based on the pharmacokinetic data, the bioavailability of API from the patches was calculated. The pharmacokinetic data of an intravenous application of API free base was used as reference: $F = AUC_{dermal} / AUC_{intravenous} \times dose_{intravenous} / dose_{dermal}$

It becomes evident from the above tables that the bioavailability of the API hemicitrate in Ethanol:DMSO:Lipoxol (8:1:1 v/v/v) was 100% after dermal application compared to intraveneous administration. Further, a dose-proportional increase of $AUC_{0-t}$ was found.

*b) Study no. 2*

The compositions of the solutions that were applied to the different groups are summarized in the table here

below. The results of the tail-flick test and the plasma concentration measurements are depicted in Figure 4.

| Group | vehicle | dose of API [μg] |
|---|---|---|
| 1 | PEG 400 (12 $cm^2$ patch) | 300 μg/kg (API hemicitrate) |
| 2 | PEG 400 (12 $cm^2$ patch) | 1500 μg/kg (API free base) |

*c) Study no. 3*

The composition of the patches and the solution that were applied to the different groups are summarized in the table here below. The results of the tail-flick test and the plasma concentration measurements are depicted in Figures 5 to 7.

| Group | vehicle / amount | dose of API free base [μg] |
|---|---|---|
| 1 | PEG 400, 0.5 mL/kg[1)] | 300 μg/kg[1)] |
| 2 | 25 $cm^2$ patch (82.4 μg/$cm^2$) | 2,060 |
| 3 | 12.5 $cm^2$ patch (82.4 μg/$cm^2$) | 1,030 |
| 4 | 5 $cm^2$ patch (82.4 μg/$cm^2$) | 412 |
| [1)]: body weight: approx. 200 g | | |

Based on the pharmacological data, the bioavailability of API from the patches was calculated. The pharmacokinetic data of an intravenous application of API free base was used as reference: $F = AUC_{dermal} / AUC_{intravenous} \times dose_{intravenous} / dose_{dermal}$

| Ex. | Dose[1)] [μg/kg] | $C_{max}$ [ng/mL] | $AUC_{0-t}$ [h · ng/mL] | $t_{last}$ [h] | F [Ex./ref.] |
|---|---|---|---|---|---|
| 12-2 | 10,300 | 1.02 ± 0.380 (37%) | 18.9 ± 5.86 (31%) | 51.25 | 1.8% |
| 12-3 | 5,150 | 0.432 ± 0.201 (46%) | 8.47 ± 1.06 (13%) | 51.25 | 1.6% |
| 12-4 | 2,060 | 0.274 ± 0.217 (79%) | 4.17 ± 1.53 (37%) | 51.25 | 2.0% |
| reference[2)] | 160 | 11.7 ± 0.940 (79%) | 16.1 ± 1.91 (12%) | 12-24 | |
| [1)]: body weight: approx. 200g/animal<br>[2)]: reference: Sprague-Dawley rat, API free base intravenous | | | | | |

It becomes evident from the above tables that the bioavailability of the patches is approximately 1.8%. Further, a dose-proportional (area-proportional) increase of $AUC_{0-t}$ was found.

Figure 8 shows the dose dependency of the patches of study no. 3.

*d) Study no. 4*

The composition of the patches and the solution that were applied to the different groups are summarized in the table here below.

| Group | vehicle | dose of API free base [μg] |
|---|---|---|
| 1 | PEG 400 (3 $cm^2$ Finn chamber) | 180 μg (1029 μg/kg) |
| 2 | SEDDS (3 $cm^2$ Finn chamber) | 180 μg (1029 μg/kg) |

The results of the tail-flick test and the plasma concentration measurements are depicted in Figures 9 and 10.

Figure 9 shows the tail-flick data and plasma concentrations of API free base in PEG400 or SEDDS applied in Finn chambers to approximately 3 $cm^2$.

Figure 10 shows the tail-flick data and plasma concentrations of API free base after application of Patch 12D

(acrylate) and Patch 12E (silicone).

e) *Overview (studies no. 2, 3 and 4)*

The absolute bioavailability of API free base and API hemicitrate is summarized in the table here below.

| study | group | dose [μg/ animal] | $C_{max}$ [ng/mL] | $AUC_{0-t}$ [h·ng/mL] | $t_{last}$ [h] | F [T/R] |
|---|---|---|---|---|---|---|
| ref. | API free base (i.v.) (Sprague-Dawley) | 32 $^A$ | 11.7±0.940 (8%) | 16.1±1.91 (12%) | 12-24 | |
| 2 | PEG/API hemicitrate (750μg/mL; 60%) | 60 $^A$ | 0.47±0.12 (26%) | 9.88±1.73 (17%) | 34 -48.25 | 32% |
| | PEG/API free base (3000μg/mL; 60%) | 300 $^A$ | 8.51±5.17 (61%) | 128±26.8 (21%) | 48.25 | 82% |
| 3 | PEG/API free base (600μg/mL; 12%) | 60 $^A$ | 1.88±1.33 (71%) | 22.3±11.8 (53%) | 34 -51.25 | 71% |
| 4 (Finn chamber) | PEG/API free base (1000μg/mL; 180μL) | 180 | 0.208±0.129 (62%) | 2.66±0.654 (25%) | 51.25 | 2.8% |
| | SEEDS/API free base (1000μg/mL; 180μL) | 180 | 0.171±0.020 (12%) | 3.26±1.01 (31%) | 51.25 | 3.5% |
| body weight: approx. 200g/animal | | | | | | |

Based on the pharmacokinetic data, the bioavailability of API from the patches was calculated. The pharmacokinetic data of an intravenous application of API free base was used as reference: $F = AUC_{dermal} / AUC_{intravenous} \times dose_{intravenous} / dose_{dermal}$

It becomes evident from the above table that the bioavailability of the API hemicitrate is approximately 2.5 fold lower compared to the API free base in PEG400. The bioavailability of API free base in human SEDDS is slightly higher compared to the PEG formulation (Finn chamber).

Further, a dose-proportional increase of $AUC_{0-t}$ for the API free base in PEG400 was observed.

Figure 11 shows the dose dependency of patches according to studies no. 2 and 3.

The absolute bioavailability of API free base after application of Patch 12C (acrylate), Patch 12D (acrylate) and Patch 12E (silicone) is summarized in the table here below.

| study | group | dose [μg/ animal] | $C_{max}$ [ng/mL] | $AUC_{0-t}$ [h·ng/mL] | $t_{last}$ [h] | F [T/R] |
|---|---|---|---|---|---|---|
| ref. | API free base (i.v.) (Sprague-Dawley) | 32 | 11.7±0.940 (8%) | 16.1 ± 1.91 (12%) | 12 - 24 | - |
| 3 | Acrylate: 25cm$^2$ Patch 12C (82μg/cm$^2$; 2060 μg/Animal) | 2060 | 1.02±0.380 (37%) | 18.9 ± 5.86 (31%) | 51.25 | 1.8% |
| | Acrylate: 12.5cm$^2$ Patch 12C (82μg/cm$^2$; 1030 μg/Animal) | 1030 | 0.432±0.201 (46%) | 8.47 ± 1.06 (13%) | 51.25 | 1.6% |
| | Acrylate: 5cm$^2$ Patch 12C (82μg/cm$^2$; 412 μg/Animal) | 412 | 0.274±0.217 (79%) | 4.17 ± 1.53 (37%) | 51.25 | 1.9% |
| 4 | Acrylate: 12.5cm$^2$ Patch 12D (42μg/cm$^2$; 528 μg/Animal) | 528 | 1.03±0.413 (40%) | 23.5 ± 7.05 (30%) | 51.25 | 8.5% |
| | Silicone: 25cm$^2$ Patch 12E (20μg/cm$^2$; 500 μg/Animal) | 500 | 0.824±0.491 (60%) | 16.8 ± 7.43 (44%) | 51.25 | 6.4% |
| body weight: approx. 200g/animal | | | | | | |

Based on the pharmacokinetic data, the bioavailability of API from the patches was calculated. The pharmacokinetic data of an intravenous application of API free base was used as reference: F = $AUC_{dermal}$ /$AUC_{intravenous}$ x $dose_{intravenous}$/$dose_{dermal}$

It becomes evident from the above table that the bioavailability of acrylate Patch 12C is approximately 1.8%. The bioavailability of API free base in Patch 12D is slightly higher compared to the silicone Patch 12E (8.5% vs. 6.4%).

Further, a dose-proportional (area-proportional) increase of $AUC_{0-t}$ was observed.

The absolute bioavailability and flux of API free base for different topical vehicles is summarized in the table here below.

| vehicle | group | dose [µg/ animal] | $AUC_{0-t}$ [h·ng/mL] | F [T/R] | size [$cm^2$] | flux [A)] [ng/$cm^2$/h] |
|---|---|---|---|---|---|---|
| Solution 12A Ethanol:DMSO:Lipoxol (8:1:1) | 52 µg/kg; API hemicitrate | 8.3 | - | 99% | ~12 | 28 |
| | 102 µg/kg; API hemicitrate | 16 | - | 76% | ~12 | 42 |
| | 188 µg/kg; API hemicitrate | 30 | - | 99% | ~12 | 103 |
| Solution 12B PEG 400 | API hemicitrate (750µg/mL; 60%) | 60 | - | 32% | ~12 | 66 |
| | API free base (3000µg/mL; 60%) | 300 | - | 82% | ~12 | 852 |
| | API free base (600µg/mL; 12%) | 60 | - | 71% | ~12 | 148 |
| | API free base (1000µg/mL; 20%) Finn chamber | 180 | - | 2.8% | ~3 | 71 |
| SEDDS | API free base (1000µg/mL) Finn Chamber | 180 | - | 3.5% | ~3 | 87 |
| Patch 12C (82.4 µg/$cm^2$) Acrylate patch | 25$cm^2$, 2060 µg API free base per animal | 2060 | 18.9 | 1.8% | 25 | 60 |
| | 12.5$cm^2$, 1030 µg API free base per animal | 1030 | 8.47 | 1.6% | 12.5 | 54 |
| | 5$cm^2$, 412 µg API free base per animal | 412 | 4.17 | 1.9% | 5 | 67 |
| Patch 12D (42.2 µg/$cm^2$) Acrylate patch | 12.5$cm^2$, 528 µg API free base per animal | 528 | 23.5 | 8.5% | 12.5 | 150 |
| Patch 12E (20 µg/$cm^2$) Silicone patch | 25$cm^2$, 500 µg API free base per animal | 500 | 16.8 | 6.4% | 25 | 54 |
| A) based on total patch load or load in solution<br>Flux = (F·$Dose_{Dermal}$)/($Size_{Dermal}$·Time) | | | | | | |

Based on the pharmacokinetic data, bioavailability F of API from the different formulations was calculated. For the calculation, the corresponding pharmacokinetic data of an intravenous application of API free base was used as reference: F = $AUC_{dermal}$ /$AUC_{intravenous}$ x $dose_{intravenous}$/$dose_{dermal}$ x 100%.

It becomes evident from the above table that highest flux rates were derived from highly dosed PEG400 solution and from patch 12D (acrylate).

Example 13: Overview of bioavailability and flux of API in hairless rats

Patches with vehicles 13-1 to 13-12 were prepared and tested as described above in Example 12:

13-1 Acrylate Patch 12D: 89.25 wt.-% DT87-4287 (acrylate), 5 wt.-% dipropylene glycol, 5 wt.-% polyvinyl pyrrolidone, 1 wt.-% butylhydroxy tolulol, 0.75 wt.-% API free base (dry wt.-%)

13-2 44.75 wt.-% DT87-4287 (acrylate), 44.75 wt.-% Eudragit® EPO, 5 wt.-% Transcutol, 5 wt.-% Levulinic acid,

0.5 wt.-% API free base (dry wt.-%)
13-3 49.4 wt.-% DT87-4287 (acrylate), 49.4 wt.-% Eudragit® EPO, 1.2 wt.-% API free base (dry wt.-%)
13-4 44.3 wt.-% DT87-4287 (acrylate), 44.3 wt.-% Eudragit® EPO, 10 wt.-% Transcutol, 1.4 wt.-% API free base (dry wt.-%)
13-5 44.81 wt.-% DT87-4287 (acrylate), 44.81 wt.-% Eudragit® EPO, 10 wt.-% Levulinic acid, 0.38 wt.-% API free base (dry wt.-%)
13-6 94.8 wt.-% Bio-PSA 7-4503 (silicone), 5 wt.-% Transcutol, 0.2 wt.-% API free base (dry wt.-%)
13-7 85.0 wt.-% Bio-PSA 7-4503 (silicone), 13.5 wt.-% polyvinyl alcohol, 1.5 wt.-% API free base (dry wt.-%)
13-8 85.0 wt.-% Bio-PSA 7-4602/3 (silicone), 13.5 wt.-% polyvinyl pyrrolidone, 1.5 wt.-% API free base (dry wt.-%)
13-9 99.75 wt.-% DT87-6911 (styrene), 0.25 wt.-% API free base (dry wt.-%)
13-10 89.7 wt.-% DT87-6911 (styrene), 5 wt.-% Transcutol, 5 wt.-% Levulinic acid, 0.3 wt.-% API free base (dry wt.-%)
13-11 89.5 wt.-% DT87-6911 (styrene), 10 wt.-% Transcutol, 0.5 wt.-% API free base (dry wt.-%)
13-12 89.7 wt.-% DT87-6911 (styrene), 10 wt.-% Levulinic acid, 0.3 wt.-% API free base (dry wt.-%)

The absolute bioavailability of API free base and API hemicitrate is summarized in the table here below.

| vehicle | group | dose [µg/animal] | duration [h] | size [cm²] | $AUC_{0-t}$ [h·ng/mL] | F [T/R] | flux A) [ng/cm²/h] | size norm. $AUC_{0-t}$ |
|---|---|---|---|---|---|---|---|---|
| 13-1 | 12.5cm² à 42µg/cm² API free base | 528<br>520 | 30<br>24 | 12.5 | 23.5<br>15.1 | 7.1% | 108 | 1.6 |
| 13-2 | 3, 5 and 10cm² à 29µg/cm² API free base | 86<br>145<br>290 | 24 | 3<br>5<br>10 | 1.78<br>2.94/3.61<br>9.80 | 4.8% | 58 | 0.72 |
| 13-3 | 5cm² à 68µg/cm² API free base | 341 | 24 | 5 | 6.46 | 3.6% | 103 | 1.3 |
| 13-4 | 5cm² à 76µg/cm² API free base | 382 | 24 | 5 | 5.30 | 2.7% | 85 | 1.1 |
| 13-5 | 5cm² à 22µg/cm² API free base | 111 | 24 | 5 | 5.42 | 9.4% | 87 | 1.1 |
| 13-6 | 25cm² à 20µg/cm² API free base | 500 | 30 | 25 | 16.8 | 6.4% | 43 | 0.67 |
| 13-7 | 12.5cm² à 80µg/cm² API free base | 1000 | 24 | 12.5 | 3.66 B) | 0.70% | 23 | 0.29 |
| 13-8 | 12.5cm² à 80µg/cm² API free base | 1000 | 24 | 12.5 | 4.43 B) | 0.85% | 28 | 0.35 |
| 13-9 | 12.5cm² à 14µg/cm² API free base | 175 | 24 | 12.5 | 18.7 | 20% | 119 | 1.5 |
| 13-10 | 5cm² à 17µg/cm² API free base | 84 | 24 | 5 | 0.919 | 2.1% | 15 | 0.18 |
| 13-11 | 5cm² à 28µg/cm² API free base | 139 | 24 | 5 | 4.42 | 6.1% | 71 | 0.88 |
| 13-12 | 5cm² à 17µg/cm² API free base | 83 | 24 | 5 | 0.650 | 1.5% | 10 | 0.13 |

A) based on total patch load or load in solution
B) $AUC_{0-24h}$
Flux = $(F \cdot Dose_{Dermal})/(Size_{Dermal} \cdot Time)$

Based on the pharmacokinetic data, bioavailability F and flux rate of API from the different formulations was calculated. For the calculation, the corresponding pharmacokinetic data of an intravenous application of API free base was used as reference.

$$F = AUC_{dermal} / AUC_{intravenous} \text{ x } dose_{intravenous} / dose_{dermal} \text{ x } 100\%$$

$$\text{Flux (ng/cm}^2\text{/h)} = (AUC_{dermal} \text{ x } dose_{dermal}) / (AUC_{intravenous} \text{ x } size_{dermal} \text{ x duration})$$

It becomes evident from the above table that the flux of the styrene patch (13-9) was slightly higher but still comparable to the acrylate prototype (13-1). The silicone formulations 13-7 and 13-8 showed significantly reduced plasma levels.

None of the combinations of adhesives with enhancers demonstrate increased flux in comparison to the acrylate prototype (13-1). In contrast, levels were significantly reduced in some cases.

Example 14: Studies in minipigs

*a) Study on male minipigs*

According to Example 6, laminates were prepared from the following adhesive compositions:

i) acrylate-based adhesive composition:

88.25 wt.-% DT87-4287, 5 wt.-% dipropylene glycol, 5 wt.-% polyvinyl pyrrolidone, 0.75 wt.-% API free base (dry wt.-%). For the adhesive composition an area weight of 55 g/$m^2$ was selected.

ii) silicone-based adhesive composition:

94.80 wt.-% Bio-PSA 7-4503, 5 wt.-% transcutol, 0.20 wt.-% API free base (dry wt.-%). For the adhesive composition an area weight of 100 g/$m^2$ was selected.

Patches of 100 $cm^2$ size were cut from the two laminates.

An animal study was conducted using eight male mini-pigs. In two periods, four different formulations of API free base were administered to the animals:

| Period | formulation | vehicle | amount / concentration of API free base | dose [µg/ animal] |
|---|---|---|---|---|
| 1 | | patch with acrylate-based adhesive | 6 x 100 $cm^2$ / 42.2 µg/$cm^2$ | 25,320 |
| | B | PEG 400 using Finn chamber (18 mm), application area: approx. 2.5-3.1 $cm^2$ | 5 x 330 µL / 1 mg/mL | 1,650 |
| 2 | C | patch with silicone-based adhesive | 6 x 100 $cm^2$/20 µg/$cm^2$ | 12,000 |
| | D | PEG 400, application area: 400 $cm^2$ | 5 mL / 1 mg/mL | 5,000 |

Plasma concentrations of API free base were determined at different times after application of the formulations.

The resulting mean pharmacokinetic data are summarized in the table here below and in Figure 12.

| Formulation | Dose [µg/animal] | $C_{max}$ [ng/mL] | $t_{max}$ | $AUC_{0-t}$ [h ng/mL] | $t_{last}$ | AUC [h · ng/mL] | $AUC_{t\%}$ [%] | $t_{1/2,z}$ |
|---|---|---|---|---|---|---|---|---|
| A | 25320 | 0.137 | 33.0 | 9.06 | 132 | 9.50 | 95.5 | 24.9 |
| B[1)] | 1650 | 0.033 | 12.0 | 2.07 | 144 | 3.06 | 67.6 | 74.4 |
| C[2)] | 12000 | 0.042 | 56.0 | 0.622 | 64 | - | - | - |

(continued)

| Formulation | Dose [µg/animal] | $C_{max}$ [ng/mL] | $t_{max}$ | $AUC_{0-t}$ [h ng/mL] | $t_{last}$ | AUC [h · ng/mL] | $AUC_{t\%}$ [%] | $t_{1/2,z}$ |
|---|---|---|---|---|---|---|---|---|
| D | 5000 | 0.053 | 39.0 | 3.85 | 138 | 5.01 | 76.5 | 64.5 |
| 1): data of one animal only<br>2): n = 3 | | | | | | | | |

Based on the pharmacokinetic data, bioavailability F and flux rate of API from the different formulations was calculated. For the calculation, the corresponding pharmacokinetic data of an intravenous application of API free base was used as reference.

$$F = AUC_{dermal} / AUC_{intravenous} \times dose_{intravenous} / dose_{dermal} \times 100\%$$

$$Flux\ (ng/cm^2/h) = (AUC_{dermal} \times dose_{dermal}) / (AUC_{intravenous} \times size_{dermal} \times duration)$$

| Formulation | Dose [µg/animal] | AUC [h · ng/mL] | F [form./ref.] | size [$cm^2$] | flux[2] [ng/$cm^2$·h] |
|---|---|---|---|---|---|
| A | 25320 | 9.50 | 1.7% | 600 | 10 |
| C | 12000 | 0.622 | 0.24% | 600 | 0.7 |
| D | 5000 | 5.01 | 4.6% | 400 | 8 |
| reference [1] | 564 | 12.3 | | | |
| 1): reference: intravenous dose of API free base: 40 µg/kg to 4 male pigs (3 were evaluated, mean body weight: 14.1 kg)<br>2): duration = 72 h | | | | | |

*b) Study on male and female minipigs*

Patches were prepared as described above. An overview on the study is provided by the table below:

| | Study no. 1 (= Example 14 a) | Study no. 2 |
|---|---|---|
| Species | Male Göttinger SPF minipigs | Female Göttinger SPF minipigs |
| Animals | 8 (4 per group) two periods with 4 weeks washout | 16 (4 per group) |
| Vehicle | PEG 400 Patch 12D + Patch 12E | PEG 400 Patch 12D + Patch 12E |
| Dose | Period 1:<br>A) Acrylate-Patch 12D: 6 x 100$cm^2$ (42.2µg/$cm^2$), 25320µg/animal<br>B) PEG: 5 x ~330µL (Finn chamber à 3 $cm^2$), 1.65mL (1000µg/mL), 1650µg/animal<br>Period 2:<br>C) Silicone-Patch 12E: 6 x 100$cm^2$ (20.0µg/$cm^2$), 12000µg/animal<br>D) PEG: 5mL (1000µg /mL), 5000µg/animal | Period 1:<br>A) Control: two patch prototypes and PEG 400<br>B) Acrylate-Patch 12D: 6 x 100$cm^2$ (41.1 µg/$cm^2$), 24660µg/animal<br>Period 2:<br>C) Silicone-Patch 12E: 6 x 100$cm^2$ (20.3µg/$cm^2$), 12180µg/animal<br>D) PEG: 5mL (3000µg /mL), 15000µg/animal |
| Application size | Patches: A) and C) = 600$cm^2$<br>PEG solution: B) = 15$cm^2$ and D) = ~300$cm^2$ | Patches: B) and C) = 600$cm^2$<br>PEG solution: D) = ~300$cm^2$ |

(continued)

| | Study no. 1 (= Example 14 a) | Study no. 2 |
|---|---|---|
| Treatment duration | 72 hours | 96 hours |
| PK | 0, 1, 2, 4, 6, 8, 12, 24, 36, 48, 60, 72 (=5 min before removal), 84, 96, 108, 120, 132 and 144h | 0, 1, 2, 4, 6, 8, 12, 24, 36, 48, 60, 72, 84, 96 (=5 min before removal), 108, 120, 132, 144, 156 and 168h |

*Male minipigs (cf. Example 14 a)*

The plasma concentrations of API free base after application of Patch 12D (acrylate) and Patch 12E (silicone) and PEG to male minipigs (Study no. 1) are displayed in Figure 13.

The resulting mean pharmacokinetic data of Study no. 1 (male) are summarized in the table here below:

| study | group | dose [µg/ animal] | $C_{max}$ [ng/mL] | $AUC_{0-t}$ [h·ng/mL] | $t_{last}$ [h] | F [T/R] |
|---|---|---|---|---|---|---|
| ref. | i.v. API free base (male minipig) | 564 | 4.86 ± 1.47 (30%) | 11.9 ± 1.33 (11%) | 36-48 | - |
| 1-A | Acrylate: 600cm$^2$ Patch 12D (42.2µg/cm$^2$; 25320µg/animal) | 25320 | 0.137 ± 0.036 (26%) | 9.06 ± 2.90 (32%) | 108-144 | 1.7% |
| 1-C | Silicone: 600cm$^2$ Patch 12E (20.0µg/cm$^2$; 12000µg/animal) | 12000 | 0.042 ± 0.025 (60%) | 0.622 ± 0.524 (84%) | 60-72 | 0.24% |
| 1-B | PEG400: ~15cm$^2$ Finn Chamber (1.65mL à 1mg/mL=1650µg/ animal) | 1650 | 0.033 $^A$ | 2.07 $^A$ | 144 | <5.9%$^A$ |
| 1-D | PEG400: ~300cm$^2$ (5mL à 1mg/mL = 5000µg/animal) | 5000 | 0.053 ± 0.012 (23%) | 3.85 ± 0.780 (20%) | 117-144 | 3.6% |
| A: one out of four animals showed concentrations >LLOQ (lower limit of quantification) | | | | | | |

Based on the pharmacokinetic data, bioavailability F of API from the different formulations was calculated. For the calculation, the corresponding pharmacokinetic data of an intravenous application of API free base was used as reference: $F = AUC_{dermal} / AUC_{intravenous} \times dose_{intravenous} / dose_{dermal} \times 100\%$.

It becomes evident from the above table that the bioavailability of API free base in acrylate Patch 12D is 7-fold higher compared to the silicone patch (1.7% vs. 0.24%). Without wanting to be limited by theory, this could be a consequence of the insufficient adherence of the silicone patch to the skin compared to the acrylate patch.

Only one out of four animals showed concentrations >LLOQ after PEG400 in Finn chambers.

*Female minipigs*

The plasma concentrations of API free base after application of Patch 12D (acrylate) and Patch 12E (silicone) and PEG to female minipigs (Study no. 2) are displayed in Figure 14.

The resulting mean pharmacokinetic data of Study no. 2 (female) are summarized in the table here below:

| study | group | dose [µg/ animal] | $C_{max}$ [ng/mL] | $AUC_{0-t}$ [h·ng/mL] | $t_{last}$[h] | F [T/R] |
|---|---|---|---|---|---|---|
| ref. | i.v. API free base (female minipig) | 648 | 6.97±1.17 (17%) | 11.4±0.82 (7%) | 36-48 | - |

(continued)

| study | group | dose [$\mu$g/ animal] | $C_{max}$ [ng/mL] | $AUC_{0-t}$ [h·ng/mL] | $t_{last}$[h] | F [T/R] |
|---|---|---|---|---|---|---|
| 2-B | Acrylate: 600cm$^2$ Patch 12D (41.1$\mu$g/cm$^2$; 24660$\mu$g/animal) | 24660 | 0.047±0.010 (21%) | 3.93±0.497 (13%) | 117-168 | 0.9% |
| 2-C | Silicone: 600cm$^2$ Patch 12E (20.3$\mu$g/cm$^2$; 12180$\mu$g/animal) | 12180 | not calculated $^A$ | not calculated $^A$ | - | - |
| 2-D | PEG400: ~300cm$^2$ (5mL á 3mg/mL = 15000$\mu$g/animal) | 15000 | 0.038±0.013 (34%) | 3.21±1.10 (34%) | 108-168 | 1.2% |
| A: one out of four animals showed concentrations >LLOQ | | | | | | |

Based on the pharmacokinetic data, bioavailability F of API from the different formulations was calculated. For the calculation, the corresponding pharmacokinetic data of an intravenous application of API free base was used as reference: $F = AUC_{dermal}/AUC_{intravenous} \times dose_{intravenous}/dose_{dermal} \times 100\%$.

It becomes evident from the above table that with the silicone patch, due to the very low plasma concentrations in three out of four animals no PK parameters were calculated. Without wanting to be limited by theory, this may be an effect of the low adhesiveness.

The bioavailability of API free base in female animals was 2-3 times lower compared to the study in male animals which might be a consequence of the skin lesions in male minipigs.

<u>*Summary*</u>

The absolute bioavailability and flux of API free base for different topical vehicles is summarized in the table below:

| study no. | group | $dose_{Dermal}$ [$\mu$g/animal] | $AUC_{0-t}$ [h·ng/mL] | F [T/R] | size [cm$^2$] | time [h] | flux$^{B)}$ [ng/cm$^2$/h] |
|---|---|---|---|---|---|---|---|
| 1 (male) | Acrylate Patch 12D: 600cm$^2$ | 25320 | 9.06 | 1.7% | 600 | 72 | 9.9 |
| | Silicone Patch 12E: 600cm$^2$ | 12000 | 0.622 | 0.24% | 600 | 72 | 0.7 |
| | PEG400: 15cm$^2$ Finn Chamber (1mg/mL) | 1650 | <<2.07$^A$ | <<5.9%$^A$ | ~15 | 72 | <<90$^A$ |
| | PEG400: 300cm$^2$ (1mg/mL) | 5000 | 3.85 | 3.6% | ~300 | 72 | 8.4 |
| 2 (female) | Acrylate Patch 12D: 600cm$^2$ | 24660 | 3.93 | 0.9% | 600 | 96 | 3.9 |
| | Silicone Patch 12E: 600cm$^2$ | 12180 | n.c. | n.c. $^A$ | 600 | 96 | n.c. $^A$ |
| | PEG400: 300cm$^2$ (3mg/mL) | 15000 | 3.21 | 1.2% | ~300 | 96 | 6.3 |
| A: one out of four animals showed concentrations >LLOQ<br>B: based on total patch load or load in solution<br>Flux = $(F \cdot DOSe_{Dermal})/(Size_{Dermal} \cdot Time)$ | | | | | | | |

Based on the pharmacokinetic data, bioavailability F of API from the different formulations was calculated. For the calculation, the corresponding pharmacokinetic data of an intravenous application of API free base was used as reference: $F = AUC_{dermal} / AUC_{intravenous} \times dose_{intravenous}/dose_{dermal} \times 100\%$.

It becomes evident from above table that in the minipig, penetration of API free base was not found for all tested

formulations (reliable plasma concentration was measured only for the "painted" PEG400, Acrylate patch and Silicone patch in males only).

The females showed 2-3 fold lower plasma concentrations compared to the males, which might be a consequence of the skin lesions in male minipigs.

Plasma concentrations increased within the first 2 days after dermal application and thereafter a concentration plateau was observed until removal of the foil (solutions) or patch at 72h/96h.

The absolute bioavailability was low for all tested formulations (PEG400 = 3.6% male/ 1.2% female and Acrylate patch = 1.7% male/ 0.9% female).

The face-to-face comparison of the patch types showed significantly lower performances of the Silicone patch, which might be a consequence of the insufficient adherence of the Silicone Patch to the skin compared to the Acrylate Patch.

Example 15: Ex vivo studies across pig skin

The permeation of API free base across dermatomized pig skin (incl. stratum corneum) from the following patches has been investigated:

- P1 Acrylate Patch 12D: 89.25 wt.-% DT87-4287 (acrylate), 5 wt.-% dipropylene glycol, 5 wt.-% polyvinyl pyrrolidone, 1 wt.-% butylhydroxy tolulol, 0.75 wt.-% API free base (dry wt.-%)
- P2 Silicone Patch 12E: 94.8 wt.-% Bio-PSA 7-4503 (silicone), 5 wt.-% Transcutol, 0.2 wt.-% API free base (dry wt.-%)
- P3 99.75 wt.-% DT87-6911 (styrene), 0.25 wt.-% API free base (dry wt.-%)
- P4 85.0 wt.-% Bio-PSA 7-4602/3 (silicone), 13.5 wt.-% polyvinyl pyrrolidone, 1.5 wt.-% API free base (dry wt.-%)
- P5 49.4 wt.-% DT87-4287 (acrylate), 49.4 wt.-% Eudragit® EPO, 1.2 wt.-% API free base (dry wt.-%)
- P6 44.75 wt.-% DT87-4287 (acrylate), 44.75 wt.-% Eudragit® EPO, 5 wt.-% Transcutol, 5 wt.-% Levulinic acid, 0.5 wt.-% API free base (dry wt.-%)

The results of ex vivo testing of patches P1 to P6 across dermatomized pig skin are summarized in the table below and in Figure 16.

| patch | | receptor fluid | dermis | epidermis | SUM (dermal absorption) |
|---|---|---|---|---|---|
| P1 | Acrylate prototype | 26.60 | 51.44 | 22.15 | 100.19 |
| P2 | Silicone prototype | 35.90 | 62.80 | 18.47 | 117.17 |
| P3 | DT 87-6911 styrene | 25.19 | 184.20 | 17.97 | 227.36 |
| P4 | Bio-PSA 7-4602/3 silicone / PVP | 7.39 | 32.15 | 10.26 | 49.80 |
| P5 | DT 87-4287 acrylate / Eudragit EPO 1:1 | 13.96 | 59.84 | 9.16 | 82.96 |
| P6 | DT 87-4287 acrylate / Eudragit EPO 1:1 + 5% Levulinic acid + 5% Transcutol | 14.98 | 77.60 | 18.57 | 111.15 |

It becomes evident from Figure 16 that the permeation across dermatomized pig skin does not reveal a significant improvement in comparison to the acrylate prototype patch, although the styrene prototype performed slightly better (while being in the same range). Furthermore, no increased permeation could be observed using Transcutol and Levulinic acid as enhancer combination.

Example 16: Permeation testing through EVA membrane

In a series of experiments under comparable conditions, the permeation of the API from various patches across a synthetic EVA membrane was tested.

Patch 16-1 corresponds to 12E (silicone prototype). Patches 16-2 to 16-4 correspond to 12E (acrylate prototype).

The results are summarized in Figures 17 (PEG 400 as acceptor medium) and 18 (ammonium acetate buffer as acceptor medium).

Summarizing, both EVA-membrane permeation systems showed a permeation kinetic which could not be obtained by using human skin, however, data achieved from the two patch prototypes are in a comparable order and ratio as observed in hairless rats. Both buffer systems yielded a similar permeation performance of patches containing API free base. The data obtained with the ammonium acetate buffer were more sophisticated compared to the system with a PEG 400 solution as acceptor medium. Accordingly, the EVA-membrane permeation system using ammonium acetate buffer is suitable to compare patch formulations containing API free base.

Example 17: Compatibility testing (polymer screening)

In a series of experiments under comparable conditions, patches of identical basic compositions but of different polymers were tested in terms of compatibility with the API free base. All patches contained 0.75 wt.-% of API free base.

The different polymers which were tested are summarized in the table below:

| batch no. | adhesive | | | API (0.75 wt.-%) |
|---|---|---|---|---|
| | name | description | solvent | solvent |
| 17-1 | DuroTak 87-2054 | Acrylate-vinylacetate (COOH) x-linked | Ethyl acetate, Heptane, Isopropyl alcohol, Pentanedione, Toluene | Isppropyl alcohol, Toluene |
| 17-2 | DuroTak 87-2510 | Acrylate (OH) | Ethyl acetate, Hexane | Isppropyl alcohol, Toluene |
| 17-3 | DuroTak 87-2051 | Acrylate-vinylacetate (COOH) | Ethyl acetate, Heptane | Isppropyl alcohol, Toluene |
| 17-4 | DuroTak 87-2353 | Acrylate (COOH) | Ethyl acetate, Hexane | Isppropyl alcohol, Toluene |
| 17-5 | DuroTak 87-502A | Acrylic-rubber hybrid (OH) | Ethyl acetate, Heptane, Hexane | Isppropyl alcohol, Toluene |
| 17-6 | DuroTak 87-503A | Acrylic-rubber hybrid (OH), x-linked | Ethyl acetate, Heptane, Hexane, Pentanedione | Isppropyl alcohol, Toluene |
| 17-7 | DuroTak 87-202A | Acrylate (OH), x-linked | Ethyl acetate, Isopropyl alcohol, Methyl alcohol, Pentanedione | Isppropyl alcohol, Toluene |
| 17-8 | DuroTak 87-6908 | Polyisobutylene | Heptane | Isppropyl alcohol, Toluene |
| 17-9 | DuroTak 87-6911 | Styrenic rubber | Toluene, Heptane | Toluene |
| 17-10/ 17-11 | DuroTak 87-9301 | Acrylate copolymer | Ethyl acetate | Isppropyl alcohol, Toluene |
| 17-12/ 17-13 | DuroTak 87-4287 | Acrylate-vinylacetate (OH) | Ethyl acetate | Isppropyl alcohol, Toluene |
| 17-14 | DuroTak 387-2516 | Acrylate-vinylacetate (OH), x-linked | Ethyl acetate, Ethyl alcohol, Heptane, Methyl alcohol | Isppropyl alcohol, Toluene |

All polyacrylate based adhesives showed no re-crystallisation of the API at start and after 6 days at 60 °C. The styrenic rubber (17-9) as well as the isobutylene prototype (17-8) showed after the manufacturing and after 6 days at 60 °C re-crystallisation of the API.

The results of a stress stability testing of API free base containing wet polymer matrices at room temperature (RT) over 6 days are summarized in the table below:

| | | API free base [%] | DEGR1 [%] | DEGR2 [%] |
|---|---|---|---|---|
| 17-1 | Start | 105.0 | - | - |
| | 6d RT | 98.3 | 0.70 | - |

(continued)

| | | API free base [%] | DEGR1 [%] | DEGR2 [%] |
|---|---|---|---|---|
| 17-2 | Start | 98.3 | 5.70 | - |
| | 6d RT | 103.2 | 5.27 | 0.07 |
| 17-3 | Start | 105.7 | - | - |
| | 6d RT | 113.1 | 0.76 | - |
| 17-4 | Start | 105.8 | - | - |
| | 6d RT | 108.3 | 2.25 | - |
| 17-5 | Start | 75.5 | 4.25 | - |
| | 6d RT | 73.8 | 4.22 | - |
| 17-6 | Start | 72.0 | 3.12 | - |
| | 6d RT | 68.4 | 2.89 | 0.13 |
| 17-7 | Start | 105.3 | 3.13 | - |
| | 6d RT | 109.1 | 4.02 | - |
| 17-8 | Start | 83.1 | - | - |
| | 6d RT | 84.1 | - | - |
| 17-9 | Start | 94.7 | - | - |
| | 6d RT | 94.1 | - | - |
| 17-10 | Start | 107.5 | 0.45 | - |
| | 6d RT | 104.6 | 0.52 | - |
| 17-12 | Start | 104.9 | 1.02 | - |
| | 6d RT | 99.1 | 0.60 | - |
| 17-14 | Start | 97.3 | 0.73 | - |
| | 6d RT | 83.3 | 0.60 | - |

The results of a stress stability testing of API free base containing patch samples at 60°C over 6 days are summarized in the table below:

| | | API free base [%] | DEGR1 [%] | DEGR2 [%] |
|---|---|---|---|---|
| 17-1 | Start | 98.5 | - | - |
| | 6d 60°C | 95.4 | 1.31 | - |
| 17-2 | Start | 93.2 | 4.29 | 0.21 |
| | 6d 60°C | 89.5 | 6.77 | 1.15 |
| 17-3 | Start | 97.0 | - | - |
| | 6d 60°C | 97.0 | 0.74 | - |
| 17-4 | Start | 100.0 | - | - |
| | 6d 60°C | 98.3 | 1.00 | - |
| 17-5 | Start | 95.5 | 3.53 | 0.39 |
| | 6d 60°C | 70.6 | 24.62 | 2.77 |
| 17-6 | Start | 93.5 | 2.64 | 0.42 |
| | 6d 60°C | 80.0 | 10.38 | 4.72 |

(continued)

| | | API free base [%] | DEGR1 [%] | DEGR2 [%] |
|---|---|---|---|---|
| 17-7 | Start | 96.3 | 0.74 | - |
| | 6d 60°C | 93.8 | 1.45 | 0.45 |
| 17-8 | Start | 98.3 | - | - |
| | 6d 60°C | 98.3 | 0.18 | - |
| 17-9 | Start | 100.0 | - | - |
| | 6d 60°C | 102.4 | - | - |
| 17-10 | Start | 90.5 | 0.66 | - |
| | 6d 60°C | 91.8 | 0.90 | - |
| 17-11 | Start | 91.8 | 0.59 | - |
| | 6d 60°C | 95.9 | 0.81 | - |
| 17-12 | Start | 95.0 | 0.60 | - |
| | 6d 60°C | 95.1 | 1.14 | 0.42 |
| 17-13 | Start | 95.0 | 0.66 | - |
| | 6d 60°C | 96.7 | 1.13 | 0.30 |
| 17-14 | Start | 95.9 | 0.13 | - |
| | 6d 60°C | 98.3 | 0.68 | 0.28 |

The styrenic rubber DuroTak 87-6911 as well as the polyisobutylene prototypes DuroTak 87-6908 yielded formulations with very low amounts of impurities. The acrylate adhesives DuroTak 87-2051, as well as DuroTak 87-2353 showed the lowest degradation profiles of the acrylate formulations.

Example 18: Solubilizer screening

In a series of experiments under comparable conditions, patches made of silicone adhesives and various solubilizers were tested in terms of compatibility with the API free base and the recrystallization tendency for the API free base.

An overview of the different batches which were prepared is given in the table below:

| batch no. | adhesive | excipient | excipient amount [wt.-%] | API free base amount [wt.-%] |
|---|---|---|---|---|
| 18-1 | BioPSA 7-4503 | PVP (Kollidon 25) | 4.5 | 0.5 |
| 18-2 | BioPSA 7-4503 | PVP (Kollidon 25) | 9.0 | 1.0 |
| 18-3 | BioPSA 7-4503 | PVP (Kollidon 25) | 13.5 | 1.5 |
| 18-4 | BioPSA 7-4503 | Glycerol (anhydrous) | 4.5 | 0.5 |
| 18-5 | BioPSA 7-4503 | Glycerol (anhydrous) | 9.0 | 1.0 |
| 18-6 | BioPSA 7-4503 | Glycerol (anhydrous) | 13.5 | 1.5 |
| 18-7 | BioPSA 7-4503 | Polyvinylalcohol (Mowiol 4-88) | 4.5 | 0.5 |
| 18-8 | BioPSA 7-4503 | Polyvinylalcohol (Mowiol 4-88) | 9.0 | 1.0 |
| 18-9 | BioPSA 7-4503 | Polyvinylalcohol (Mowiol 4-88) | 13.5 | 1.5 |

(continued)

| batch no. | adhesive | excipient | excipient amount [wt.-%] | API free base amount [wt.-%] |
|---|---|---|---|---|
| 18-10 | BioPSA 7-4302/3 | PVP (Kollidon 25) | 4.5 | 0.5 |
| 18-11 | BioPSA 7-4302/3 | PVP (Kollidon 25) | 9.0 | 1.0 |
| 18-12 | BioPSA 7-4302/3 | PVP (Kollidon 25) | 13.5 | 1.5 |
| 18-13 | BioPSA 7-4302/3 | Glycerol (anhydrous) | 4.5 | 0.5 |
| 18-14 | BioPSA 7-4302/3 | Glycerol (anhydrous) | 9.0 | 1.0 |
| 18-15 | BioPSA 7-4302/3 | Glycerol (anhydrous) | 13.5 | 1.5 |
| 18-16 | BioPSA 7-4302/3 | Polyvinylalcohol (Mowiol 4-88) | 4.5 | 0.5 |
| 18-17 | BioPSA 7-4302/3 | Polyvinylalcohol (Mowiol 4-88) | 9.0 | 1.0 |
| 18-18 | BioPSA 7-4302/3 | Polyvinylalcohol (Mowiol 4-88) | 13.5 | 1.5 |
| 18-19 | BioPSA 7-4602/3 | PVP (Kollidon 25) | 4.5 | 0.5 |
| 18-20 | BioPSA 7-4602/3 | PVP (Kollidon 25) | 9.0 | 1.0 |
| 18-21 | BioPSA 7-4602/3 | PVP (Kollidon 25) | 13.5 | 1.5 |
| 18-22 | BioPSA 7-4602/3 | Glycerol (anhydrous) | 4.5 | 0.5 |
| 18-23 | BioPSA 7-4602/3 | Glycerol (anhydrous) | 9.0 | 1.0 |
| 18-24 | BioPSA 7-4602/3 | Glycerol (anhydrous) | 13.5 | 1.5 |
| 18-25 | BioPSA 7-4602/3 | Polyvinylalcohol (Mowiol 4-88) | 4.5 | 0.5 |
| 18-26 | BioPSA 7-4602/3 | Polyvinylalcohol (Mowiol 4-88) | 9.0 | 1.0 |
| 18-27 | BioPSA 7-4602/3 | Polyvinylalcohol (Mowiol 4-88) | 13.5 | 1.5 |

Re-crystallization was tested at the start and after 6 days at room temperature (RT). Square-shaped and needle-shaped crystals could be detected. The results are summarized in the table below:

| batch no. | API free base amount [wt.-%] | excipient | start [morphology] | 6 days/RT [morphology] |
|---|---|---|---|---|
| 18-1 | 0.5 | PVP (Kollidon 25) | - | - |
| 18-2 | 1.0 | PVP (Kollidon 25) | + [0] | + [0] |
| 18-3 | 1.5 | PVP (Kollidon 25) | + [o] | + [o] |
| 18-4 | 0.5 | Glycerol (anhydrous) | + [-] | + [-] |
| 18-5 | 1.0 | Glycerol (anhydrous) | + [-] | + [-] |
| 18-6 | 1.5 | Glycerol (anhydrous) | + [-] | + [-] |

(continued)

| batch no. | API free base amount [wt.-%] | excipient | start [morphology] | 6 days/RT [morphology] |
|---|---|---|---|---|
| 18-7 | 0.5 | Polyvinylalcohol (Mowiol 4-88) | + [0] | - |
| 18-8 | 1.0 | Polyvinylalcohol (Mowiol 4-88) | + [0] | (+) [0] |
| 18-9 | 1.5 | Polyvinylalcohol (Mowiol 4-88) | + [0] | (+) [0] |
| 18-10 | 0.5 | PVP (Kollidon 25) | - | - |
| 18-11 | 1.0 | PVP (Kollidon 25) | + [-] | + [-] |
| 18-12 | 1.5 | PVP (Kollidon 25) | + [-] | + [-] |
| 18-13 | 0.5 | Glycerol (anhydrous) | + [-] | + [-] |
| 18-14 | 1.0 | Glycerol (anhydrous) | + [-] | + [-] |
| 18-15 | 1.5 | Glycerol (anhydrous) | + [-] | + [-] |
| 18-16 | 0.5 | Polyvinylalcohol (Mowiol 4-88) | + [-] | + [0] |
| 18-17 | 1.0 | Polyvinylalcohol (Mowiol 4-88) | + [0] | + [0] |
| 18-18 | 1.5 | Polyvinylalcohol (Mowiol 4-88) | + [0] | + [0] |
| 18-19 | 0.5 | PVP (Kollidon 25) | - | - |
| 18-20 | 1.0 | PVP (Kollidon 25) | - | - |
| 18-21 | 1.5 | PVP (Kollidon 25) | - | - |
| 18-22 | 0.5 | Glycerol (anhydrous) | + [-] | + [-] |
| 18-23 | 1.0 | Glycerol (anhydrous) | + [-] | + [-] |
| 18-24 | 1.5 | Glycerol (anhydrous) | + [-] | + [-] |
| 18-25 | 0.5 | Polyvinylalcohol (Mowiol 4-88) | + [-] | + [-] |
| 18-26 | 1.0 | Polyvinylalcohol (Mowiol 4-88) | + [-] | + [-] |
| 18-27 | 1.5 | Polyvinylalcohol (Mowiol 4-88) | + [-] | + [-] |
| - : no crystals; +: crystals; (+): single crystals; [o]: square-shaped crystals; [-]: needle-shaped crystals | | | | |

Two silicone adhesives (BioPSA 7-4503 and BioPSA 7-4603) in combination with either PVP or PVA showed

promising results.

The results of a stress stability testing of API free base containing patch samples at 60°C over 6 days are summarized in the table below:

| | | API free base Assay [%] | DEGR1 [%] | DEGR2 [%] |
|---|---|---|---|---|
| 18-1 | Start | 99.3 | 0.22 | - |
| | 6d 60°C | 101.0 | 0.35 | 0.32 |
| 18-2 | Start | 98.1 | 0.07 | - |
| | 6d 60°C | 99.5 | 0.28 | 0.26 |
| 18-3 | Start | 99.1 | - | - |
| | 6d 60°C | 99.0 | 0.15 | 0.14 |
| 18-4 | Start | 99.0 | 0.25 | - |
| | 6d 60°C | 100.5 | 0.33 | 0.12 |
| 18-5 | Start | 99.1 | 0.20 | - |
| | 6d 60°C | 99.9 | 0.20 | 0.09 |
| 18-6 | Start | 100.0 | - | - |
| | 6d 60°C | 100.7 | 0.08 | 0.06 |
| 18-7 | Start | 96.4 | 0.25 | - |
| | 6d 60°C | 99.9 | 0.28 | 0.09 |
| 18-8 | Start | 82.4 | 0.11 | - |
| | 6d 60°C | 84.0 | 0.14 | 0.06 |
| 18-9 | Start | 75.4 | - | - |
| | 6d 60°C | 74.5 | 0.12 | 0.06 |
| 18-10 | Start | 96.3 | 0.08 | - |
| | 6d 60°C | 101.3 | 0.32 | 0.31 |
| 18-11 | Start | 101.5 | 0.92 | - |
| | 6d 60°C | 111.3 | 0.17 | 0.12 |
| 18-12 | Start | 101.8 | - | - |
| | 6d 60°C | 99.7 | 0.19 | 0.11 |
| 18-13 | Start | 96.9 | 0.24 | - |
| | 6d 60°C | 100.9 | 0.28 | 0.10 |
| 18-14 | Start | 102.1 | 1.14 | - |
| | 6d 60°C | 98.7 | 0.12 | 0.09 |
| 18-15 | Start | 98.4 | - | - |
| | 6d 60°C | 97.8 | 0.06 | 0.11 |
| 18-16 | Start | 100.7 | 0.15 | - |
| | 6d 60°C | 103.2 | 0.23 | 0.32 |
| 18-17 | Start | 63.9 | 0.29 | - |
| | 6d 60°C | 61.8 | 0.15 | 0.12 |
| 18-18 | Start | 104.6 | - | - |
| | 6d 60°C | 98.9 | 0.08 | 0.09 |

(continued)

| | | API free base Assay [%] | DEGR1 [%] | DEGR2 [%] |
|---|---|---|---|---|
| 18-19 | Start | 97.5 | - | - |
| | 6d 60°C | 98.1 | 0.31 | 0.17 |
| 18-20 | Start | 100.3 | 0.99 | - |
| | 6d 60°C | 99.0 | 0.15 | 0.16 |
| 18-21 | Start | 96.1 | - | - |
| | 6d 60°C | 96.5 | 0.17 | 0.27 |
| 18-22 | Start | 98.0 | - | - |
| | 6d 60°C | 98.0 | 0.28 | 0.08 |
| 18-23 | Start | 93.5 | 1.43 | - |
| | 6d 60°C | 95.9 | 0.13 | 0.08 |
| 18-24 | Start | 99.8 | - | - |
| | 6d 60°C | 101.1 | 0.05 | 0.08 |
| 18-25 | Start | 84.6 | - | - |
| | 6d 60°C | 83.3 | 0.25 | 0.10 |
| 18-26 | Start | 96.6 | 1.47 | - |
| | 6d 60°C | 96.2 | 0.11 | 0.17 |
| 18-27 | Start | 84.9 | - | - |
| | 6d 60°C | 84.3 | 0.11 | 0.26 |

All formulations containing 1.5% API free base and 13.5% hydrophilic polymer showed an acceptable degradation profile.

Example 19: Characterization of polymer screening prototypes

In a series of experiments under comparable conditions, patches of identical basic compositions but of different polymers were tested in terms of recrystallization of the API free base and permeation across a synthetic EVA membrane.

An overview of the different batches which were prepared is given in the table below:

| batch no. | adhesive | API free base amount [wt.-%] |
|---|---|---|
| 19-1 | DuroTak 87-6908 (polyisobutylene) | 0.75 |
| 19-2 | DuroTak 87-6908 (polyisobutylene) | 0.50 |
| 19-3 | DuroTak 87-6908 (polyisobutylene) | 0.25 |
| 19-4 | DuroTak 87-6911 (styrenic rubber) | 0.75 |
| 19-5 | DuroTak 87-6911 (styrenic rubber) | 0.50 |
| 19-6 | DuroTak 87-6911 (styrenic rubber) | 0.25 |
| 19-7 | DuroTak 87-2051 (acrylate-vinylacetate) | 0.75 |
| 19-8 | DuroTak 87-2051 (acrylate-vinylacetate) | 1.00 |
| 19-9 | DuroTak 87-2051 (acrylate-vinylacetate) | 1.25 |
| 19-10 | DuroTak 87-2353 (acrylate) | 0.75 |
| 19-11 | DuroTak 87-2353 (acrylate) | 1.00 |
| 19-12 | DuroTak 87-2353 (acrylate) | 1.25 |

(continued)

| batch no. | adhesive | API free base amount [wt.-%] |
|---|---|---|
| 19-13 (reference) | DuroTak 87-4287 (acrylate) | 0.75 |

Re-crystallization was tested at the start and after 4 weeks at 25°C/60% r.h. (relative humidity) as well as 40°C/75% r.h. The results are summarized in the table below:

| batch no. | API free base content [wt.-%] | start [morphology] | 4 weeks/25 °C/60% r.h. [morphology] | 4 weeks/40 °C/75% r.h. [morphology] |
|---|---|---|---|---|
| 19-1 | 0.75 | +<br>[-] | +<br>[-] | +<br>[-] |
| 19-2 | 0.50 | + | + | + |
| | | [-] | [-] | [-] |
| 19-3 | 0.25 | +<br>[-] | +<br>[-] | +<br>[-] |
| 19-4 | 0.75 | +<br>[0] | +<br>[o] | +<br>[0] |
| 19-5 | 0.50 | (+)<br>[0] | +<br>[0] | +<br>[0] |
| 19-6 | 0.25 | - | - | - |
| 19-7 | 0.75 | - | - | - |
| 19-8 | 1.00 | - | - | - |
| 19-9 | 1.25 | - | - | - |
| 19-10 | 0.75 | - | - | - |
| 19-11 | 1.00 | - | - | - |
| 19-12 | 1.25 | - | - | - |
| - : no crystals; +: crystals;<br>[0]: square-shaped crystals; [-]: needle-shaped crystals | | | | |

Re-crystallization occurred in the isobutylene as well as in the styrenic rubber adhesive at higher concentrations. Both acrylate adhesives showed no recrystallization events.

API free base containing patch samples were tested on assay and purity after manufacturing. The results are summarized in the table below:

| | 19-1 | 19-2 | 19-3 | 19-4 | 19-5 | 19-6 | 19-7 | 19-8 | 19-9 | 19-10 | 19-11 | 19-12 | 19-13 (ref.) |
|---|---|---|---|---|---|---|---|---|---|---|---|---|---|
| API free base [%] | 88.9 | 80.8 | 49.5 | 98.4 | 89.6 | 106.9 | 98.5 | 99.7 | 101.3 | 98.4 | 100.5 | 99.3 | 110.5 |
| DEGR1 [%] | 0.06 | 0.08 | 0.15 | / | / | / | 0.16 | 0.14 | 0.12 | 0.19 | 0.16 | 0.14 | 0.66 |
| DEGR2 [%] | / | / | / | 0.14 | 0.16 | 0.33 | / | / | / | / | / | / | 0.18 |

The permeation through an EVA membrane was tested.

Figure 19 shows the EVA membrane permeation testing of polyisobutylene formulations containing API free base (19-1, 19-2, 19-3) vs. an acrylate reference patch (19-13, composition 12D). All three API concentrations showed a lower flux rate over the EVA membrane compared to the reference patch 19-13.

Figure 20 shows the EVA membrane permeation testing of styrenic rubber formulations containing API free base (19-4, 19-5, 19-6) vs. an acrylate reference patch (19-13, composition 12D). All three API concentrations showed a higher flux rate over the EVA membrane compared to the reference patch 19-13.

The EVA membrane permeation testing of API free base containing prototypes vs. an acrylate reference patch (19-13) is also summarized in the table below:

| | adhesive | steady-state interval [h] | correlation coefficient [$r^2$] | steady-state flux rate [µg/cm$^2$/h] | factor |
|---|---|---|---|---|---|
| 19-13 (ref.) | DuroTak 87-4287 (acrylate) | 8 - 72 | 0.999 | 0.036 | 1.00 |
| 19-1 | DuroTak 87-6908 (polyisobutylene) | 8 - 72 | 0.999 | 0.009 | 0.25 |
| 19-2 | | 8 - 72 | 0.996 | 0.008 | 0.22 |
| 19-3 | | 8 - 72 | 0.999 | 0.009 | 0.25 |
| 19-4 | DuroTak 87-6911 (styrenic rubber) | 6 - 22 | 1.000 | 0.081 | 2.25 |
| 19-5 | | 6 - 22 | 0.998 | 0.075 | 2.08 |
| 19-6 | | 8 - 72 | 0.999 | 0.056 | 1.56 |
| 19-7 | DuroTak 87-2051 (acrylate-vinylacetate) | 0 - 72 | 1.000 | 0.001 | - |
| 19-8 | | 8 - 30 | 0.997 | <0.001 | - |
| 19-9 | | 22 - 46 | 0.999 | 0.001 | - |
| 19-10 | DuroTak 87-2353 (acrylate) | 22 - 46 | 0.997 | <0.001 | - |
| 19-11 | | 22 - 46 | 0.999 | <0.001 | - |
| 19-12 | | 22 - 46 | 0.997 | <0.001 | - |

It becomes evident from the above table that the styrenic rubber polymer DuroTak 87-6911 shows increased permeation rate while being lower concentrated in comparison to an acrylate reference patch 12D.

Example 20: EVA permeation testing of silicone prototypes

Following silicone patches were tested:

| batch no. | adhesive | excipient | excipient amount [wt.-%] | API free base amount [wt.-%] |
|---|---|---|---|---|
| 18-7 | BioPSA 7-4503 | Polyvinylalcohol (Mowiol 4-88) | 4.5 | 0.5 |
| 18-8 | BioPSA 7-4503 | Polyvinylalcohol (Mowiol 4-88) | 9.0 | 1.0 |
| 18-9 | BioPSA 7-4503 | Polyvinylalcohol (Mowiol 4-88) | 13.5 | 1.5 |
| 18-19 | BioPSA 7-4602/3 | PVP (Kollidon 25) | 4.5 | 0.5 |
| 18-21 | BioPSA 7-4602/3 | PVP (Kollidon 25) | 13.5 | 1.5 |

The permeation through an EVA membrane was tested.

Figure 21 shows the EVA membrane permeation testing of silicone/PVA formulations containing API free base (18-7, 18-8, 18-9) vs. an acrylate reference patch (19-13, composition 12D).

Figure 22 shows the EVA membrane permeation testing of silicone/PVP formulations containing API free base (18-19, 18-21) vs. an acrylate reference patch (19-13, composition 12D).

The EVA membrane permeation testing of API free base containing prototypes vs. an acrylate reference patch (19-13) is also summarized in the table below:

| | adhesive | steady-state interval [h] | correlation coefficient [$r^2$] | steady-state flux rate [$\mu g/cm^2/h$] | factor |
|---|---|---|---|---|---|
| 19-13 (ref.) | DuroTak 87-4287 (acrylate) | 19-72 | 0.999 | 0.047 | 1.00 |
| 18-7 | BioPSA 7-4503 PVA | 6-72 | 0.997 | 0.028 | 0.60 |
| 18-8 | | 6-72 | 0.995 | 0.037 | 0.79 |
| 18-9 | | 19-72 | 0.998 | 0.095 | 2.02 |
| 18-19 | BioPSA 7-4602/3 PVP | 30-72 | 0.996 | 0.048 | 1.02 |
| 18-21 | | 8-72 | 0.999 | 0.108 | 2.30 |

It becomes evident from the above table that both, the silicone/PVA and the silicone/PVP formulation with 1.5% API free base shows increased permeation rate in comparison to an acrylate reference patch 12D.

Example 21: Solubility and permeation of acrylate prototypes

In a series of experiments under comparable conditions, patches of identical basic compositions of two acrylate adhesives were tested in terms of saturation solubility of the API free base and permeation across a synthetic EVA membrane.

An overview of the different batches which were prepared is given in the table below:

| batch no. | adhesive | API free base amount [wt.-%] |
|---|---|---|
| 21-1 | DuroTak® 87-2353 | 3.0 |
| 21-2 | DuroTak® 87-2353 | 3.5 |
| 21-3 | DuroTak® 87-2353 | 4.0 |
| 21-4 | DuroTak® 87-2051 | 3.0 |
| 21-5 | DuroTak® 87-2051 | 3.5 |
| 21-6 | DuroTak® 87-2051 | 4.0 |

Re-crystallization was not observed at the start, and neither after 7 days at 25°C/60% r.h. (relative humidity) or 40°C/75% r.h. Even with 4% API free base in the polymers, the patch samples showed no re-crystallization over a time period of 7 days.

API free base containing patch samples were tested on assay and purity after manufacturing. The results are summarized in the table below:

| | 21-1 | 21-2 | 21-3 | 21-5 | 24-7 | 21-6 | 19-13 (ref.) |
|---|---|---|---|---|---|---|---|
| API free base [%] | 99.1 | 100.6 | 108.3 | 99.6 | 99.5 | 100.6 | 110.5 |
| DEGR1 [%] | / | 0.02 | / | / | 0.05 | 0.04 | 0.66 |
| DEGR2 [%] | 0.07 | 0.04 | / | 0.05 | 0.06 | 0.05 | 0.18 |

The permeation through an EVA membrane was tested.

Figure 23 shows the EVA membrane permeation testing of Duro Tak® 87-2353 formulations containing API free base (21-1, 21-2, 21-3) vs. an acrylate reference patch (19-13, composition 12D). All tested DuroTak® 87-2353 showed a significant lower flux rate compared to the reference patch.

Figure 24 shows the EVA membrane permeation testing of DuroTak® 87-2051 formulations containing API

free base (21-5, 21-6, 24-7) vs. an acrylate reference patch (19-13, composition 12D). All tested DuroTak® 87-2051 showed a significant lower flux rate compared to the reference patch.

The EVA membrane permeation testing of API free base containing prototypes vs. an acrylate reference patch (19-13) is also summarized in the table below:

| | adhesive | steady-state interval [h] | correlation coefficient [$r^2$] | steady-state flux rate [μg/cm$^2$/h] |
|---|---|---|---|---|
| 19-13 (ref.) | DuroTak 87-4287 (acrvlate) | 19-72 | 0.999 | 0.037 |
| 21-1 | DuroTak 87-2353 | 8-72 | 0.998 | 0.001 |
| 21-2 | | 8-54 | 0.996 | 0.001 |
| 21-3 | | 8-54 | 0.999 | 0.001 |
| 21-5 | DuroTak 87-2051 | 30-72 | 0.999 | 0.001 |
| 24-7 | | 30-72 | 0.995 | 0.001 |
| 21-6 | | 8-46 | 0.998 | 0.001 |

Both acrylate adhesive have acidic functional groups. It is likely that an interaction with API free base occurs so that a saturation of the polymer with the API free base could not reached even at a high concentration of 4% API free base. The low flux rate observed is a consequence of the non-saturated system.

Example 22: Eudragit® EPO loading in Duro Tak® 87-4287

In a series of experiments under comparable conditions, patches made of acrylate DuroTak 87-4287 and Eudragit EPO were tested in terms of adhesiveness.

An overview of the different batches which were prepared is given in the table below:

| number | DuroTak® 87-4287 amount [%] | Eudragit® EPO amount [%] |
|---|---|---|
| I | 100 | 0 |
| II | 90 | 10 |
| III | 80 | 20 |
| IV | 70 | 30 |
| V | 60 | 40 |
| VI | 50 | 50 |

The *in vitro* peel strength of Duro Tak® 87-4287 prototypes containing different amounts of Eudragit® EPO was determined. The results are summarized in the table below:

| | Eudragit® EPO amount [%] | mean $F_{max}$ (n=6) [N/25 mm] | SD $F_{max}$ (n=6) [N/25 mm] | mean $F_{mean}$ (n=6) [N/25 mm] | SD $F_{mean}$ (n=6) [N/25 mm] |
|---|---|---|---|---|---|
| reference | 0 | 5.3 | 0.57 | 4.8 | 0.51 |
| I | 0 | 12.6 | 0.54 | 10.7 | 0.73 |
| II | 10 | * | * | * | * |
| III | 20 | 7.1 | 0.18 | 6.0 | 0.32 |
| IV | 30 | 5.8 | 0.46 | 4.9 | 0.45 |
| V | 40 | 5.7 | 0.79 | 4.7 | 0.65 |
| VI | 50 | 5.0 | 0.77 | 3.5 | 0.43 |

(continued)

| | Eudragit® EPO amount [%] | mean $F_{max}$ (n=6) [N/25 mm] | SD $F_{max}$ (n=6) [N/25 mm] | mean $F_{mean}$ (n=6) [N/25 mm] | SD $F_{mean}$ (n=6) [N/25 mm] |
|---|---|---|---|---|---|
| *no data obtained | | | | | |

A decrease of peel strength with increasing methacrylate amount could be detected. No differences *in vivo* between formulations III to VI could be seen (data not shown).

Example 23: Polymer screening of acrylates

In a series of experiments under comparable conditions, patches made of various acrylate adhesives were tested in terms of saturation solubility and stability of API free base and permeation across a synthetic EVA membrane..

The saturation concentrations of API free base in the coating masses were determined. An overview of the different batches which were prepared is given in the table below:

| batch | adhesive | adhesive solvent | API solvent | saturation solubility of API free base [%] |
|---|---|---|---|---|
| 23-1 | DuroTak 87-4287/ Eudragit® EPO | Ethyl acetate | Toluene/2-Propanol | 1.2 |
| 23-2 | DuroTak 87-2516 | Ethyl acetate, Ethyl alcohol, | Toluene/2-Propanol | 0.8 |
| | | Heptane, Methyl alcohol | | |
| 23-3 | DuroTak 87-208A | Ethyl acetate | Toluene/2-Propanol | 1.2 |
| 23-4 | DuroTak 87-9088 | Ethyl acetate | Toluene/2-Propanol | 0.9 |

Over a time period of four weeks at 25°C/65% r.h. and 40°C/75% r.h. there was no re-crystallization of any of patch systems 23-1 to 23-4, neither with nor without seeding crystals.

The assay under stress stability conditions at 60°C over a period of 6 days was determined. The results are summarized in the table below:

| batch | API free base assay at start [n=3] | | API free base assay after 6 days at 60°C [n=3] | |
|---|---|---|---|---|
| | amount [%] | SD [%] | amount [%] | SD [%] |
| 23-1 | 104.1 | 1.36 | 103.7 | 1.22 |
| 23-2 | 100.4 | 0.62 | 97.8 | 1.53 |
| 23-3 | 98.2 | 0.72 | 97.1 | 0.50 |
| 23-4 | 93.8 | 1.78 | 94.2 | 0.45 |

The purity under stress stability conditions at 60°C for 6 days was determined. The results are summarized in the table below:

| | time [days] | known impurities (n=3) [%] | | unknown impurities (n=3) [%] | | | | | |
|---|---|---|---|---|---|---|---|---|---|
| | | DEGR1 | DEGR2 | RRT0.27 | RRT0.36 | RRT0.54 | RRT0.60 | RRT0.81 | RRT0.93 |
| 23-1 | 0 | - | - | 0.32 | 0.28 | 0.53 | - | - | - |
| | 6 | - | 0.11 | 0.33 | 0.33 | 0.52 | - | - | - |
| 23-2 | 0 | 0.39 | 0.29 | 0.46 | 0.36 | 0.96 | 0.10 | 0.14 | - |
| | 6 | 0.67 | 0.52 | 0.49 | 0.36 | 0.89 | - | 0.23 | - |
| 23-3 | 0 | 0.51 | 0.19 | 0.43 | 0.33 | 1.09 | - | 0.15 | - |
| | 6 | 0.82 | 0.37 | 0.47 | 0.43 | 1.18 | - | 0.21 | - |
| 23-4 | 0 | 1.68 | - | 0.36 | 0.31 | 1.60 | - | - | - |
| | 6 | 2.51 | 0.33 | 0.46 | 0.58 | 2.51 | - | 0.19 | 0.17 |
| RRT0.27, RRT0.36, RRT0.54, RRT0.60, RRT0.81 and RRT0.93 are unknown impurities characterized by a specific HPLC retention time | | | | | | | | | |

Figure 25 shows the EVA membrane permeation testing of acrylate formulations containing API free base (23-1 to 23-4) vs. an acrylate reference patch (19-13, composition 12D).

The EVA membrane permeation testing of API free base containing prototypes vs. an acrylate reference patch (19-13) is also summarized in the table below:

| | adhesive | steady-state interval [h] | correlation coefficient [$r^2$] | steady-state flux rate [$\mu g/cm^2/h$] | factor |
|---|---|---|---|---|---|
| 19-13 | DuroTak 87-4287 (acrylate) | 8-46 | 0.990 | 0.040 | 1.00 |
| 23-1 | DuroTak 87-4287/ Eudragit® EPO | 8-54 | 0.991 | 0.076 | 1.90 |
| 23-2 | DuroTak 87-2516 | 8-46 | 0.993 | 0.045 | 1.13 |
| 23-3 | DuroTak 87-208A | 8-54 | 0.995 | 0.064 | 1.60 |
| 23-4 | DuroTak 87-9088 | 8-54 | 0.990 | 0.072 | 1.80 |

It becomes evident from the above tables that a combination of DuroTak 87-4287 and Eudragit EPO lead to reduced degradation of the API free base. Furthermore, this formulation shows increased permeation increased rate in comparison to an acrylate reference patch 12D.

Example 24: Enhancer screening

The solubility of API free base in different liquid permeation enhancer substances was determined. The results are summarized in the table below:

| enhancer | solubility of API free base [mg/mL] |
|---|---|
| Laurocapram (Azone®) | 20.0 |
| Levulinic acid | 11.7 |
| Diethylene glycol monoethyl ether (Transcutol P) | 6.3 |
| Benzyl alcohol | 5.3 |
| Polyethylene glycol 400 | 3.9 |
| Lauryl lactate | 2.6 |

(continued)

| enhancer | solubility of API free base [mg/mL] |
|---|---|
| Propylene glycol monolaurate | 1.8 |
| Dibutylene sebacate | 1.5 |
| Oleyl oleate | 1.4 |
| 1-Dodecanol | 1.3 |
| Caprvlic acid | 1.2 |
| Polvsorbate 80 | 0.8 |
| Isopropyl myristate | 0.7 |
| Glycerol | 0.7 |
| Triacetine | 0.6 |
| Isopropyl palmitate | 0.5 |
| Ethyl oleate | 0.5 |

Out of the substances mentioned in the table above, the following enhancers were selected to be tested further.

E1 Oleyloleate
E2 Isopropylmyristate
E3 Levulinic acid
E4 Transcutol
E5 Triacetin
E6 Laurocapram
E7 Dodecanol
E8 PEG400

The permeation of API free base across dermatomized pig skin (incl. stratum corneum) from saturated solutions in enhancers E1 to E8 has been investigated, results are shown in Figure 15.
It becomes evident from Figure 15 that Levulinic acid and Transcutol demonstrated both, absolute and relative, highest flux rates from saturated solutions of API free base in liquid enhancers across dermatomized pig skin.

Example 25: Eudragit® EPO containing polymer systems

In a series of experiments under comparable conditions, patches made of acrylate adhesive DuroTak 87-4287 or styrenic rubber adhesive DuroTak 87-6911 in combination with Eudragit EPO were tested in terms of saturation solubility and stability of the API free base and permeation across a synthetic EVA membrane.
The saturation concentration of API free base in Eudragit® EPO containing polymer systems was determined. The results are summarized in the table below:

| batch | adhesive | saturation solubility of API free base [%] |
|---|---|---|
| 25-1 | 90% DuroTak 87-4287/ 10% Eudragit® EPO | 1.00 |
| 23-1 | 50% DuroTak 87-4287/ 50% Eudragit® EPO | 1.20 |
| 25-2 | 90% DuroTak 87-208A/ 10% Eudragit® EPO | 1.00 |
| 25-3 | 50% DuroTak 87-208A/ 50% Eudragit® EPO | 1.00 |
| 25-4 | 50% DuroTak 87-6911/ 50% Eudragit® EPO | 0.25 |
| 25-5 | 50% DuroTak 87-6911/ 50% Eudragit® EPO | 0.30 |

By adding more Eudragit® EPO to the formulation no or only a low increase of the saturation concentration of API free base in the patch could be obtained.

Over a time period of four weeks at 25°C/60% r.h. and 40°C/75% r.h. there was no re-crystallization of any of patch systems 25-1 to 25-5 and 23-4, neither with nor without seeding crystals. The patches were stored in Surlyn® pouches.

Stress stability was tested at 60°C over a period of 6 days in Surlyn® pouches. The results are summarized in the table below:

| batch | adhesive | Eudragit [%] | time [days] | API free base [%] | DEGR1 [%] | DEGR2 [%] |
|---|---|---|---|---|---|---|
| 25-1 | DuroTak® 87-4287 | 10 | 0 | 104 | 0.04 | 0.13 |
| | | | 6 | 94 | 0.12 | 0.25 |
| 23-1 | | 50 | 0 | 104 | - | - |
| | | | 6 | 104 | - | 0.11 |
| 25-2 | DuroTak® 87-208A | 10 | 0 | 99 | 0.04 | 0.10 |
| | | | 6 | 92 | 0.12 | 0.23 |
| 25-3 | | 50 | 0 | 102 | - | 0.08 |
| | | | 6 | 95 | 0.04 | 0.21 |
| 25-4 | DuroTak® 87-6911 | 10 | 0 | 86 | - | 0.17 |
| | | | 6 | 90 | 0.17 | 0.22 |
| 25-5 | | 50 | 0 | 103 | - | - |
| | | | 6 | 99 | - | 0.10 |

It becomes evident from above table that by increasing the Eudragit® EPO amount in the formulation the amount of degradation products could be decreased.

Figure 26 shows the EVA membrane permeation testing of Eudragit® EPO containing adhesives vs. an acrylate reference patch (19-13, composition 12D). In the case of DuroTak® 87-208A and DuroTak® 87-6911 an increase of the Eudragit® EPO amount decreased the thermodynamic activity. For DuroTak® 87-4287 an increase of the Eudragit® EPO content yielded an increase of the thermodynamic activity.

The EVA membrane permeation data and calculation of flux rate across the membrane is summarized in the table below:

| | adhesive | steady-state interval [h] | correlation coefficient [$r^2$] | steady-state flux rate [μg/cm$^2$/h] | factor |
|---|---|---|---|---|---|
| 19-13 | DuroTak 87-4287 (acrylate) | 8-54 | 0.990 | 0.050 | 1.00 |
| 25-1 | 90% DuroTak 87-4287/ 10% Eudragit® EPO | 19-54 | 0.995 | 0.061 | 1.22 |
| 23-1 | 50% DuroTak 87-4287/ 50% Eudragit® EPO | 19-54 | 0.999 | 0.076 | 1.52 |
| 25-2 | 90% DuroTak 87-208A/ 10% Eudragit® EPO | 8-54 | 0.997 | 0.058 | 1.16 |
| 25-3 | 50% DuroTak 87-208A/ 50% Eudragit® EPO | 8-54 | 1.000 | 0.039 | 0.78 |
| 25-4 | 50% DuroTak 87-6911/ 50% Eudragit® EPO | 8-54 | 0.999 | 0.047 | 0.94 |
| 25-5 | 50% DuroTak 87-6911/ 50% Eudragit® EPO | 8-54 | 0.997 | 0.025 | 0.50 |

It becomes evident from the above tables that Eudragit EPO lead to reduced degradation of the API free base. In addition, an increased permeation rate in comparison to an acrylate reference patch 12D could be observed in combination with acrylate adhesive DuroTak 87-4287 but not in combination with the styrenic rubber adhesive DuroTak

87-6911.

## Claims

1. A pharmaceutical patch for transdermal administration of the pharmacologically active ingredient (1r,4r)-6'-fluoro-N,N-dimethyl-4-phenyl-4',9'-dihydro-3'H-spiro-[cyclohexane-1,1'-pyrano[3,4-b]indol]-4-amine, the patch comprising a surface layer, an adhesive layer, and a removable protective layer, wherein

    - the adhesive layer is located between the surface layer and the removable protective layer;
    - the adhesive layer comprises at least a portion of the total amount of the pharmacologically active ingredient that is contained in the pharmaceutical patch;
    - the concentration of the pharmacologically active ingredient in the adhesive layer is at least 0.50 wt.-%, relative to the total weight of the adhesive layer; and
    - the adhesive layer comprises a polyacrylate based pressure sensitive adhesive.

2. The pharmaceutical patch according to any of the preceding claims, wherein the surface layer has a thickness within the range of from 0.1 to 5000 $\mu$m and/or the adhesive layer has a thickness within the range of from 1.0 to 1000 $\mu$m.

3. The pharmaceutical patch according to any of the preceding claims, wherein the adhesive layer comprises a copolymer comprising monomer units originating from monomers A which are selected from $C_{1-18}$-alkyl (meth)acrylates and/or monomers B which are copolymerizable with monomers A.

4. The pharmaceutical patch according to claim 3, wherein

    (i) monomers A are selected from the group consisting of methyl (meth)acrylate, ethyl (meth)acrylate, propyl (meth)acrylate, butyl (meth)acrylate, pentyl (meth)acrylate, hexyl (meth)acrylate, cyclohexyl (meth)acrylate, octyl (meth)acrylate, isobornyl (meth)acrylate, and mixtures thereof; and/or
    (ii) monomers B are selected from the group consisting of 2-hydroxyethyl (meth)acrylate, glyceryl mono(meth)acrylate, glycidyl (meth)acrylate, acrylamide, N,N-diethyl(meth)acrylamide, 2-ethoxyethyl (meth)acrylate, 2-ethoxyethoxyethyl (meth)acrylate, tetrahydrofuryl (meth)acrylate, vinyl acetate, N-vinyl pyrrolidone and mixtures thereof.

5. The pharmaceutical patch according to claim 3 or 4, wherein the acrylate copolymer is derived from a monomer composition comprising vinyl acetate, 2-ethylhexyl acrylate and 2-hydroxyethyl acrylate, optionally also comprising glycidyl methacrylate.

6. The pharmaceutical patch according to any of the preceding claims, wherein the adhesive layer comprises a permeation component which enhances permeation of the pharmacologically active ingredient through human skin.

7. The pharmaceutical patch according to claim 6, wherein the relative weight ratio of the pharmacologically active ingredient to the permeation component is within the range of from 1 : 1 to 1 : 500.

8. The pharmaceutical patch according to claim 6 or 7, wherein the permeation component comprises a non-cyclic compound of formula $C_{2n}H_{4n+2}O_n$, where index n is 2, 3 or 4.

9. The pharmaceutical patch according to claim 8, wherein the non-cyclic compound is diethylene glycol monomethylether, dipropylene glycol or a mixture thereof.

10. The pharmaceutical patch according to any of the preceding claims, wherein the permeation component comprises an organic acid; preferably levulinic acid.

11. The pharmaceutical patch according to any of the preceding claims, wherein the adhesive layer comprises an antioxidant.

12. The pharmaceutical patch according to any of the preceding claims, wherein the adhesive layer has an area within the range of from 10 to 750 $cm^2$.

**13.** The pharmaceutical patch according to any of the preceding claims, which upon application to the human skin provides

- release of the pharmacologically active ingredient at a rate of at least 1.0 $ng \cdot cm^{-2} \cdot h^{-1}$ over a period of at least 6 hours; or
- plasma concentrations of the pharmacologically active ingredient of at least 10 $pg \cdot ml^{-1}$ over a period of at least 6 hours.

## Patentansprüche

**1.** Ein pharmazeutisches Pflaster zur transdermalen Verabreichung des pharmazeutisch aktiven Bestandteils (1r,4r)-6'-Fluor-N,N-dimethyl-4-phenyl-4',9'-dihydro-3'H-spiro[cyclohexan-1,1'-pyrano[3,4-b]indol]-4-amin, wobei das Pflaster eine Oberflächenschicht, eine Klebstoffschicht und eine entfernbare Schutzschicht umfasst, wobei

- die Klebstoffschicht zwischen der Oberflächenschicht und der entfernbaren Schutzschicht angeordnet ist;
- die Klebstoffschicht wenigstens einen Teil der Gesamtmenge des pharmazeutisch aktiven Bestandteils umfasst, der in dem pharmazeutischen Pflaster enthalten ist;
- die Konzentration des pharmazeutisch aktiven Bestandteils in der Klebstoffschicht wenigstens 0,50 Gew.-% ist, bezogen auf das Gesamtgewicht der Klebstoffschicht; und
- die Klebstoffschicht einen Polyacrylat-basierten druckempfindlichen Klebstoff umfasst.

**2.** Das pharmazeutische Pflaster gemäß einem der vorhergehenden Ansprüche, wobei die Oberflächenschicht eine Dicke innerhalb des Bereichs von ab 0,1 bis 5000 μm aufweist und/oder die Klebstoffschicht eine Dicke innerhalb des Bereichs von ab 1,0 bis 1000 μm aufweist.

**3.** Das pharmazeutische Pflaster gemäß einem der vorhergehenden Ansprüche, wobei die Klebstoffschicht ein Copolymer umfassend Monomereinheiten abgeleitet von Monomeren A umfasst, die ausgewählt werden aus $C_{1-18}$-Alkyl(meth)acrylaten, und/oder von Monomeren B, die mit den Monomeren A copolymerisierbar sind.

**4.** Das pharmazeutische Pflaster gemäß Anspruch 3, wobei

(i) Monomere A ausgewählt werden aus der Gruppe bestehend aus Methyl(meth)acrylat, Ethyl(meth)acrylat, Propyl(meth)acrylat, Butyl(meth)acrylat, Pentyl(meth)acrylat, Hexyl(meth)acrylat, Cyclohexyl(meth)acrylat, Octyl(meth)acrylat, Isobornyl(meth)acrylat und deren Mischungen; und/oder
(ii) Monomere B ausgewählt aus der Gruppe bestehend aus 2-Hydroxyethyl(meth)acrylat, Glycerylmono(meth)acrylat, Glycidyl(meth)acrylat, Acrylamid, N,N-Diethyl(meth)acrylamid, 2-Ethoxyethyl(meth)acrylat, 2-Ethoxyethoxyethyl(meth)acrylat, Tetrahydrofuryl(meth)acrylat, Vinylacetat, N-Vinylpyrrolidon und deren Mischungen.

**5.** Das pharmazeutische Pflaster gemäß Anspruch 3 oder 4, wobei das Acrylatcopolymer abgeleitet wird von einer Monomerzusammensetzung umfassend Vinylacetat, 2-Ethylhexylacrylat und 2-Hydroxyethylacrylat, gegebenenfalls ebenfalls umfassend Glycidylmethacrylat.

**6.** Das pharmazeutische Pflaster gemäß einem der vorhergehenden Ansprüche, wobei die Klebstoffschicht einen Permeationsbeschleuniger umfasst, der die Durchlässigkeit des pharmazeutisch aktiven Bestandteils durch die menschliche Haut verbessert.

**7.** Das pharmazeutische Pflaster gemäß Anspruch 6, wobei das relative Gewichtsverhältnis des pharmazeutisch aktiven Bestandteils zu dem Permeationsbeschleuniger innerhalb des Bereichs von ab 1 : 1 bis 1 : 500 liegt.

**8.** Das pharmazeutische Pflaster gemäß Anspruch 6 oder 7, wobei der Permeationsbeschleuniger eine nicht-zyklische Verbindung der Formel $C_{2n}H_{4+2}O_n$, umfasst, wobei der Index n 2, 3 oder 4 ist.

**9.** Das pharmazeutische Pflaster gemäß Anspruch 8, wobei die nicht-zyklische Verbindung Diethylenglycolmonomethylether, Dipropylenglycol oder deren Mischung ist.

**10.** Das pharmazeutische Pflaster gemäß einem der vorhergehenden Ansprüche, wobei der Permeationsbeschleuniger

eine organische Säure umfasst, bevorzugt Lävulinsäure.

**11.** Das pharmazeutische Pflaster gemäß einem der vorhergehenden Ansprüche, wobei die Klebstoffschicht ein Antioxidationsmittel umfasst.

**12.** Das pharmazeutische Pflaster gemäß einem der vorhergehenden Ansprüche, wobei die Klebstoffschicht eine Fläche innerhalb des Bereichs von ab 10 bis 750 $cm^2$ aufweist.

**13.** Das pharmazeutische Pflaster gemäß einem der vorhergehenden Ansprüche, das bei der Anwendung auf die Haut

- eine Freisetzung des pharmazeutisch aktiven Bestandteils mit einer Geschwindigkeit von wenigstens 1,0 $ng \cdot cm^{-2} \cdot h^{-1}$ über einen Zeitraum von wenigstens 6 Stunden; oder
- Plasmakonzentrationen des pharmazeutisch aktiven Bestandteils von wenigstens 10 $pg \cdot ml^{-1}$ über einen Zeitraum von wenigstens 6 Stunden zur Verfügung stellt.

**Revendications**

**1.** Timbre pharmaceutique pour administration transdermique du principe actif pharmacologique (1r,4r)-6'-fluoro-N,N-diméthyl-4-phényl-4',9'-dihydro-3'H-spiro-[cyclohexane-1,1'-pyrano[3,4-b]indol]-4-amine, le timbre comprenant une couche de surface, une couche adhésive et une couche protectrice pouvant être enlevée, où

- la couche adhésive est située entre la couche de surface et la couche protectrice pouvant être enlevée ;
- la couche adhésive comprend au moins une partie de la quantité totale du principe actif pharmacologique qui est contenue dans le timbre pharmaceutique ;
- la concentration du principe actif pharmacologique dans la couche adhésive est d'au moins 0,50 % en masse par rapport à la masse totale de la couche adhésive ; et
- la couche adhésive comprend un auto-adhésif à base de polyacrylate.

**2.** Timbre pharmaceutique selon l'une quelconque des revendications précédentes, où la couche de surface a une épaisseur comprise dans l'intervalle allant de 0,1 à 5000 μm et/ou la couche adhésive a une épaisseur comprise dans l'intervalle allant de 1,0 à 1000 μm.

**3.** Timbre pharmaceutique selon l'une quelconque des revendications précédentes, où la couche adhésive comprend un copolymère comprenant des motifs monomères issus des monomères A qui sont choisis parmi les (méth)acrylates d'alkyle en $C_{1-18}$ et/ou les monomères B qui sont copolymérisables avec les monomères A.

**4.** Timbre pharmaceutique selon la revendication 3, où

(i) les monomères A sont choisis dans le groupe constitué par (méth)acrylate de méthyle, (méth)acrylate d'éthyle, (méth)acrylate de propyle, (méth)acrylate de butyle, (méth)acrylate de pentyle, (méth)acrylate d'hexyle, (méth)acrylate de cyclohexyle, (méth)acrylate d'octyle, (méth)acrylate de isobornyle, et les mélanges de ceux-ci ; et/ou
(ii) les monomères B sont choisis dans le groupe constitué par (méth)acrylate de 2-hydroxyéthyle, mono(méth)acrylate de glycéryle, (méth)acrylate de glycidyle, acrylamide, N,N-diéthyl(méth)acrylamide, (méth)acrylate de 2-éthoxyéthyle, (méth)acrylate de 2-éthoxyéthoxyéthyle, (méth)acrylate de tétrahydrofuryle, acétate de vinyle, N-vinylpyrrolidone et les mélanges de ceux-ci.

**5.** Timbre pharmaceutique selon la revendication 3 ou 4, où le copolymère d'acrylate est dérivé d'une composition de monomère comprenant acétate de vinyle, acrylate de 2-éthylhexyle et acrylate de 2-hydroxyéthyle, éventuellement comprenant également du méthacrylate de glycidyle.

**6.** Timbre pharmaceutique selon l'une quelconque des revendications précédentes, où la couche adhésive comprend un composant de perméation qui amplifie la perméation du principe actif pharmacologique à travers la peau humaine.

**7.** Timbre pharmaceutique selon la revendication 6, où le rapport massique relatif du principe actif pharmacologique sur le composant de perméation est compris dans l'intervalle allant de 1 : 1 à 1 : 500.

**8.** Timbre pharmaceutique selon la revendication 6 ou 7, où le composant de perméation comprend un composé non cyclique de formule $C_{2n}H_{4n+2}O_n$, où l'indice n est 2, 3 ou 4.

**9.** Timbre pharmaceutique selon la revendication 8, où le composé non cyclique est l'éther monométhylique de diéthylène glycol, le dipropylène glycol ou l'un de leurs mélanges.

**10.** Timbre pharmaceutique selon l'une quelconque des revendications précédentes, où le composant de perméation comprend un acide organique ; préférentiellement l'acide lévulinique.

**11.** Timbre pharmaceutique selon l'une quelconque des revendications précédentes, où la couche adhésive comprend un antioxydant.

**12.** Timbre pharmaceutique selon l'une quelconque des revendications précédentes, où la couche adhésive présente une surface comprise dans l'intervalle allant de 10 à 750 $cm^2$.

**13.** Timbre pharmaceutique selon l'une quelconque des revendications précédentes, qui, lors de l'application à la peau humaine, confère

- une libération du principe actif pharmacologique à un taux d'au moins 1,0 $ng.cm^{-2}.h^{-1}$ sur une période d'au moins 6 heures ; ou
- des concentrations plasmatiques du principe actif pharmacologique d'au moins 10 $pg.ml^{-1}$ sur une période d'au moins 6 heures.

**Figure 1**

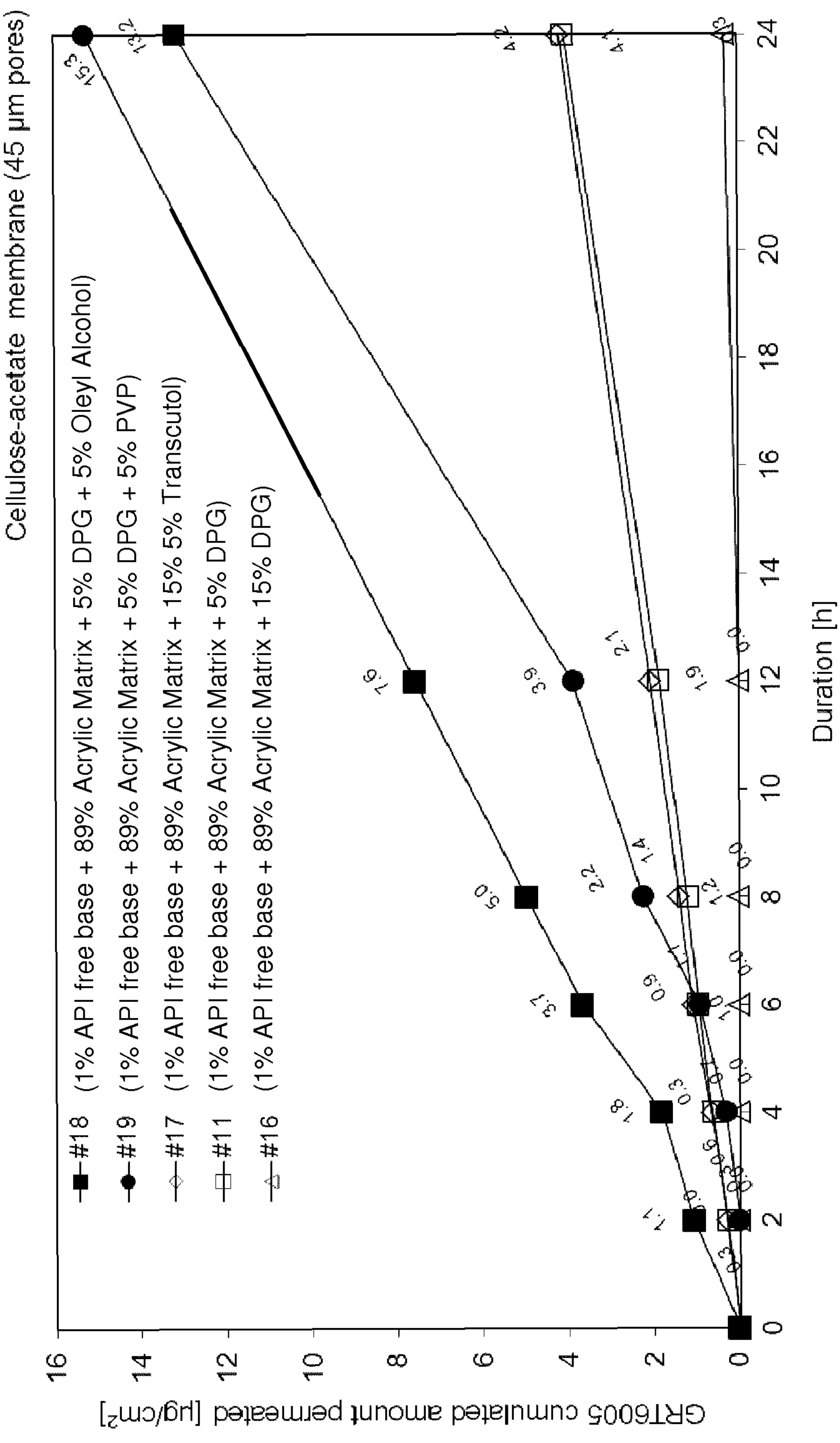
Cellulose-acetate membrane (45 µm pores)
#18 (1% API free base + 89% Acrylic Matrix + 5% DPG + 5% Oleyl Alcohol)
#19 (1% API free base + 89% Acrylic Matrix + 5% DPG + 5% PVP)
#17 (1% API free base + 89% Acrylic Matrix + 15% 5% Transcutol)
#11 (1% API free base + 89% Acrylic Matrix + 5% DPG)
#16 (1% API free base + 89% Acrylic Matrix + 15% DPG)
GRT6005 cumulated amount permeated [µg/cm²]
Duration [h]

**Figure 2**

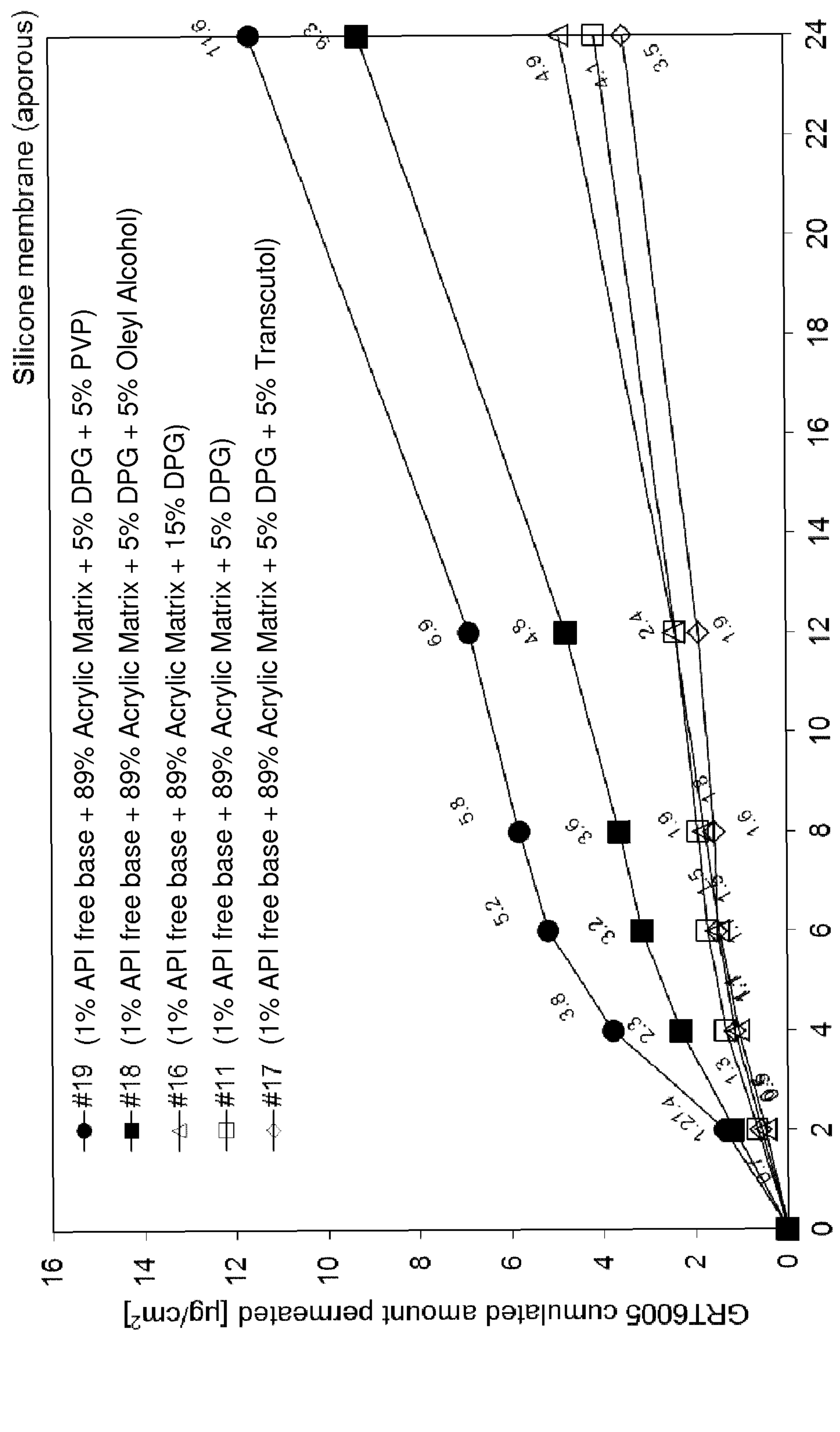
Silicone membrane (aporous)
#19 (1% API free base + 89% Acrylic Matrix + 5% DPG + 5% PVP)
#18 (1% API free base + 89% Acrylic Matrix + 5% DPG + 5% Oleyl Alcohol)
#16 (1% API free base + 89% Acrylic Matrix + 15% DPG)
#11 (1% API free base + 89% Acrylic Matrix + 5% DPG)
#17 (1% API free base + 89% Acrylic Matrix + 5% DPG + 5% Transcutol)
GRT6005 cumulated amount permeated [µg/cm²]
Duration [h]
0
2
4
6
8
10
12
14
16
18
20
22
24

**Figure 3**

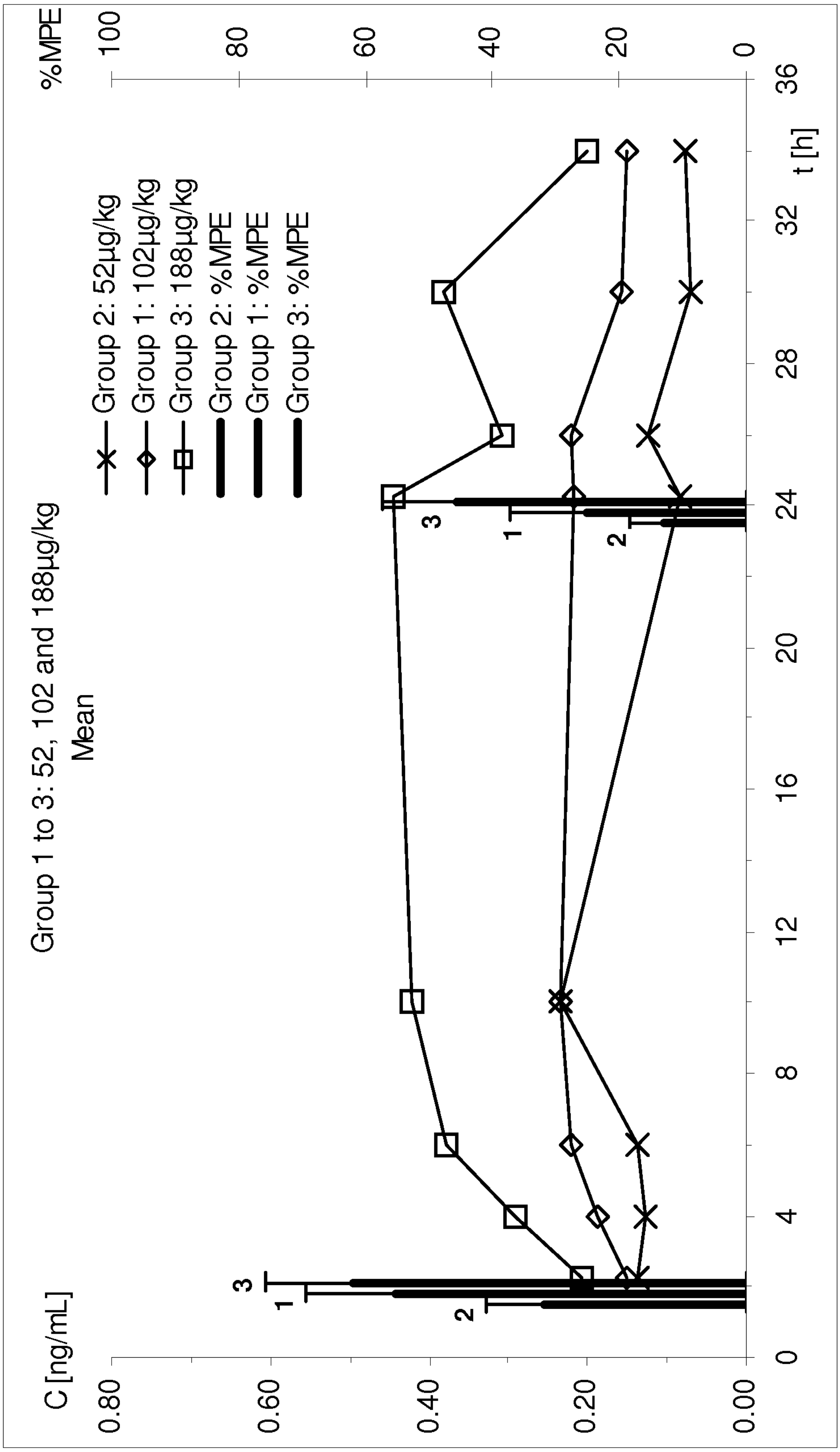
Group 1 to 3: 52, 102 and 188µg/kg
Mean
C [ng/mL]
0.80
0.60
0.40
0.20
0.00
%MPE
100
80
60
40
20
0
0
4
8
12
16
20
24
28
32
36
t [h]
Group 2: 52µg/kg
Group 1: 102µg/kg
Group 3: 188µg/kg
Group 2: %MPE
Group 1: %MPE
Group 3: %MPE
1
2
3
1
2
3

**Figure 4**

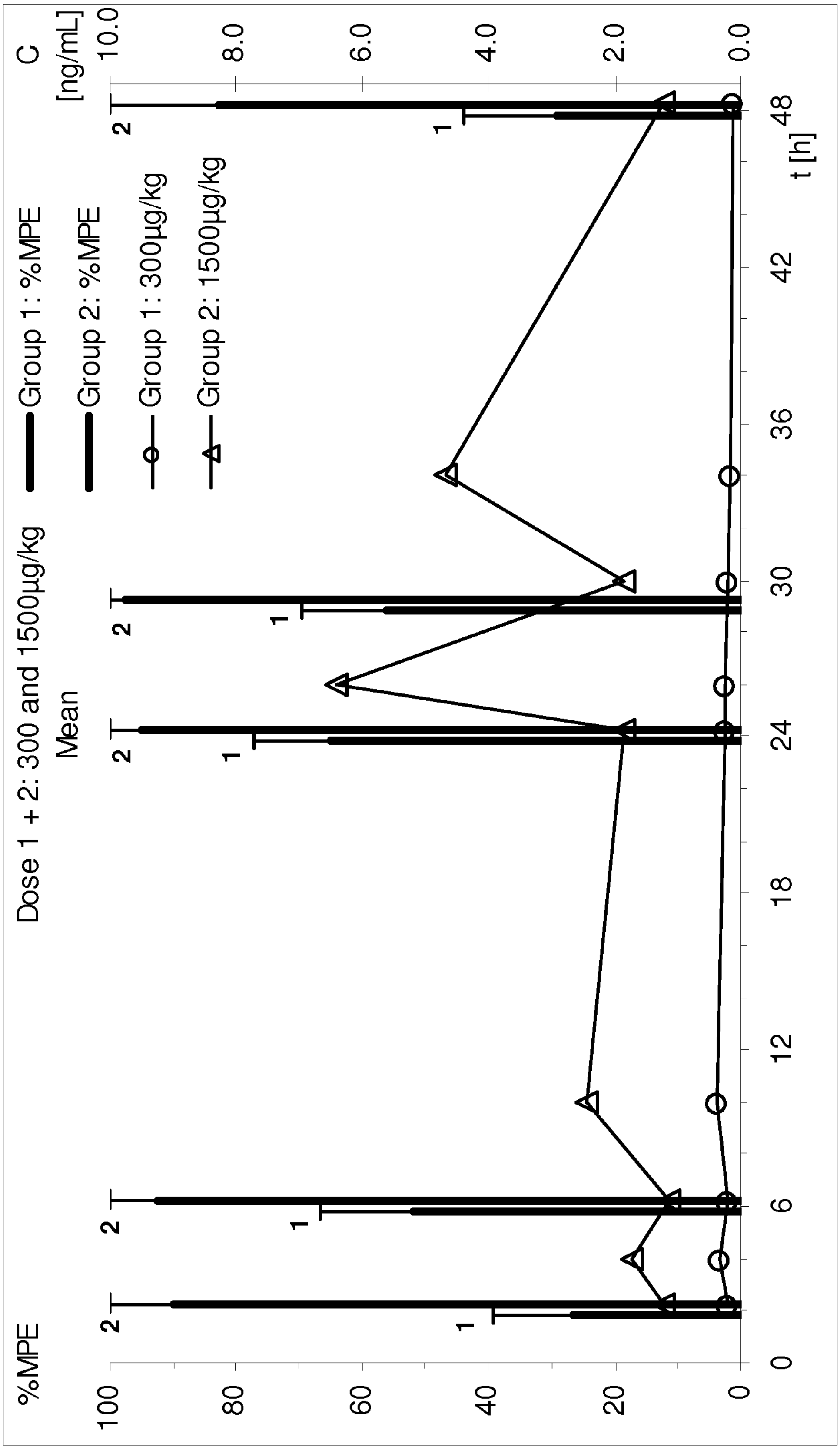
Dose 1 + 2: 300 and 1500µg/kg
Mean
%MPE
C
[ng/mL]
t [h]
Group 1: %MPE
Group 2: %MPE
Group 1: 300µg/kg
Group 2: 1500µg/kg
0
20
40
60
80
100
0.0
2.0
4.0
6.0
8.0
10.0
0
6
12
18
24
30
36
42
48
1
2

Figure 5

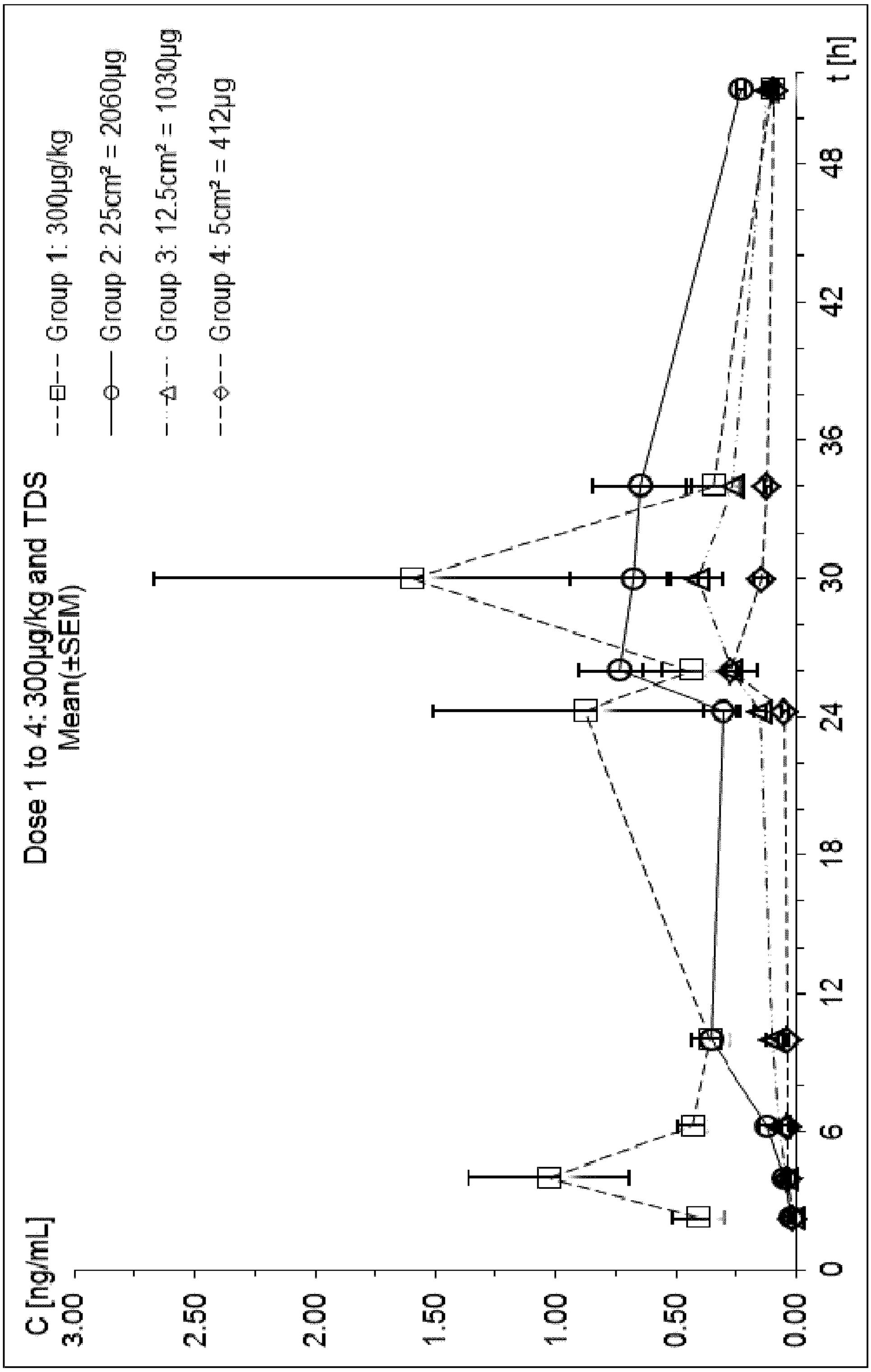
Dose 1 to 4: 300µg/kg and TDS
Mean(±SEM)
C [ng/mL]
t [h]
Group 1: 300µg/kg
Group 2: 25cm² = 2060µg
Group 3: 12.5cm² = 1030µg
Group 4: 5cm² = 412µg
3.00
2.50
2.00
1.50
1.00
0.50
0.00
0
6
12
18
24
30
36
42
48

Figure 6

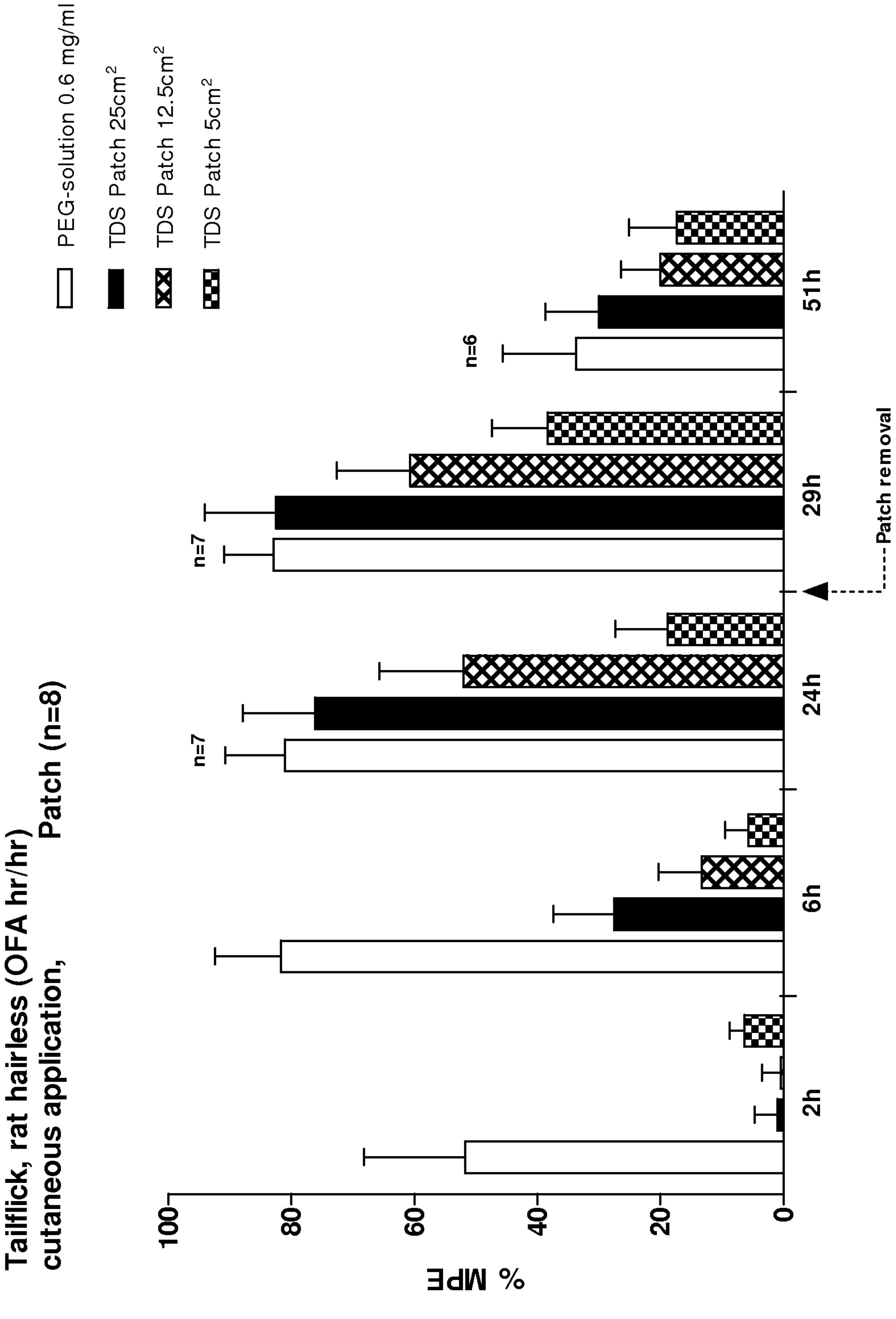

Tailflick, rat hairless (OFA hr/hr)
cutaneous application, Patch (n=8)
% MPE
100
80
60
40
20
0
2h
6h
24h
29h
51h
n=7
n=7
n=6
Patch removal
PEG-solution 0.6 mg/ml
TDS Patch 25cm2
TDS Patch 12.5cm2
TDS Patch 5cm2

Figure 7

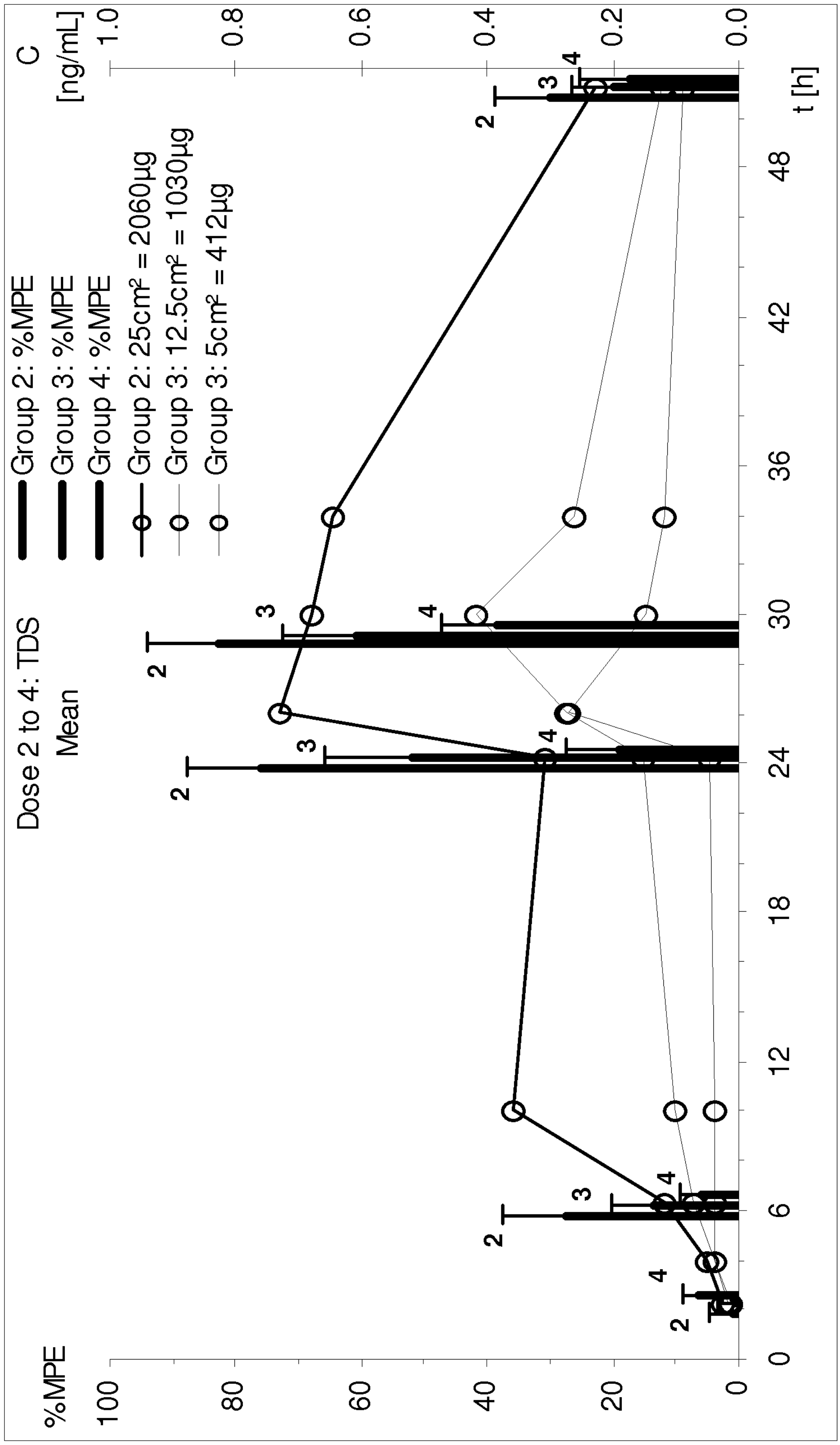

Dose 2 to 4: TDS
Mean
Group 2: %MPE
Group 3: %MPE
Group 4: %MPE
Group 2: 25cm² = 2060µg
Group 3: 12.5cm² = 1030µg
Group 3: 5cm² = 412µg
%MPE
100
80
60
40
20
0
C
[ng/mL]
1.0
0.8
0.6
0.4
0.2
0.0
0
6
12
18
24
30
36
42
48
t [h]
2
3
4

Figure 8

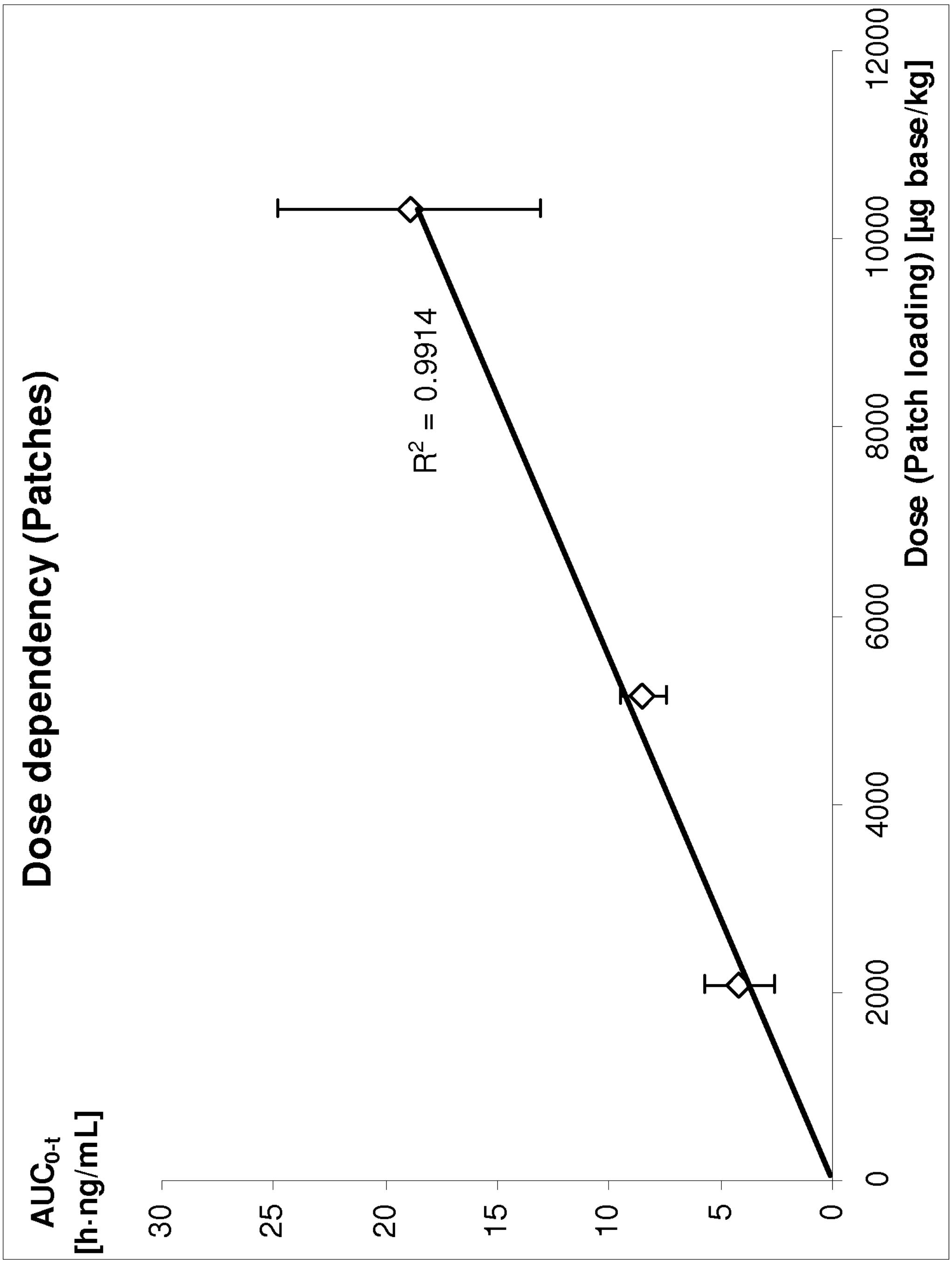
Dose dependency (Patches)
AUC0-t
[h·ng/mL]
30
25
20
15
10
5
0
0
2000
4000
6000
8000
10000
12000
Dose (Patch loading) [µg base/kg]
R2 = 0.9914

Figure 9

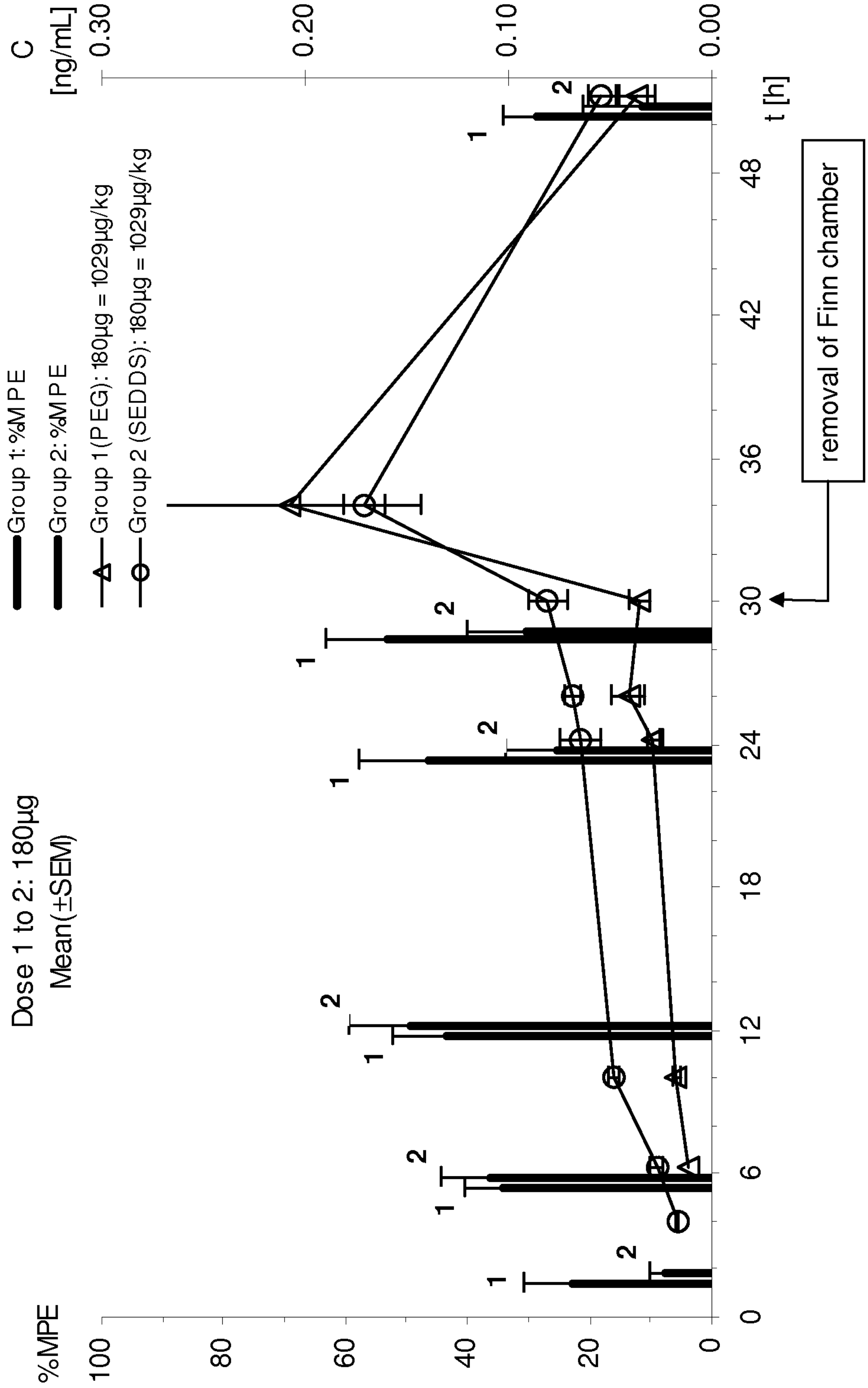
Dose 1 to 2: 180µg
Mean(±SEM)
Group 1: %MPE
Group 2: %MPE
Group 1 (PEG): 180µg = 1029µg/kg
Group 2 (SEDDS): 180µg = 1029µg/kg
%MPE
100
80
60
40
20
0
C
[ng/mL]
0.30
0.20
0.10
0.00
0
6
12
18
24
30
36
42
48
t [h]
1
2
removal of Finn chamber

Figure 10

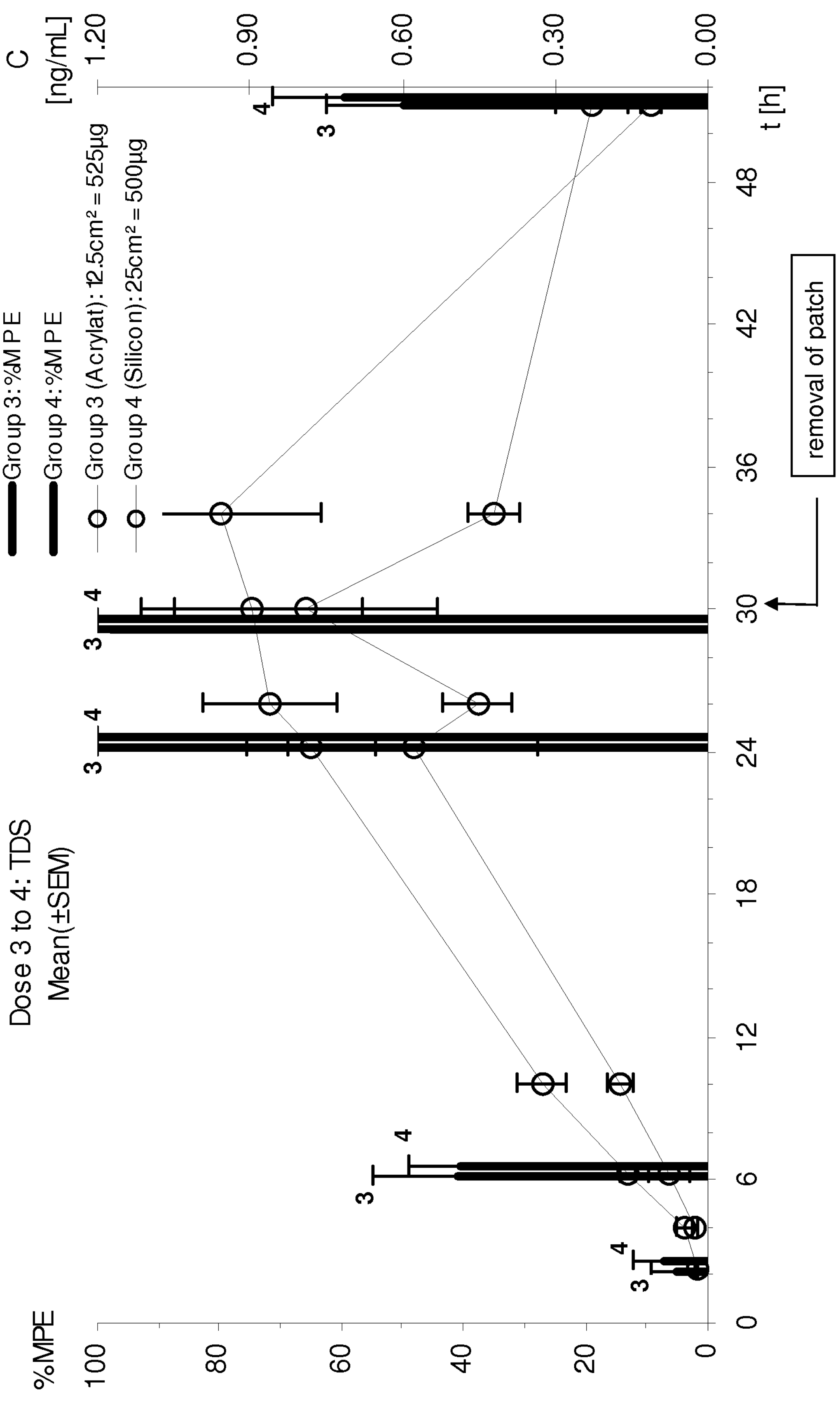
Dose 3 to 4: TDS
Mean(±SEM)
%MPE
100
80
60
40
20
0
C
[ng/mL]
1.20
0.90
0.60
0.30
0.00
t [h]
0
6
12
18
24
30
36
42
48
Group 3: %MPE
Group 4: %MPE
Group 3 (Acrylat): 12.5cm² = 525µg
Group 4 (Silicon): 25cm² = 500µg
removal of patch
3
4

Figure 11

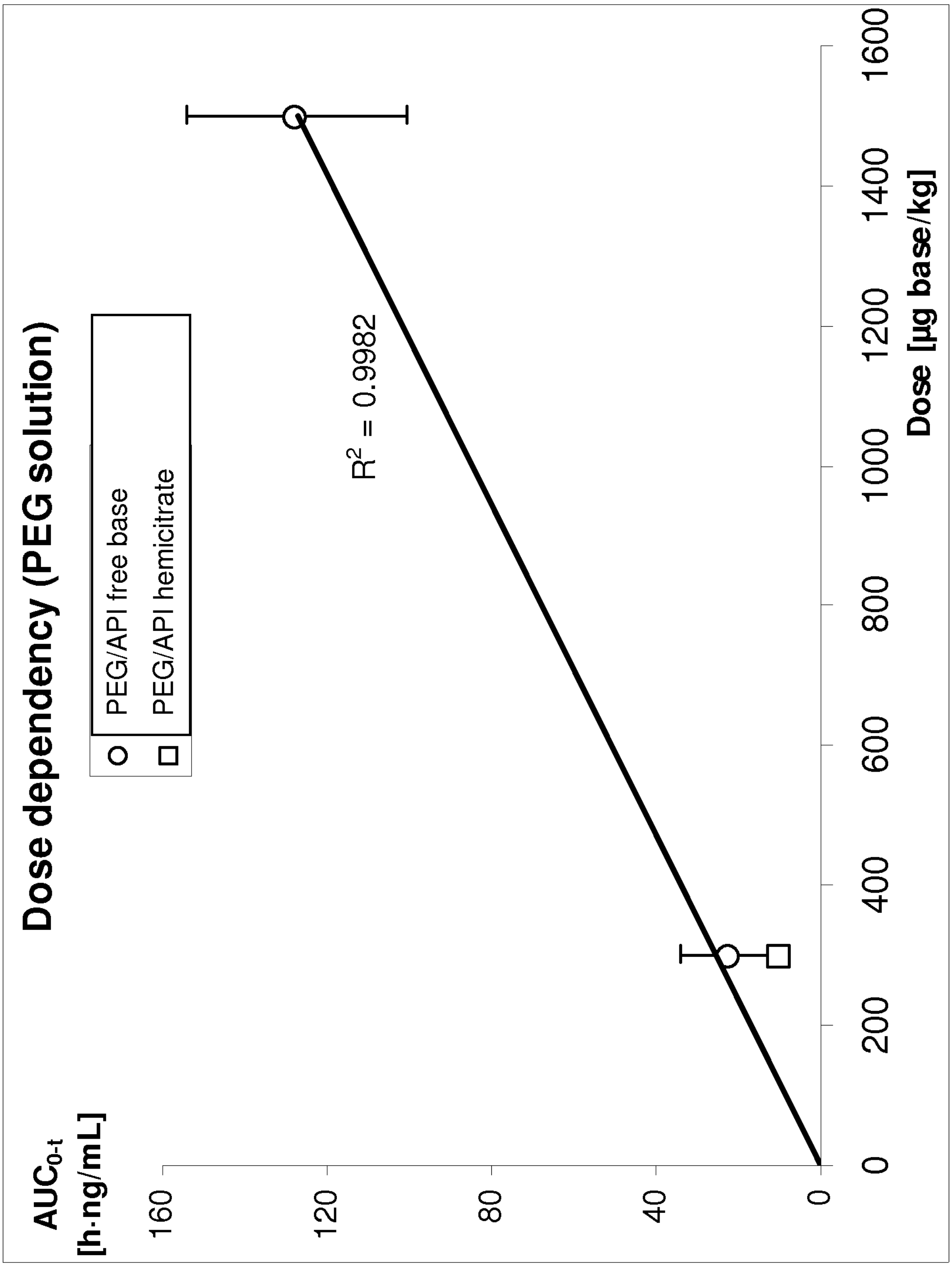
Dose dependency (PEG solution)
PEG/API free base
PEG/API hemicitrate
$R^2$ = 0.9982
AUC$_{0-t}$ [h·ng/mL]
160
120
80
40
0
0
200
400
600
800
1000
1200
1400
1600
Dose [µg base/kg]

Figure 12

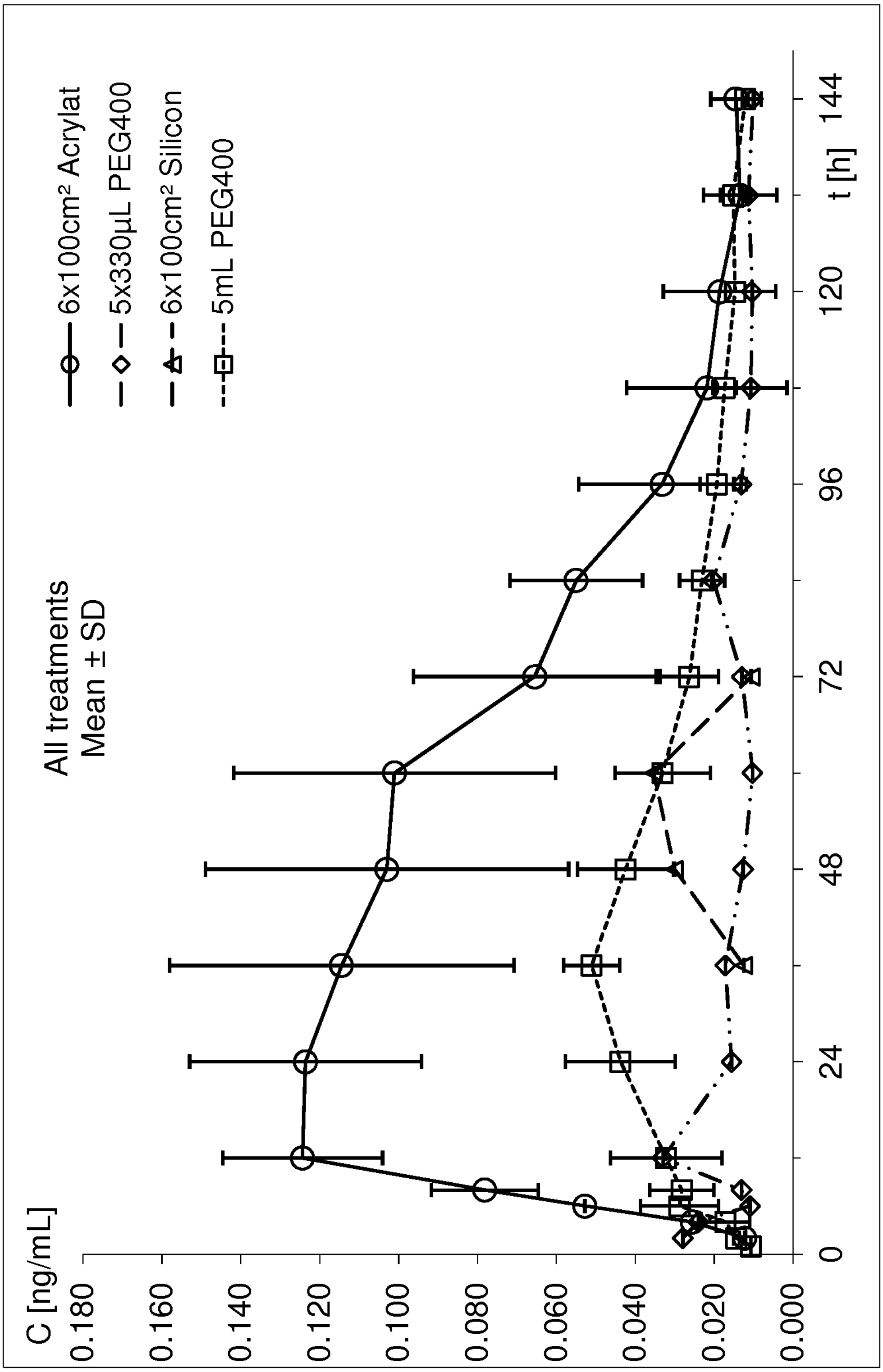
All treatments
Mean ± SD
6x100cm² Acrylat
5x330µL PEG400
6x100cm² Silicon
5mL PEG400
C [ng/mL]
0.180
0.160
0.140
0.120
0.100
0.080
0.060
0.040
0.020
0.000
0
24
48
72
96
120
144
t [h]

Figure 13

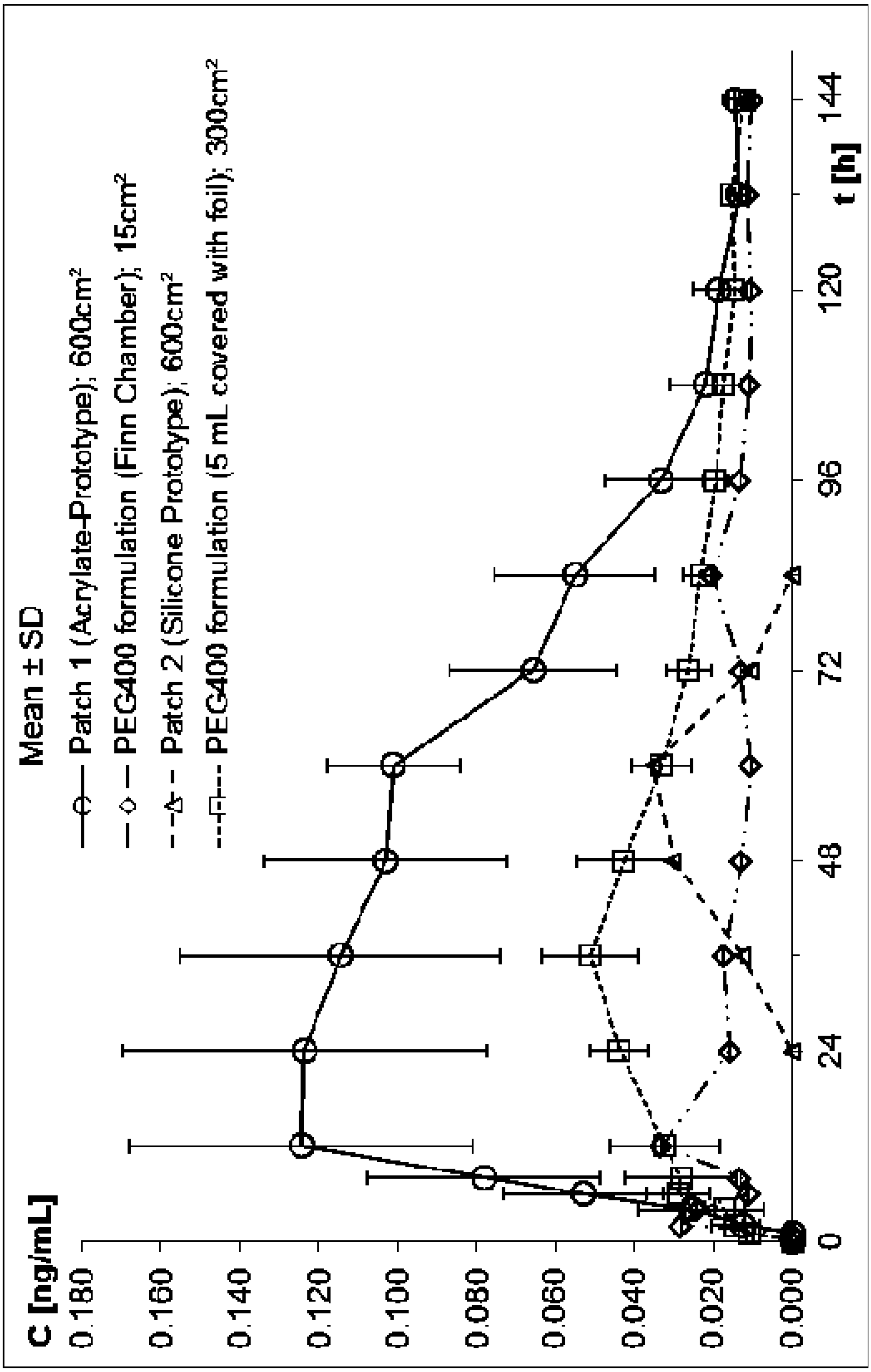
C [ng/mL]
0.180
0.160
0.140
0.120
0.100
0.080
0.060
0.040
0.020
0.000
0
24
48
72
96
120
144
t [h]
Mean ± SD
Patch 1 (Acrylate-Prototype); 600cm²
PEG400 formulation (Finn Chamber); 15cm²
Patch 2 (Silicone Prototype); 600cm²
PEG400 formulation (5 mL covered with foil); 300cm²

Figure 14

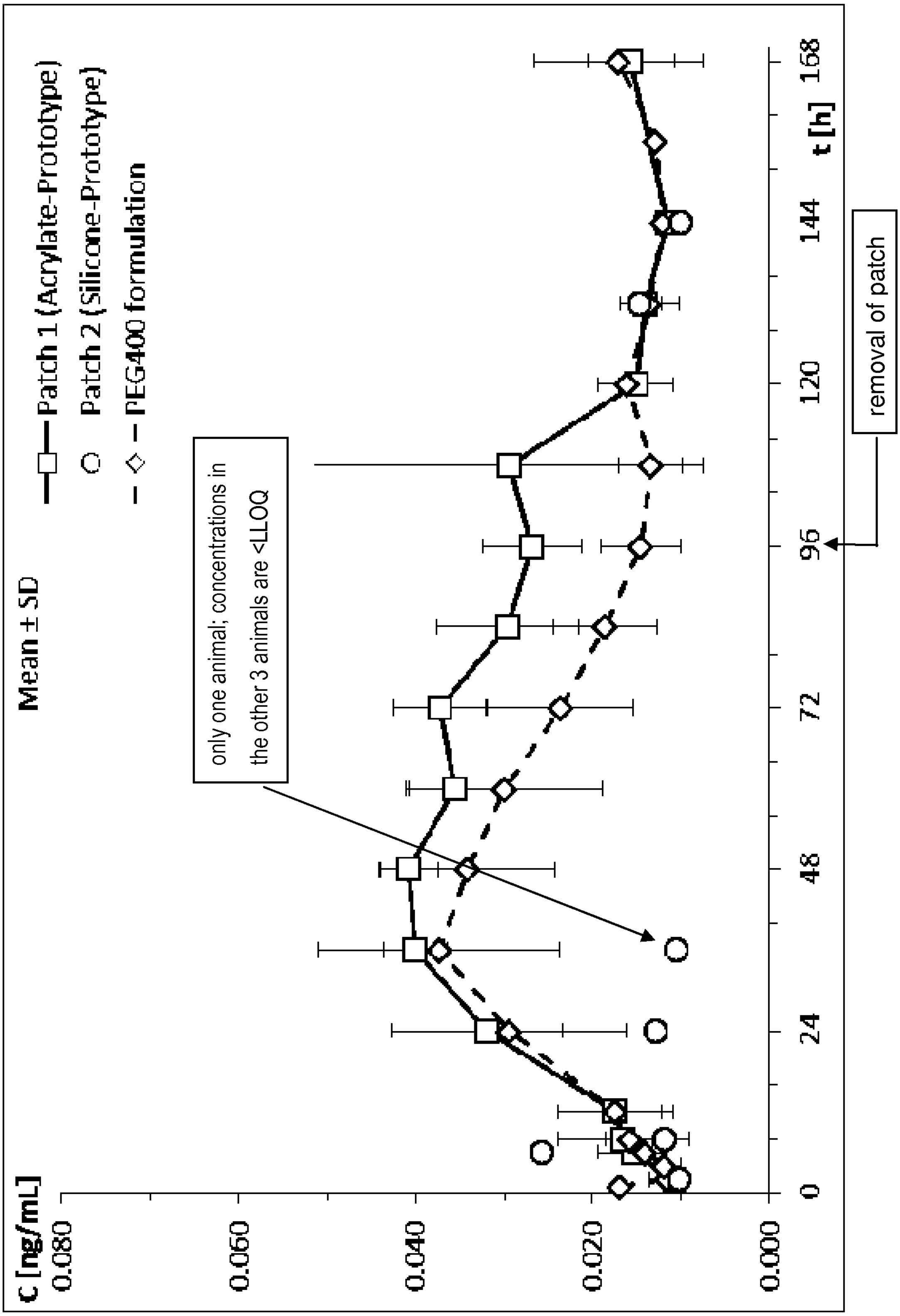
Mean ± SD
C [ng/mL]
0.080
0.060
0.040
0.020
0.000
0
24
48
72
96
120
144
168
t [h]
Patch 1 (Acrylate-Prototype)
Patch 2 (Silicone-Prototype)
PEG400 formulation
only one animal; concentrations in the other 3 animals are <LLOQ
removal of patch

Figure 15

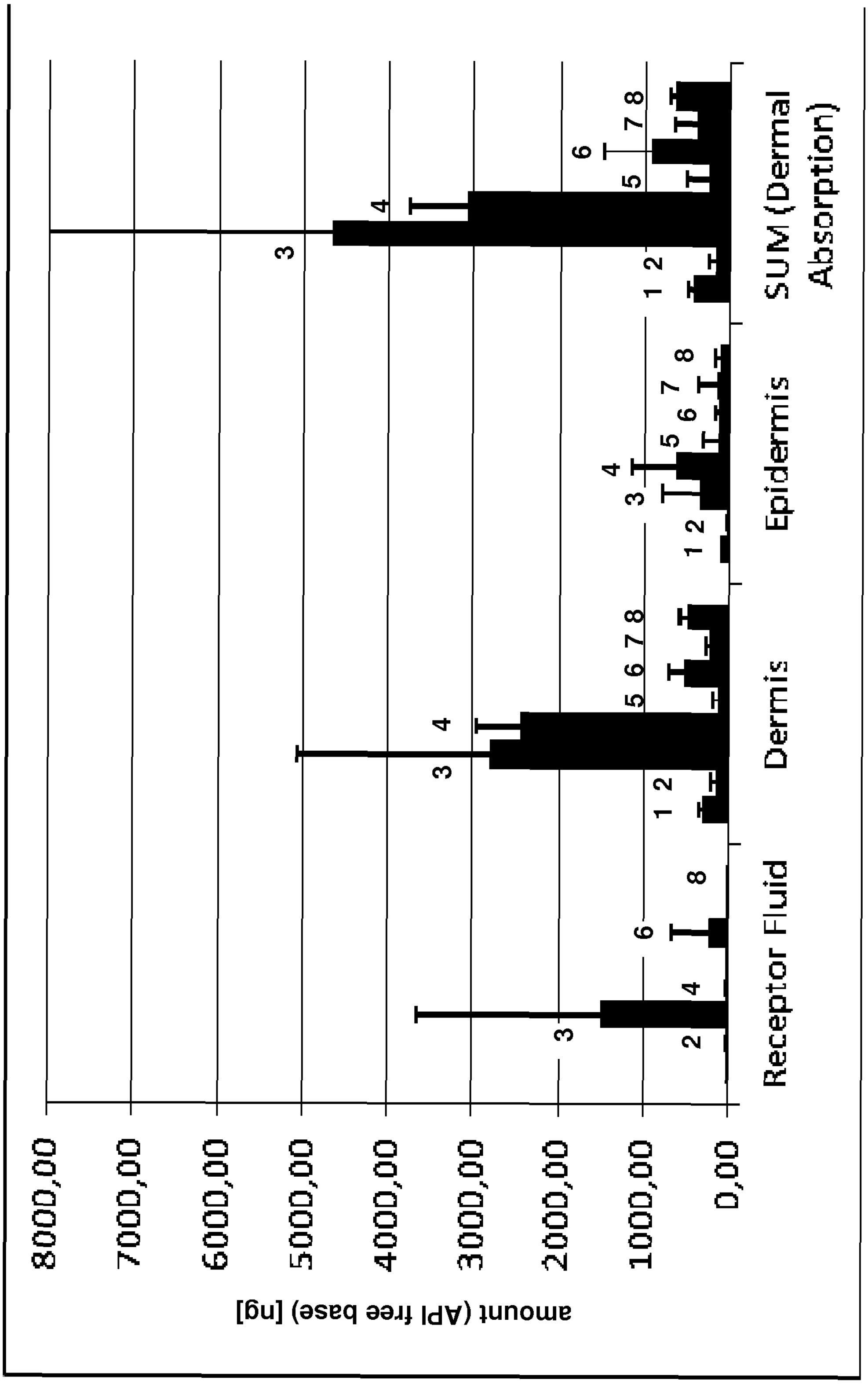
amount (API free base) [ng]
8000,00
7000,00
6000,00
5000,00
4000,00
3000,00
2000,00
1000,00
0,00
Receptor Fluid
Dermis
Epidermis
SUM (Dermal Absorption)
1
2
3
4
5
6
7
8

Figure 16

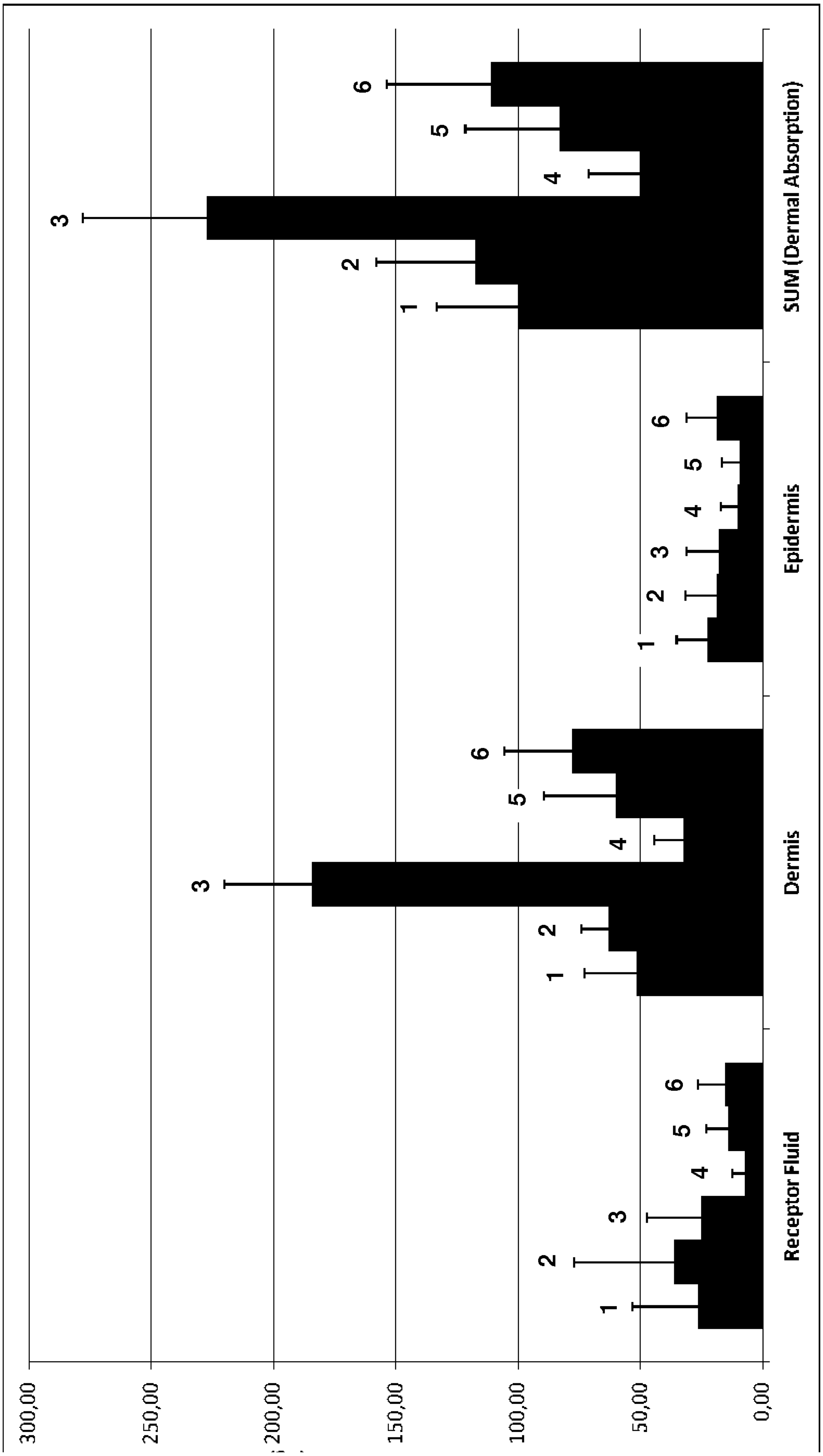
300,00
250,00
200,00
150,00
100,00
50,00
0,00
Receptor Fluid
Dermis
Epidermis
SUM (Dermal Absorption)
1
2
3
4
5
6

Figure 17

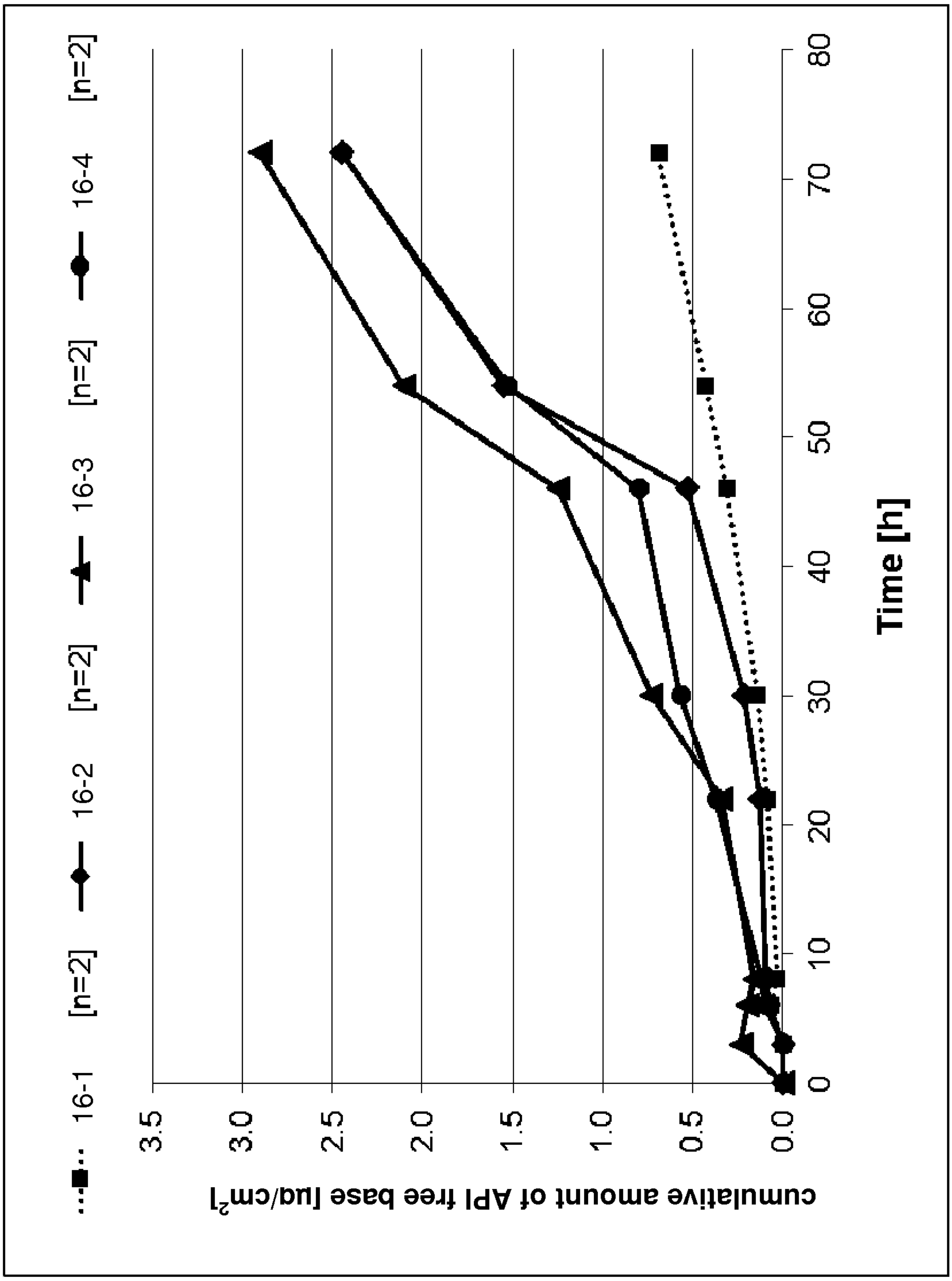
16-1 [n=2]
16-2 [n=2]
16-3 [n=2]
16-4 [n=2]
cumulative amount of API free base [µg/$cm^2$]
0.0
0.5
1.0
1.5
2.0
2.5
3.0
3.5
Time [h]
0
10
20
30
40
50
60
70
80

Figure 18

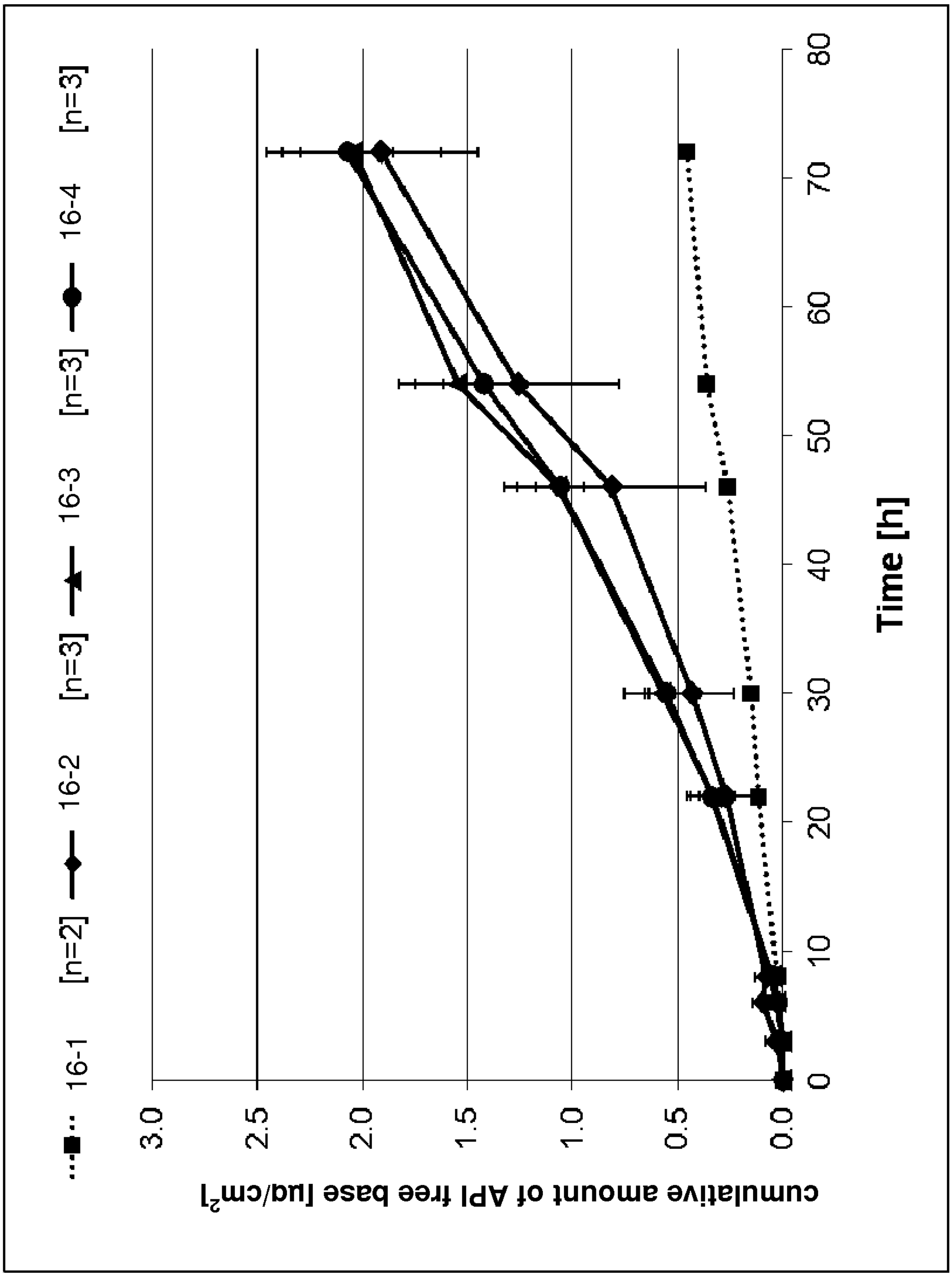

16-1 [n=2]
16-2 [n=3]
16-3 [n=3]
16-4 [n=3]
cumulative amount of API free base [µg/cm²]
0.0
0.5
1.0
1.5
2.0
2.5
3.0
0
10
20
30
40
50
60
70
80
Time [h]

Figure 19

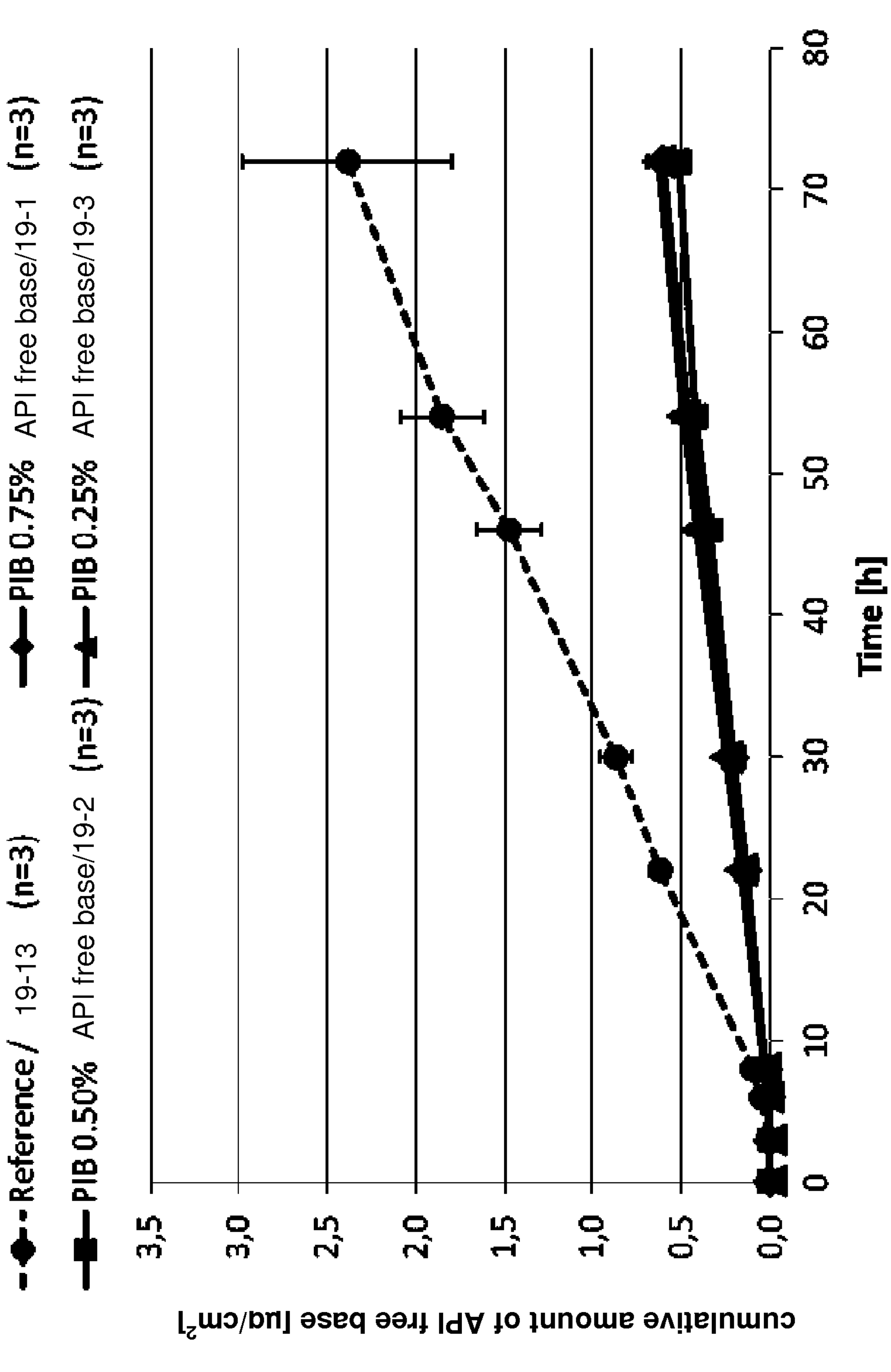
Reference / 19-13 (n=3)
PIB 0.75% API free base/19-1 (n=3)
PIB 0.50% API free base/19-2 (n=3)
PIB 0.25% API free base/19-3 (n=3)
cumulative amount of API free base [μg/cm²]
3,5
3,0
2,5
2,0
1,5
1,0
0,5
0,0
0
10
20
30
40
50
60
70
80
Time [h]

Figure 20

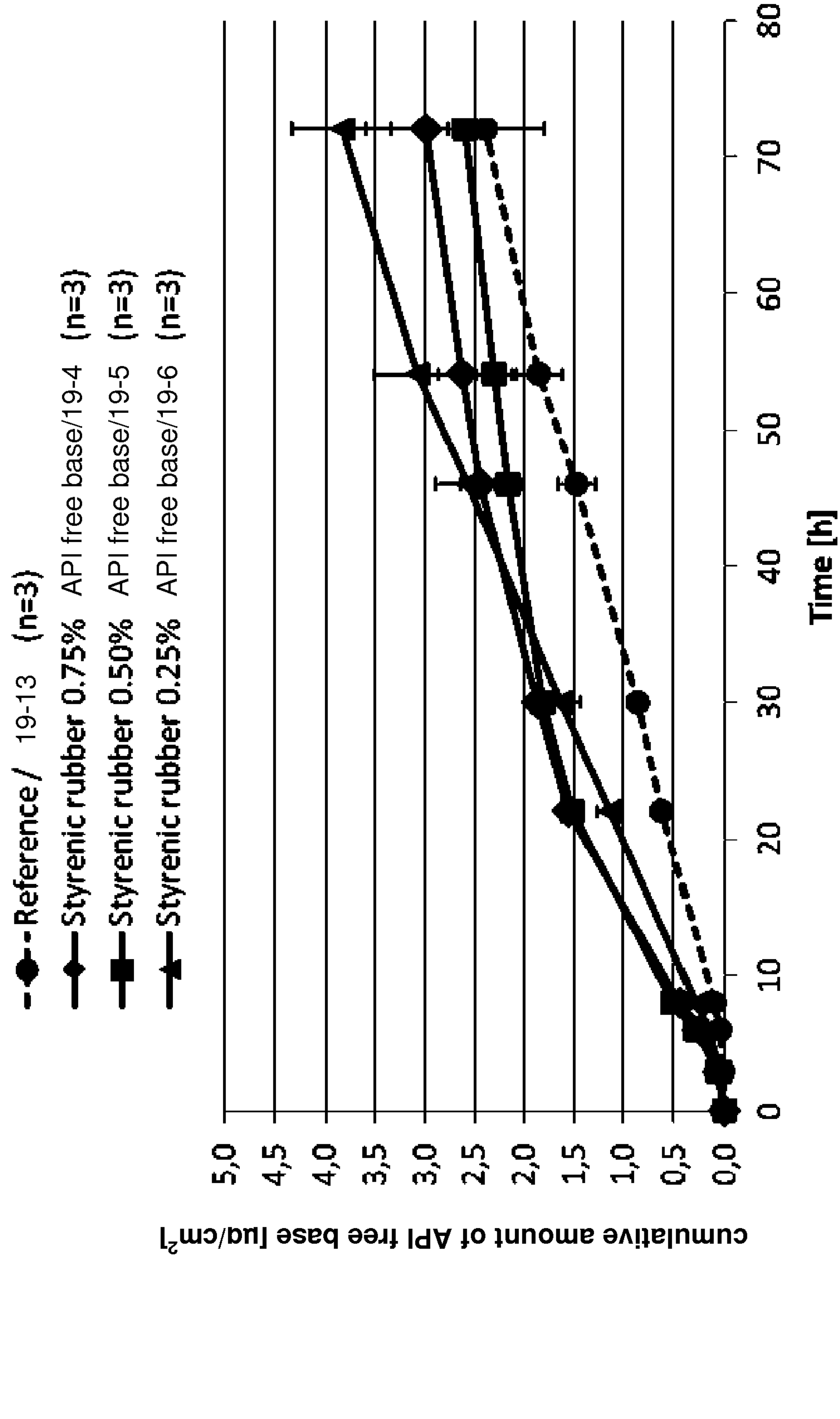

Reference / 19-13 (n=3)
Styrenic rubber 0.75% API free base/19-4 (n=3)
Styrenic rubber 0.50% API free base/19-5 (n=3)
Styrenic rubber 0.25% API free base/19-6 (n=3)
cumulative amount of API free base [µg/cm²]
5,0
4,5
4,0
3,5
3,0
2,5
2,0
1,5
1,0
0,5
0,0
0
10
20
30
40
50
60
70
80
Time [h]

Figure 21

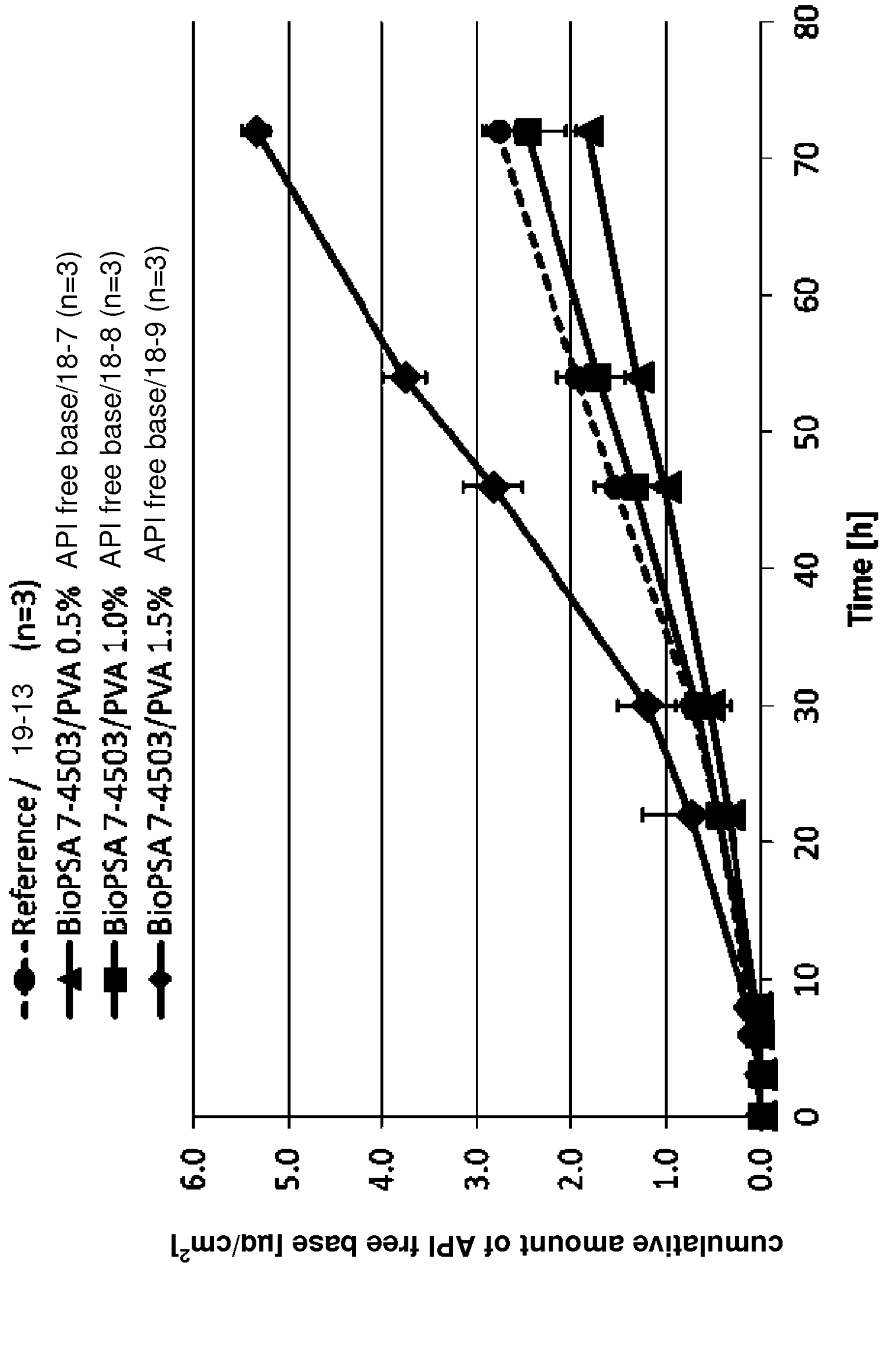
Reference / 19-13 (n=3)
BioPSA 7-4503/PVA 0.5% API free base/18-7 (n=3)
BioPSA 7-4503/PVA 1.0% API free base/18-8 (n=3)
BioPSA 7-4503/PVA 1.5% API free base/18-9 (n=3)
cumulative amount of API free base [µg/cm²]
0.0
1.0
2.0
3.0
4.0
5.0
6.0
Time [h]
0
10
20
30
40
50
60
70
80

Figure 22

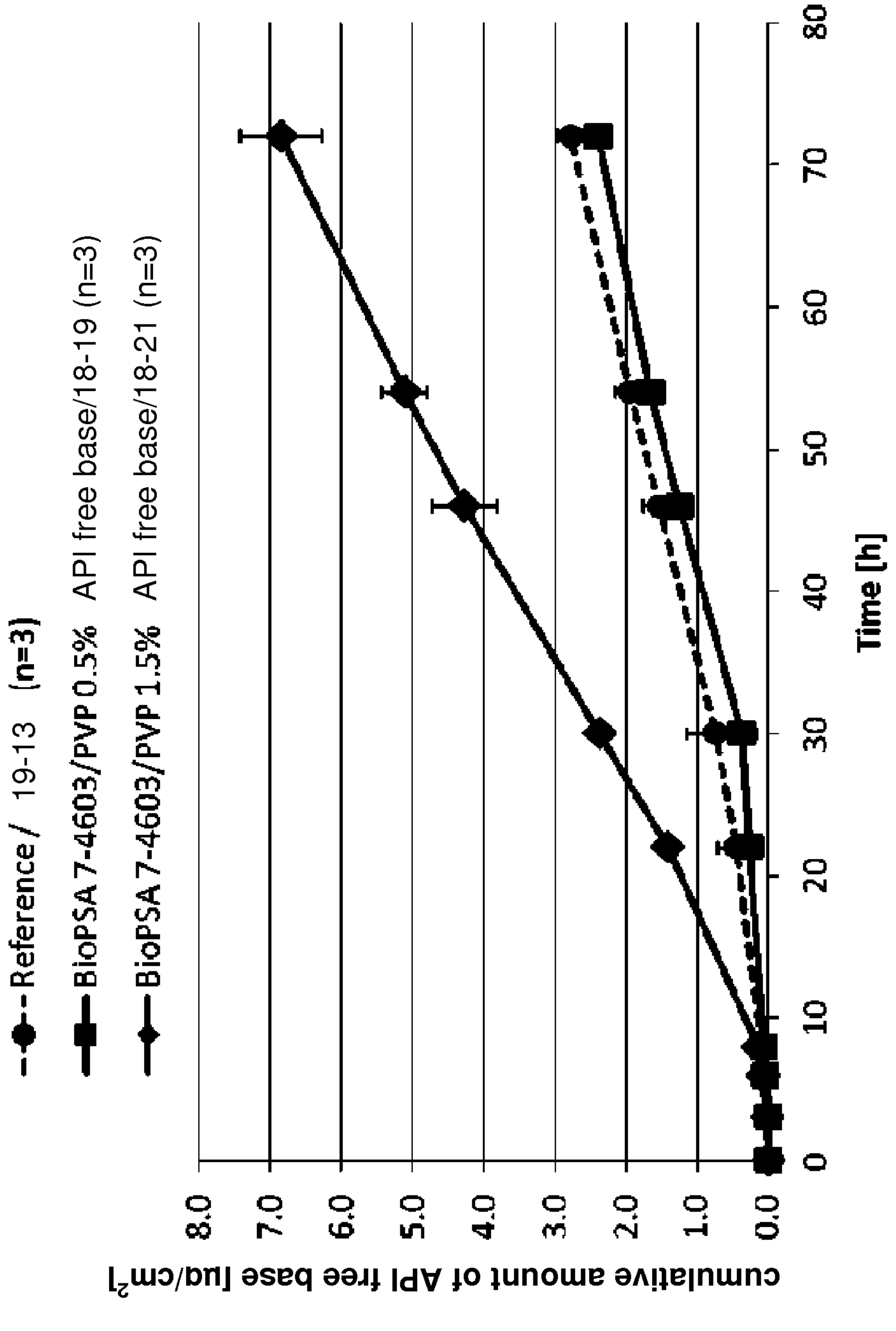
Reference / 19-13 (n=3)
BioPSA 7-4603/PVP 0.5% API free base/18-19 (n=3)
BioPSA 7-4603/PVP 1.5% API free base/18-21 (n=3)
cumulative amount of API free base [µg/cm²]
8.0
7.0
6.0
5.0
4.0
3.0
2.0
1.0
0.0
0
10
20
30
40
50
60
70
80
Time [h]

Figure 23

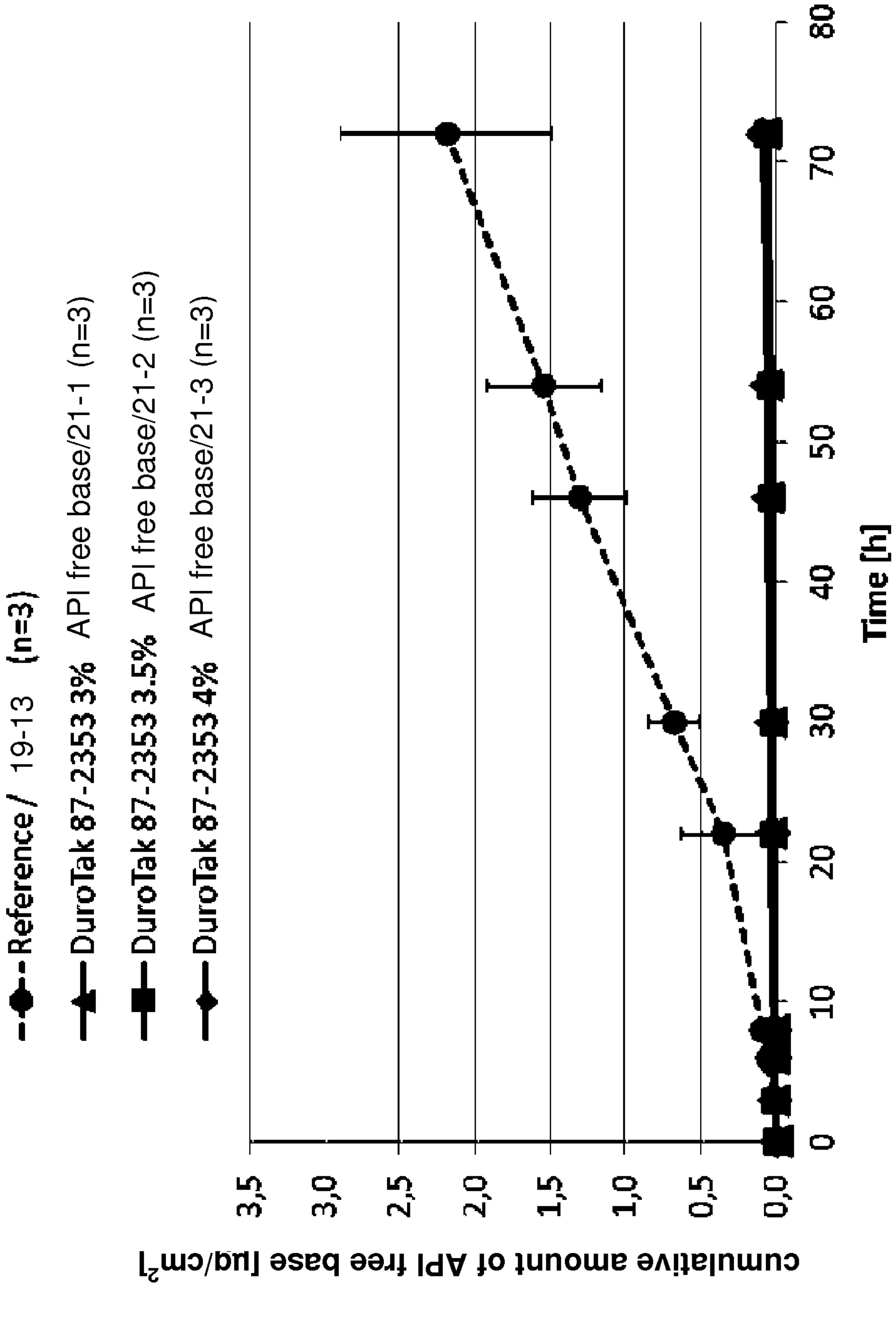
Reference / 19-13 (n=3)
DuroTak 87-2353 3% API free base/21-1 (n=3)
DuroTak 87-2353 3.5% API free base/21-2 (n=3)
DuroTak 87-2353 4% API free base/21-3 (n=3)
cumulative amount of API free base [µg/cm²]
3,5
3,0
2,5
2,0
1,5
1,0
0,5
0,0
0
10
20
30
40
50
60
70
80
Time [h]

Figure 24

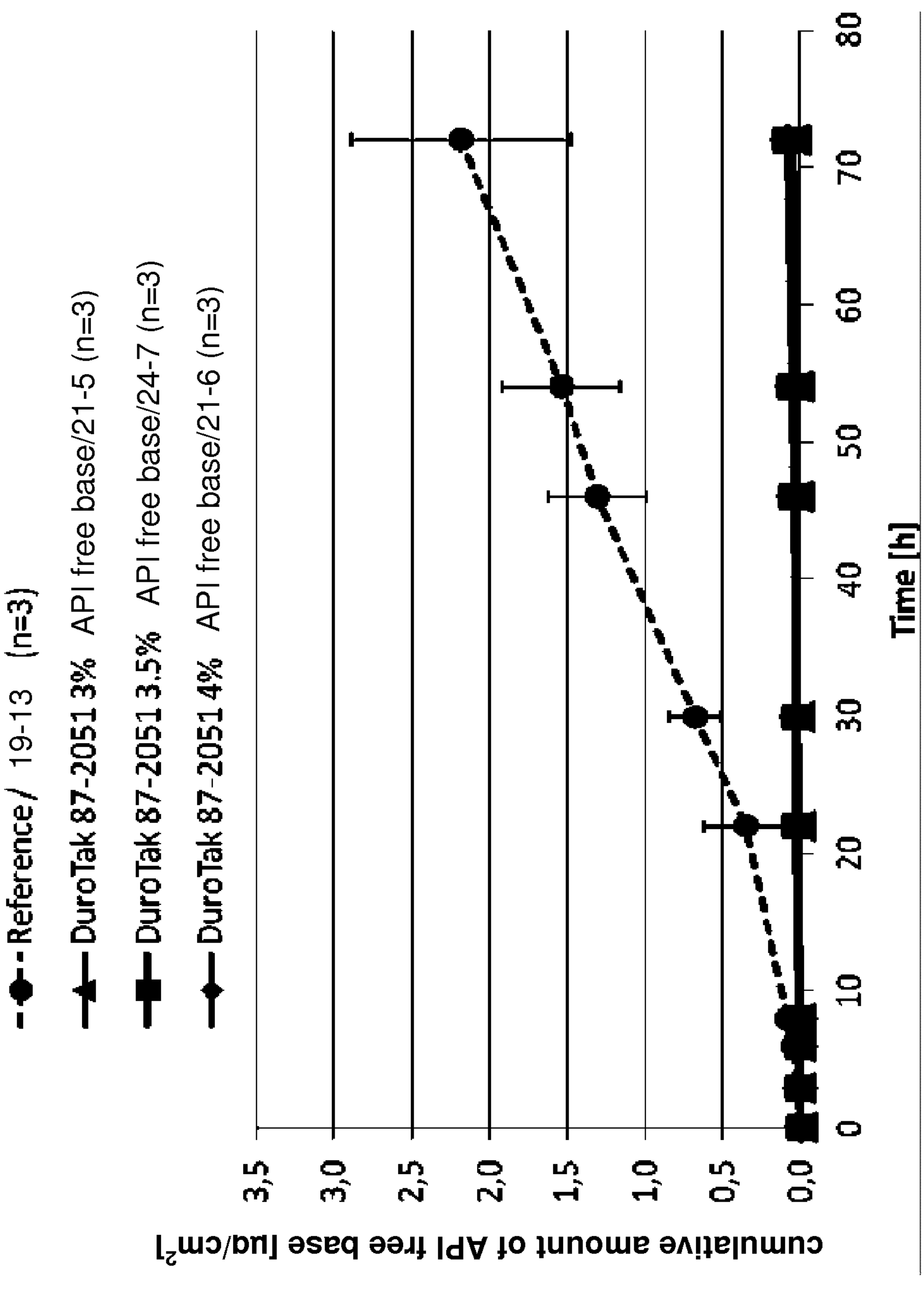
Reference / 19-13 (n=3)
DuroTak 87-2051 3% API free base/21-5 (n=3)
DuroTak 87-2051 3.5% API free base/24-7 (n=3)
DuroTak 87-2051 4% API free base/21-6 (n=3)
cumulative amount of API free base [µg/cm²]
3,5
3,0
2,5
2,0
1,5
1,0
0,5
0,0
0
10
20
30
40
50
60
70
80
Time [h]

Figure 25

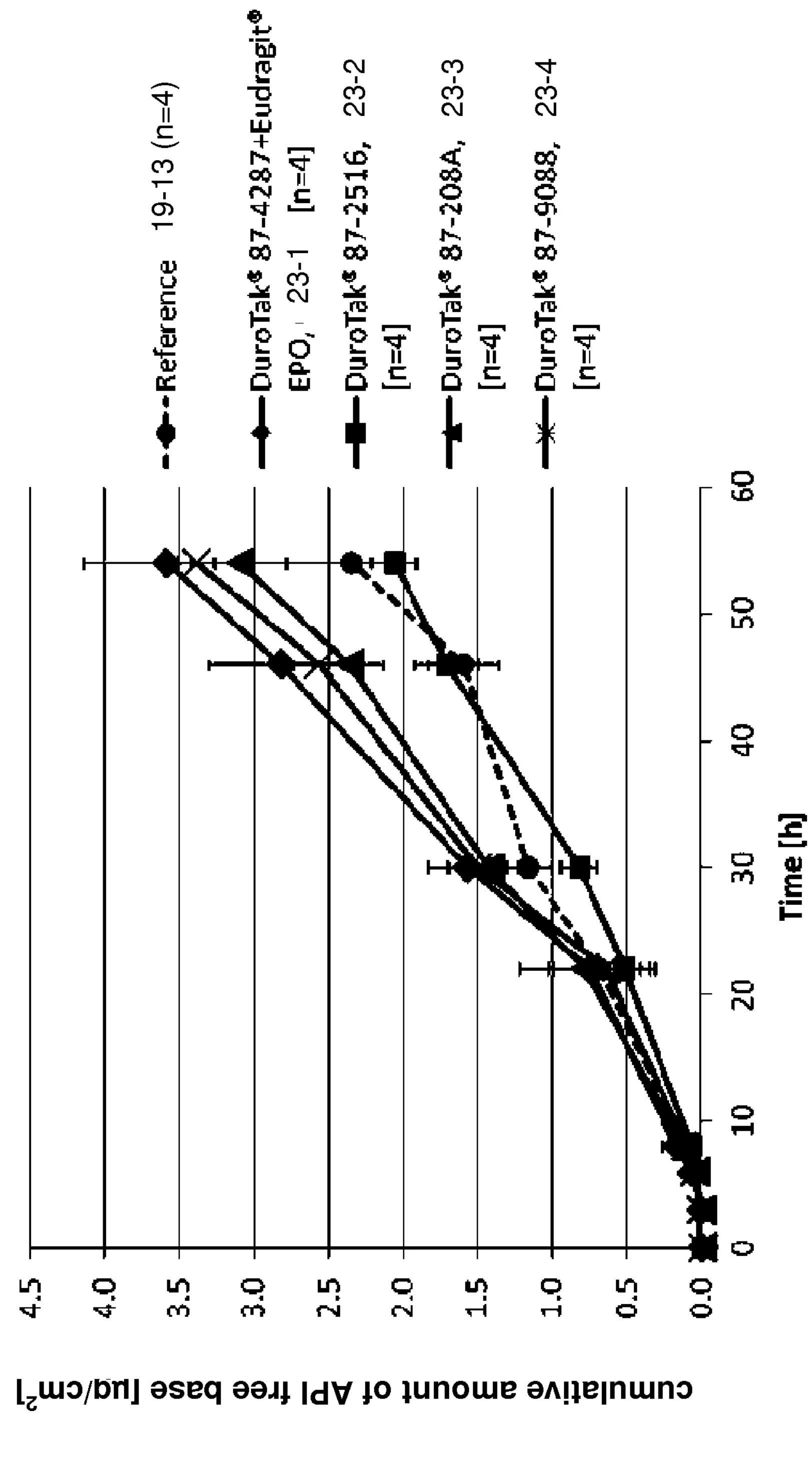
cumulative amount of API free base [µg/cm²]
4.5
4.0
3.5
3.0
2.5
2.0
1.5
1.0
0.5
0.0
0
10
20
30
40
50
60
Time [h]
Reference 19-13 (n=4)
DuroTak® 87-4287+Eudragit® EPO, 23-1 [n=4]
DuroTak® 87-2516, 23-2 [n=4]
DuroTak® 87-208A, 23-3 [n=4]
DuroTak® 87-9088, 23-4 [n=4]

Figure 26

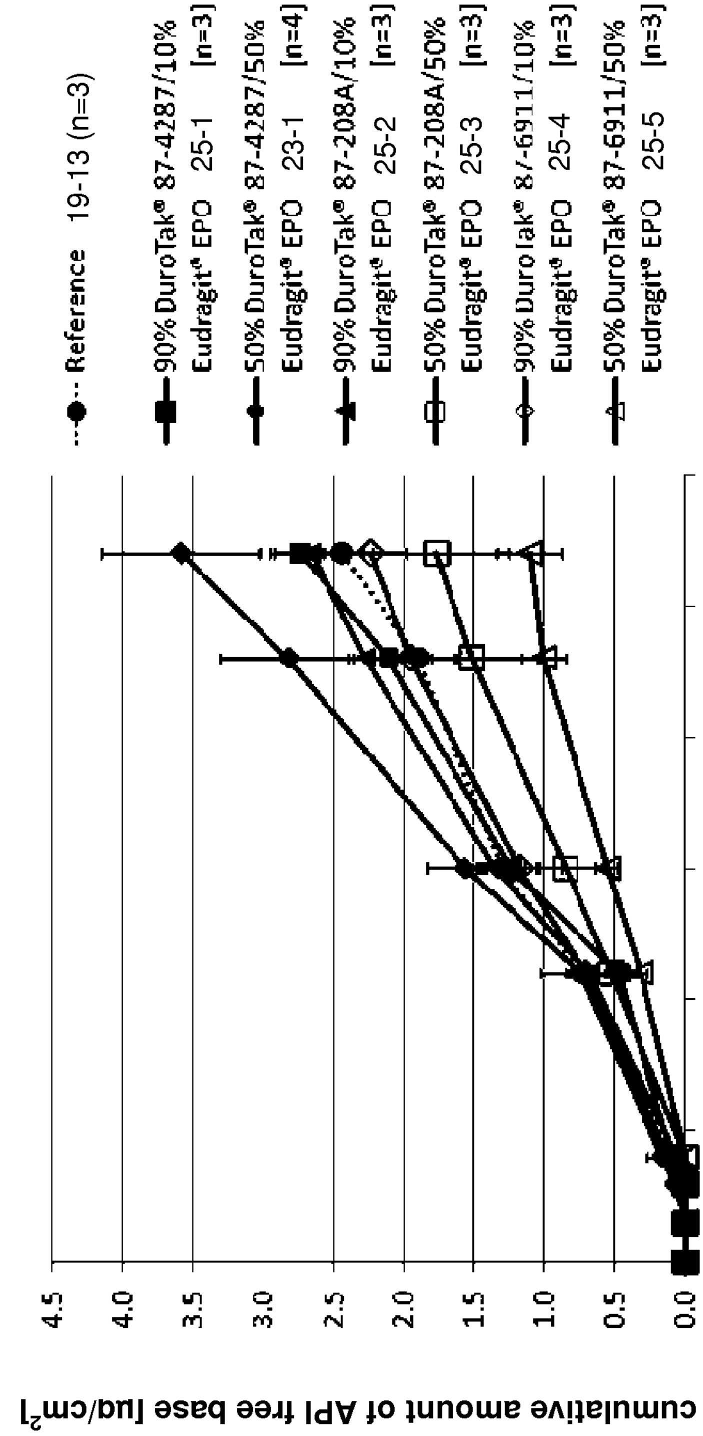
cumulative amount of API free base [µg/cm²]
4.5
4.0
3.5
3.0
2.5
2.0
1.5
1.0
0.5
0.0
0
10
20
30
40
50
60
Time [h]
Reference 19-13 (n=3)
90% DuroTak® 87-4287/10% Eudragit® EPO 25-1 [n=3]
50% DuroTak® 87-4287/50% Eudragit® EPO 23-1 [n=4]
90% DuroTak® 87-208A/10% Eudragit® EPO 25-2 [n=3]
50% DuroTak® 87-208A/50% Eudragit® EPO 25-3 [n=3]
90% DuroTak® 87-6911/10% Eudragit® EPO 25-4 [n=3]
50% DuroTak® 87-6911/50% Eudragit® EPO 25-5 [n=3]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004043967 A
- WO 2008040481 A


### Non-patent literature cited in the description


- **T.K. GHOSH et al.** Transdermal and topical drug delivery systems. Interpharm Press, 2002
- **SMITH et al.** Percutaneous Penetration Enhancers. CRC Press, 1995
- **H.A.E. BENSON et al.** Topical and Transdermal Drug Delivery: Principles and Practice. John Wiley & Sons, 2011
- **A.K. BANGA.** Transdermal and Intradermal Delivery of Therapeutic Agents: Application of Physical Technologies. CRC Press Inc, 2011
- **D'AMOUR ; SMITH.** *J. Pharm. Exp. Ther.,* 1941, vol. 72, 74-79